(19) Europäisches Patentamt
European Patent Office
Office européen des brevets

(11) EP 3 415 531 B1

(12) EUROPEAN PATENT SPECIFICATION

(45) Date of publication and mention of the grant of the patent:
**06.09.2023 Bulletin 2023/36**

(21) Application number: **18163942.8**

(22) Date of filing: **24.05.2012**

(51) International Patent Classification (IPC):
***C07K 16/28*** $^{(2006.01)}$ ***A61K 39/00*** $^{(2006.01)}$
***A61K 47/68*** $^{(2017.01)}$

(52) Cooperative Patent Classification (CPC):
**C07K 16/2878; A61K 47/6817; A61K 47/6849; A61P 29/00; A61P 35/00; A61P 35/02; A61P 37/02;** A61K 2039/505; C07K 2317/24; C07K 2317/33; C07K 2317/41; C07K 2317/567; C07K 2317/72; C07K 2317/732; C07K 2317/76;

(Cont.)

(54) **BCMA (CD269/TNFRSF17) - BINDING PROTEINS**

BCMA (CD269/TNFRSF17)-BINDENDE PROTEINE

PROTÉINES DE LIAISON À BCMA (CD269/TNFRSF17)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **27.05.2011 US 201161490732 P**
**15.05.2012 US 201261647196 P**

(43) Date of publication of application:
**19.12.2018 Bulletin 2018/51**

(60) Divisional application:
**23183917.6**

(62) Document number(s) of the earlier application(s) in accordance with Art. 76 EPC:
**12723206.4 / 2 714 737**

(73) Proprietor: **Glaxo Group Limited**
**Stevenage SG1 2NY (GB)**

(72) Inventors:
- **ALGATE, Paul**
**Issaquah, WA (US)**
- **CLEGG, Stephanie Jane**
**Stevenage, Hertfordshire SG1 2NY (GB)**
- **CRAIGEN, Jennifer L.**
**Stevenage, Hertfordshire SG1 2NY (GB)**
- **HAMBLIN, Paul Andrew**
**Stevenage, Hertfordshire SG1 2NY (GB)**
- **LEWIS, Alan Peter**
**Stevenage, Hertfordshire SG1 2NY (GB)**
- **PARMAR, Radha Shah**
**Stevenage, Hertfordshire SG1 2NY (GB)**
- **MAYES, Patrick**
**Collegeville, PA 19426-0989 (US)**
- **WATTAM, Trevor Anthony Kenneth**
**Stevenage, Hertfordshire SG1 2NY (GB)**

(74) Representative: **EIP**
**Fairfax House**
**15 Fulwood Place**
**London WC1V 6HU (GB)**

(56) References cited:
**WO-A1-2012/066058 WO-A2-2010/104949**
**WO-A2-2011/108008**

- **RYAN MAUREEN C ET AL: "Antibody targeting of B-cell maturation antigen on malignant plasma cells", MOLECULAR CANCER THERAPEUTICS, AMERICAN ASSOCIATION OF CANCER RESEARCH, US, vol. 6, no. 11, 1 November 2007 (2007-11-01), pages 3009-3018, XP002581270, ISSN: 1535-7163**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **YAMANE-OHNUKI N ET AL: "Establishment of FUT8 knockout chinese hanster ovary cells: an ideal host cell line for producing completely defucosylated antibodies with enhanced antibody-dependent cellular cytotoxicity", BIOTECHNOLOGY AND BIOENGINEERING, WILEY & SONS, HOBOKEN, NJ, US, vol. 87, no. 5, 6 August 2004 (2004-08-06) , pages 614-622, XP002983758, ISSN: 0006-3592, DOI: 10.1002/BIT.20151**
- **C. PETERS ET AL: "Antibody-drug conjugates as novel anti-cancer chemotherapeutics", BIOSCIENCE REPORTS, vol. 35, no. 4, 12 June 2015 (2015-06-12), pages e00225-e00225, XP055301629, US ISSN: 0144-8463, DOI: 10.1042/BSR20150089**


Remarks:

The complete document including Reference Table(s) and the Sequence Listing(s) can be downloaded from the EPO website

(52) Cooperative Patent Classification (CPC): (Cont.)
C07K 2317/77; C07K 2317/92

## Description

Field of the invention

The present invention relates to antigen binding proteins and fragments thereof that specifically bind B cell maturation antigen (BCMA) and in particular human BCMA (hBCMA).

The present invention also concerns methods of treating diseases or disorders with said antigen binding fragments, pharmaceutical compositions comprising said antigen binding fragments and methods of manufacture. Other embodiments of the present invention will be apparent from the description below.

Background of the invention

BCMA (CD269 or TNFRSF17) is a member of the TNF receptor superfamily. It is a non-glycosylated integral membrane receptor for the ligands BAFF and APRIL. BCMA's ligands can also bind additional receptors: TACI (Transmembrane Activator and Calcium modulator and cyclophilin ligand Interactor), which binds APRIL and BAFF; as well as BAFF-R (BAFF Receptor or BR3), which shows restricted but high affinity for BAFF. Together, these receptors and their corresponding ligands regulate different aspects of humoral immunity, B-cell development and homeostasis.

BCMA's expression is typically restricted to the B-cell lineage and is reported to increase in terminal B-cell differentiation. BCMA is expressed by human plasma blasts, plasma cells from tonsils, spleen and bone marrow, but also by tonsillar memory B cells and by germinal centre B cells, which have a TACI-BAFFR low phenotype (Darce et al, 2007). BCMA is virtually absent on naive and memory B-cells (Novak et al., 2004a and b). The BCMA antigen is expressed on the cell surface so is accessible to the antibody, but is also expressed in the golgi. As suggested by its expression profile, BCMA signalling, typically linked with B-cell survival and proliferation, is important in the late stages of B-cell differentiation, as well as the survival of long lived bone marrow plasma cells (O'Connor et al., 2004) and plasmablasts (Avery et al., 2003). Furthermore, as BCMA binds APRIL with high affinity, the BCMA-APRIL signalling axis is suggested to predominate at the later stages of B-cell differentiation, perhaps being the most physiologically relevant interaction.

Multiple Myeloma (MM) is a clonal B-cell malignancy that occurs in multiple sites within the bone marrow before spreading to the circulation; either de novo, or as a progression from monoclonal gammopathy of undetermined significance (MGUS). It is commonly characterised by increases in paraprotein and osteoclast activity, as well as hypercalcaemia, cytopenia, renal dysfunction, hyperviscosity and peripheral neuropathy. Decreases in both normal antibody levels and numbers of neutrophils are also common, leading to a life threatening susceptibility to infection. BCMA has been implicated in the growth and survival of myeloma cell lines in vitro (Novak et al., 2004a and b; Moreaux et al., 2004).

BCMA expression (both transcript and protein) is reported to correlate with disease progression in MM. Using Affymetrix microarrays, it was demonstrated that the *TACI* and *BCMA* genes were overexpressed in Multiple Myeloma Cells (MMC) compared with their normal counterparts (Moreaux et al, 2004). Gene expression analysis has been used to compare human myeloma cells with purified plasma cells from patients with MGUS and from normal bone marrow as well as with primary tumour cells from B-cell lineage leukaemias (Bellucci et al, 2005). The BCMA gene was highly expressed in all myeloma samples. Although purified plasma cells from patients with MGUS had lower expression of BCMA, there was no significant difference when compared with the expression found in normal plasma cells or myeloma cells. In contrast, BCMA expression was significantly lower in B-cell Chronic Lymphocytic Leukaemia (CLL), pre-B Acute Lymphocytic Leukaemia (ALL) and T-cellALL (T-ALL). Mouse models that transgenically over-express BAFF or APRIL have a significant increase in B-cell lymphomas (Batten et al., 2004 - BAFF; Planelles et al., 2004 - APRIL). In humans, excess BAFF and APRIL have been detected in the sera and micro-environments of patients with a number of B-cell malignancies, as well as other B-cell disorders.

An anti-BCMA IgG1 antibody SG1 has been shown to block the APRIL-dependent activation of NFkB. This antibody apparently demonstrated cytotoxicity against multiple myeloma cells as both a naked antibody, or as antibody-drug conjugates with mono methyl auristatin F (MMAF). Enhanced ADCC (100-fold) was achieved though chimerisation with a human Fc region comprising mutations that enhance FcyRIIIA binding (Ryan Maureen et al: "Antibody targeting of B-cell maturation antigen on malignant plasma cells", Molecular Cancer Therapeutics, American Association of Cancer Research, US vol. 6, no. 11, 1 November 2007 (2007-11-01), pages 3009-3018, ISSN: 1535-7163) Chimeric (humanised) anti-BCMA IgG1 antibodies, including afucosylated versions thereof have also been disclosed previously and purported to have ADCC activity (WO 2010/104949 A2)

Brief Description of Figures

**Figure 1: FMAT Binding Assay** - Figure showing the results of the FMAT assay for CA8 antibody binding to human

and cyno BCMA expressing HEK293 cells. Human chimeric CA8 binds well to human and cyno BCMA expressing cells.

**Figure 2: ELISA Binding Assay** - Figure showing the ELISA results for CA8 antibodies binding to human and cyno BCMA recombinant proteins. This clearly shows that human chimeric CA8 antibodies bind to human and cyno BCMA proteins equally.

**Figure 3: BiaCore Binding Assay** - Figure showing the binding of CA8 to BCMA-Fc, TACI-Fc and BAFF-R-Fc proteins in the Biacore experiment. CA8 chimera antibody does not bind to TACI or BAFF-R proteins.

**Figure 4: Cell binding** assay - Figure showing binding of murine S307118G03, S3222110D07, S332121 F02 and S332126E04 to H929 multiple myeloma cells and S3322110D07, S332121 F02 and S332126E04 to the BCMA transfected ARH77 cells as determined by FACS.
Multiple myeloma cell line H929 or ARH77-hBCMA 10B5 BCMA expressing transfectant cells were stained with either murine anti BCMA antibodies (solid histogram) or murine IgG2a isotype control (open histograms). Cells were analysed by FACS to detect antibody bound to the cells.

**Figure 5: Cell binding assay** - Figure showing binding of chimeric CA8 to a panel of multiple myeloma cell lines as determined by FACS. Binding to H929, OPM-2, JJN-3 and U266 was tested by flow cytometry and mean fluorescence intensity (MFI) values measured to determine binding. Synagis was used as an irrelevant isotype control.

**Figure 6: Cell binding** assay - Figure showing binding curves of humanised CA8 variants to BCMA transfected ARH77 cells (A) and multiple myeloma H929 cells (B) as determined by FACS.
Humanised variants J6M0, J6M1, J6M2, J9M0, J9M1 and J9M2 were tested by flow cytometry and mean fluorescence intensity (MFI) values measured to determine binding compared to the CA8 chimera.

**Figure 7: Ligand neutralisation assays** -

(A **and** B) Figure showing the ability of CA8 and J6M0 to neutralise binding of recombinant BAFF or APRIL to recombinant BCMA coated on an ELISA plate. OD values were used to calculate the antibody mediated inhibition of the maximal signal achieved by the relevant ligand alone binding to recombinant BCMA. Data is reported as percentage inhibition of the maximal signal. Antibodies tested were chimeric CA8 and humanised CA8 version J6M0 in both wild type and afucosylated (Potelligent) form.
**(A)** Neutralisation of BAFF ligand binding; **(B)-** Neutralisation APRIL ligand binding.
**(C)** - Figure showing the ability of J6M0 BCMA antibody in inhibition of BAFF or APRIL induced phosphorylation of NFKappaB in H929 cells. H-929 cells were washed 3 times to remove any sBCMA and resuspended in serum free medium. J6M0 potelligent antibody was added to a 96 well plate to give a final well concentrations up to 100ug/ml along with BAFF or APRIL ligand to give a final well concentration of 0.6 or 0.2 ug/ml respectively. H-929 cells were then plated at 7.5x104cells/well in serum free medium. 30 minutes later the cells were lysed and phosphorylated NFkappaB levels measured using a MSD pNFkappaB assay. MSD reader 502819.This is data from one independent experiments. Each data point is the mean/sd of two replicates.

**Figure 8: ADCC assay** - Figure showing ADCC activity of chimeric CA8 and defucosylated (Fc enhanced) CA8 with target cells expressing BCMA.
Human NK cells were incubated with europium labelled ARH77 10B5 BCMA transfected target cells in the presence of varying concentrations of antibody. Europium release from the target cells was measured and specific lysis calculated. **(A)** ADCC dose response curves of chimeric CA8 compared to isotype control. **(B)** ADCC dose response curves for chimeric CA8 and defucosylated chimeric CA8 (Fc enhanced), against the BCMA expressing cell line ARH77 10B5.

**Figure 9: ADCC assay** - Figure showing ADCC assay on CA8 humanised antibodies using ARH77 BCMA expressing target cells.
Human PBMC were incubated with europium labelled ARH77 BCMA transfected target cells in the presence of a range of concentrations of the J5, J6, J7, J8 or J9 series of humanised CA8 antibodies. Europium release from the target cells was measured and specific lysis calculated. EC50 values are shown in ug/ml.

**Figure 10: ADCC assay** - Figure showing ADCC activity of chimeric, S332121 F02 **(A),** S33221 10D07 **(B)** S307118G03 **(C)** and humanised S307118G03 H3L0 **(D)** against ARH7710B5 target cells with purified NK cells as

effector cells. Human NK target cells were incubated with europium labelled ARH77 10B5 BCMA transfected target cells in the presence of varying concentrations of antibody. Europium release from the target cells was measured and specific lysis calculated.

**Figure 11: Viability assay dose response curves** - Figure showing dose response curves in a cell viability assay for chimeric CA8 antibody, chimeric CA8-vcMMAE and chimeric CA8-mcMMAF antibody-drug conjugates in human multiple myeloma cell lines **(A)** NCI-H929 **(B)** U266-B1 **(C)** JJN3 and **(D)** OPM2. Antibody was added to the cells and the number of viable cells after 96 hours measured using CelltiterGlo.Data points represent the mean of triplicate CellTiterGlo measurements. Error bars represent standard error.

**Figure 12: Impact of CA8 chimeric antibody on cell cycle.**

**(A)** Cell cycle histograms of NCI-H929 cells treated with unconjugated chimeric CA8, chimeric CA8-vcMMAE ADC or chimeric CA8-mcMMAF ADC at 50ng/mL for the timepoints indicated. Pactitaxel (100nM) was used as a positive control for G2/M cell cycle arrest and cell death. Control human IgG1 was used as a negative control. Cell cycle analysis was carried out at the times shown on the graphs.
**(B)** Quantification of the 4N DNA cell population indicative of G2/M arrest and **(C)** sub-2N DNA cell population indicative of cell death for each of the treatments indicated. Cells were seeded in 12-well plates ($2\times10^5$ cells per well in 1mL of RPMI + 10% FBS). Antibody or ADC was added 6 hours after cell seeding.

**Figure 13: Impact of chimeric CA8 on phospho-histone H3.**
Chimeric CA8 ADC treatment results in increased phospho-Histone H3 staining of NCI-H929 cells. **(A,B)** Dot plots of cells stained with propidium iodide to measure DNA content (FL3-H) x-axis and anti-phospho-Histone H3 (Thr11) antibody (FL1-H) y-axis after treatment with either Control IgG **(A)** or chimeric CA8-mcMMAF **(B). (C)** Quantification of phospho-Histone H3 positive NCI-H929 cells after a 48 hour treatment with the indicated concentrations of chimeric CA8 ADCs. Pactitaxel (100nM) was used as a positive control for mitotic arrest and control chimera IgG1 was used as a negative control. Cells were seeded in 12-well plates ($2\times10^5$ cells per well in 1mL of RPMI + 10% FBS). Antibody or ADC was added 6 hours after cell seeding.

**Figure 14: Impact of chimeric CA8 on Annexin-V.**
Chimeric CA8 ADC treatment results in increased Annexin-V staining of NCI-H929 cells. **(A)** Histograms of Annexin-V-FITC (FL1-H; top panels) and Live cell propidium iodide staining (FL3-H; bottom panels) after treatment with increasing concentrations of chimeric CA8 ADCs **(B)** Quantification of Annexin-V positive NCI-H929 cells after a 96 hour treatment with the indicated concentrations of chimeric CA8 ADCs. Pactitaxel (100nM) was used as a positive control for apoptosis and control chimera IgG1 was used as a negative control. Cells were seeded in 12-well plates ($2\times10^5$ cells per well in 1mL of RPMI + 10% FBS). Antibody or ADC was added 6 hours after cell seeding.

**Figure 15: Viability assay dose response curves** - Figure showing dose response curves for the unconjugated (Naked) and vcMMAE and mcMMAF antibody-drug conjugates of chimeric CA8 or humanized J6M0 antibodies. Antibody drug conjugates were tested against human multiple myeloma cell lines NCl-H929 and OPM2.

**Figure 16: Viability assay dose response curves** - Figure showing dose response curves for the unconjugated antibodies, vcMMAE and mcMMAF antibody-drug conjugates of murine anti-BCMA antibodies S332121F02, S322110D07, S332126E04 and S307118G03 in human multiple myeloma cell lines NCl-H929 and U266-B1.

**Figure 17 ADCC activity of ADC J6M0 molecules** - Figure showing ADCC assay on J6M0 antibodies using ARH77 BCMA expressing target cells. Human PBMC were incubated with europium labelled ARH77 BCMA transfected target cells in the presence of a range of concentrations of J6M0 WT and potelligent BCMA antibodies conjugated to MMAE, MMAF, or unconjugated Europium release was monitored on the Victor 2 1420 multilabel reader.

**Figure 18 ADCC dose response curves of CA8 J6M0 Potelligent against a panel of 5 multiple myeloma lines** - Human PBMC were incubated with multiple myeloma target cells in the presence of varying concentrations of CA8 J6M0 potelligent antibody at an E:T ratio of 50:1 for 18 hours. The percentage of target cells remaining in the effecter plus target mixture was then measured by FACS using a fluorescently labelled anti-CD138 antibody to detect the target cells and the percent cytotoxicity calculated. A) Example dose response curves for CA8 J6M0 potelligent against the five multiple myeloma cell lines tested. Each data point is from a singlicate value.

**Figure 19 Effect of dose escalation of J6M0 and drug conjugated J6M0 on the growth and establishment of**

**NCI-H929 cells in CB.17 SCID mice** Calculated tumour volumes of NCI-H929 tumours in CB17 SCID mice following twice weekly intraperitoneal dosing of either 50 or 100ug J6M0 anti-BCMA or IgG1 isotype control unconjugated, or conjugated to MMAE or MMAF for 2 weeks. Data points represent mean tumour volume of n=5 per group

**Figure 20- Determination of soluble BCMA levels in serum from healthy volunteers and myeloma patients.** Serum samples were collected from MM patient samples were from a variety of stages (progressive disease, remission, relapsed, newly diagnosed, and others). The samples shown in the figure are those from serum diluted 1/500 prior to the assay.
A Human BCMA/TNFRSF17 sandwich ELISA kit from R& D Systems which measures soluble human BCMA levels was used to detect BCMA following the standard protocol provided with the kit.

Summary of the Invention

The invention is defined by the appended claims.
Any references in the description to methods of treatment refer to the compounds, pharmaceutical compositions and medicaments of the present invention for use in a method for treatment of the human (or animal) body by therapy (or for diagnosis).

Detailed Description of the Invention

The present invention provides an anti B-Cell Maturation Antigen (CD269) antibody comprising a heavy chain variable region of SEQ. ID. NO:23 and a light chain variable region of SEQ. ID. NO:31.
Further provided is an anti B-Cell Maturation Antigen (CD269) antibody which comprises a heavy chain of SEQ. ID. NO:55 and a light chain of SEQ. ID. NO:63. In one aspect the anti B-Cell Maturation Antigen (CD269) antibody of the invention has enhanced binding to FcγRIIIA or has enhanced FcyRIIIA mediated effector function, for example in one aspect of the invention as herein provided the antibody is defucosylated.
Herein disclosed are antigen binding proteins which bind to membrane bound targets and wherein the antigen binding protein is capable of internalisation. In a further embodiment there is provided an immunoconjugate comprising the antigen binding protein of the present invention and a cytotoxic agent. In a further embodiment the antigen binding protein has ADCC effector function for example the antigen binding protein has enhanced ADCC effector function.
In one such embodiment there is provided antigen binding proteins or fragments thereof which specifically bind to BCMA, for example which specifically binds human BCMA (hBCMA) and which inhibit the binding of BAFF and/or APRIL to the BCMA receptor.
In a further embodiment the antigen binding proteins or fragments specifically bind to BCMA and inhibit the binding of BAFF and/or APRIL to BCMA wherein the antigen binding proteins or fragments thereof have the ability to bind to FcγRIIIA and mediate FcgRIIIA mediated effector functions, or have enhanced FcγRIIIA mediated effector function. In one embodiment of the invention as herein provided the antigen binding proteins are capable of internalisation.
In one aspect of the invention there is provided an antigen binding protein according to the invention as herein described which binds to non-membrane bound BCMA, for example to serum BCMA.
In one aspect of the invention the antgen binding protein has enhanced effector function. In another aspect the antigen binding protein is conjugated to a cytotoxic agent. In yet a further embodiment the antigen binding protein has both enhanced effector function and is conjugated to a cytotoxic agent.
The antigen binding proteins of the present invention may comprise heavy chain variable regions and light chain variable regions of the invention which may be formatted into the structure of a natural antibody or functional fragment or equivalent thereof. An antigen binding protein of the invention may therefore comprise the VH regions of the invention formatted into a full length antibody, a (Fab')2 fragment, a Fab fragment, or equivalent thereof (such as scFV, bi- tri- or tetra-bodies, Tandabs etc.), when paired with an appropriate light chain. The antibody may be an IgG1, IgG2, IgG3, or IgG4; or IgM; IgA, IgE or IgD or a modified variant thereof. The constant domain of the antibody heavy chain may be selected accordingly. The light chain constant domain may be a kappa or lambda constant domain. Furthermore, the antigen binding protein may comprise modifications of all classes e.g. IgG dimers, Fc mutants that no longer bind Fc receptors or mediate C1q binding. The antigen binding protein may also be a chimeric antibody of the type described in WO86/01533 which comprises an antigen binding region and a non-immunoglobulin region.
The constant region is selected according to any functionality required e.g. an IgG1 may demonstrate lytic ability through binding to complement and/or will mediate ADCC (antibody dependent cell cytotoxicity).
In one aspect the antigen binding protein is an antibody or antigen binding fragment thereof comprising one or more CDR's according to the invention described herein, or one or both of the heavy or light chain variable domains according to the invention described herein. In one embodiment the antigen binding protein binds primate BCMA. In one such embodiment the antigen binding protein additionally binds non-human primate BCMA, for example cynomolgus

macaque monkey BCMA.

In another aspect the antigen binding protein is selected from the group consisting of a dAb, Fab, Fab', $F(ab')_2$, Fv, diabody, triabody, tetrabody, miniantibody, and a minibody.

In one aspect of the present invention the antigen binding protein is a humanised or chimaeric antibody, in a further aspect the antibody is humanised.

In one aspect the antibody is a monoclonal antibody.

In another aspect the antigen binding protein binds to human BCMA with high affinity for example when measured by Biacore the antigen binding protein binds to human BCMA with an affinity of 20nM or less or an affinity of 15nM or less or an affinity of 5nM or less or an affinity of 1000 pM or less or an affinity of 500pM or less or an affinity of 400pM or less, or 300pM or less or for example about 120pM. In a further embodiment the antigen binding protein binds to human BCMA when measured by Biacore of between about 100pM and about 500pM or between about 100pM and about 400pM, or between about 100pM and about 300pM. In one embodiment of the present invention the antigen binding protein binds BCMA with an affinity of less than 150pm.

In one such embodiment, this is measured by Biacore, for example as set out in Example 4.

In another aspect the antigen binding protein binds to human BCMA and neutralises the binding of the ligands BAFF and/or APRIL to the BCMA receptor in a cell neutralisation assay wherein the antigen binding protein has an IC50 of between about 1 nM and about 500nM, or between about 1 nM and about 100nM, or between about 1 nM and about 50nM, or between about 1 nM and about 25nM, or between about 5nM and about 15nM. In a further embodiment of the present invention the antigen binding protein binds BCMA and neutralises BCMA in a cell neutralisation assay wherein the antigen binding protein has an IC50 of about 10nM.

In one such embodiment, this is measured by a cell neutralisation assay, for example as set out in Example 4.6.

The antigen binding proteins, for example antibodies of the present invention may be produced by transfection of a host cell with an expression vector comprising the coding sequence for the antigen binding protein of the invention. An expression vector or recombinant plasmid is produced by placing these coding sequences for the antigen binding protein in operative association with conventional regulatory control sequences capable of controlling the replication and expression in, and/or secretion from, a host cell. Regulatory sequences include promoter sequences, e.g., CMV promoter, and signal sequences which can be derived from other known antibodies. Similarly, a second expression vector can be produced having a DNA sequence which encodes a complementary antigen binding protein light or heavy chain. In certain embodiments this second expression vector is identical to the first except insofar as the coding sequences and selectable markers are concerned, so to ensure as far as possible that each polypeptide chain is functionally expressed. Alternatively, the heavy and light chain coding sequences for the antigen binding protein may reside on a single vector.

A selected host cell is co-transfected by conventional techniques with both the first and second vectors (or simply transfected by a single vector) to create the transfected host cell of the invention comprising both the recombinant or synthetic light and heavy chains. The transfected cell is then cultured by conventional techniques to produce the engineered antigen binding protein of the invention. The antigen binding protein which includes the association of both the recombinant heavy chain and/or light chain is screened from culture by appropriate assay, such as ELISA or RIA. Similar conventional techniques may be employed to construct other antigen binding proteins.

Suitable vectors for the cloning and subcloning steps employed in the methods and construction of the compositions of this invention may be selected by one of skill in the art. For example, the conventional pUC series of cloning vectors may be used. One vector, pUC19, is commercially available from supply houses, such as Amersham (Buckinghamshire, United Kingdom) or Pharmacia (Uppsala, Sweden). Additionally, any vector which is capable of replicating readily, has an abundance of cloning sites and selectable genes (e.g., antibiotic resistance), and is easily manipulated may be used for cloning. Thus, the selection of the cloning vector is not a limiting factor in this invention.

The expression vectors may also be characterized by genes suitable for amplifying expression of the heterologous DNA sequences, e.g., the mammalian dihydrofolate reductase gene (DHFR). Other vector sequences include a poly A signal sequence, such as from bovine growth hormone (BGH) and the betaglobin promoter sequence (betaglopro). The expression vectors useful herein may be synthesized by techniques well known to those skilled in this art.

The components of such vectors, e.g. replicons, selection genes, enhancers, promoters, signal sequences and the like, may be obtained from commercial or natural sources or synthesized by known procedures for use in directing the expression and/or secretion of the product of the recombinant DNA in a selected host. Other appropriate expression vectors of which numerous types are known in the art for mammalian, bacterial, insect, yeast, and fungal expression may also be selected for this purpose.

The present invention also encompasses a cell line transfected with a recombinant plasmid containing the coding sequences of the antigen binding proteins of the present invention. Host cells useful for the cloning and other manipulations of these cloning vectors are also conventional. However, cells from various strains of E. Coli may be used for replication of the cloning vectors and other steps in the construction of antigen binding proteins of this invention.

Suitable host cells or cell lines for the expression of the antigen binding proteins of the invention include

mammalian cells such as NS0, Sp2/0, CHO (e.g. DG44), COS, HEK, a fibroblast cell (e.g., 3T3), and myeloma cells, for example it may be expressed in a CHO or a myeloma cell. Human cells may be used, thus enabling the molecule to be modified with human glycosylation patterns. Alternatively, other eukaryotic cell lines may be employed. The selection of suitable mammalian host cells and methods for transformation, culture, amplification, screening and product production and purification are known in the art. See, e.g., Sambrook et al., cited above.

Bacterial cells may prove useful as host cells suitable for the expression of the recombinant Fabs or other embodiments of the present invention (see, e.g., Plückthun, A., Immunol. Rev., 130:151-188 (1992)). However, due to the tendency of proteins expressed in bacterial cells to be in an unfolded or improperly folded form or in a non-glycosylated form, any recombinant Fab produced in a bacterial cell would have to be screened for retention of antigen binding ability. If the molecule expressed by the bacterial cell was produced in a properly folded form, that bacterial cell would be a desirable host, or in alternative embodiments the molecule may express in the bacterial host and then be subsequently re-folded. For example, various strains of E. Coli used for expression are well-known as host cells in the field of biotechnology. Various strains of B. Subtilis, Streptomyces, other bacilli and the like may also be employed in this method.

Where desired, strains of yeast cells known to those skilled in the art are also available as host cells, as well as insect cells, e.g. Drosophila and Lepidoptera and viral expression systems. See, e.g. Miller et al., Genetic Engineering, 8:277-298, Plenum Press (1986) and references cited therein.

The general methods by which the vectors may be constructed, the transfection methods required to produce the host cells of the invention, and culture methods necessary to produce the antigen binding protein of the invention from such host cell may all be conventional techniques. Typically, the culture method of the present invention is a serum-free culture method, usually by culturing cells serum-free in suspension. Likewise, once produced, the antigen binding proteins of the invention may be purified from the cell culture contents according to standard procedures of the art, including ammonium peroxide precipitation, affinity columns, column chromatography, gel electrophoresis and the like. Such techniques are within the skill of the art and do not limit this invention. For example, preparations of altered antibodies are described in WO 99/58679 and WO 96/16990.

Yet another method of expression of the antigen binding proteins may utilize expression in a transgenic animal, such as described in U. S. Patent No. 4,873,316. This relates to an expression system using the animal's casein promoter which when transgenically incorporated into a mammal permits the female to produce the desired recombinant protein in its milk.

In a further embodiment of the invention there is provided a method of producing an antibody of the invention which method comprises the step of culturing a host cell transformed or transfected with a vector encoding the light and/or heavy chain of the antibody of the invention and recovering the antibody thereby produced.

In accordance with the present invention there is provided a method of producing an anti-BCMA antibody of the present invention which binds to and neutralises the activity of human BCMA which method comprises the steps of;

providing a first vector encoding a heavy chain of the antibody;
providing a second vector encoding a light chain of the antibody;
transforming a mammalian host cell (e.g. CHO) with said first and second vectors;
culturing the host cell of step (c) under conditions conducive to the secretion of the antibody from said host cell into said culture media;
recovering the secreted antibody of step (d).

Once expressed by the desired method, the antibody is then examined for in vitro activity by use of an appropriate assay. Presently conventional ELISA assay formats are employed to assess qualitative and quantitative binding of the antibody to BCMA. Additionally, other in vitro assays may also be used to verify neutralizing efficacy prior to subsequent human clinical studies performed to evaluate the persistence of the antibody in the body despite the usual clearance mechanisms.

The dose and duration of treatment relates to the relative duration of the molecules of the present invention in the human circulation, and can be adjusted by one of skill in the art depending upon the condition being treated and the general health of the patient. It is envisaged that repeated dosing (e.g. once a week or once every two weeks or once every 3 weeks) over an extended time period (e.g. four to six months) maybe required to achieve maximal therapeutic efficacy.

In one embodiment of the present invention there is provided a recombinant transformed, transfected or transduced host cell comprising at least one expression cassette, for example where the expression cassette comprises a polynucleotide encoding a heavy chain of an antigen binding protein according to the invention described herein and further comprises a polynucleotide encoding a light chain of an antigen binding protein according to the invention described herein or where there are two expression cassettes and the 1st encodes the light chain and the second encodes the heavy chain. For example in one embodiment the first expression cassette comprises a polynucleotide encoding a heavy chain of an antigen binding protein comprising a constant region or antigen binding fragment thereof which is linked to

a constant region according to the invention described herein and further comprises a second cassette comprising a polynucleotide encoding a light chain of an antigen binding protein comprising a constant region or antigen binding fragment thereof which is linked to a constant region according to the invention described herein for example the first expression cassette comprises a polynucleotide encoding a heavy chain selected from SEQ. ID. NO:56, and a second expression cassette comprising a polynucleotide encoding a light chain selected from SEQ. ID. NO: 64

In another embodiment of the invention there is provided a stably transformed host cell comprising a vector comprising one or more expression cassettes encoding a heavy chain and/or a light chain of the antibody comprising a constant region or antigen binding fragment thereof which is linked to a constant region as described herein. For example such host cells may comprise a first vector encoding the light chain and a second vector encoding the heavy chain, for example the first vector encodes a heavy chain selected from SEQ. ID. NO: 55, and a second vector encoding a light chain for example the light chain of SEQ ID NO: 63.

In one such example the first vector encodes a heavy chain selected from SEQ. ID. NO: 55 and a second vector encoding a light chain for example the light chain of SEQ ID NO: 63.

In another embodiment of the present invention there is provided a host cell according to the invention described herein wherein the cell is eukaryotic, for example where the cell is mammalian. Examples of such cell lines include CHO or NS0.

In another embodiment of the present invention there is provided a method for the production of an antibody comprising a constant region or antigen binding fragment thereof which is linked to a constant region according to the invention described herein which method comprises the step of culturing a host cell in a culture media, for example serum- free culture media.

In another embodiment of the present invention there is provided a method according to the invention described herein wherein said antibody is further purified to at least 95% or greater (e.g. 98% or greater) with respect to said antibody containing serum- free culture media.

In yet another embodiment there is provided a pharmaceutical composition comprising an antigen binding protein and a pharmaceutically acceptable carrier.

In another embodiment of the present invention there is provided a kit-of-parts comprising the composition according to the invention described herein described together with instructions for use.

The mode of administration of the therapeutic agent of the invention may be any suitable route which delivers the agent to the host. The antigen binding proteins, and pharmaceutical compositions of the invention are particularly useful for parenteral administration, i.e., subcutaneously (s.c.), intrathecally, intraperitoneally, intramuscularly (i.m.) or intravenously (i.v.). In one such embodiment the antigen binding proteins of the present invention are administered intravenously or subcutaneously.

Therapeutic agents of the invention may be prepared as pharmaceutical compositions containing an effective amount of the antigen binding protein of the invention as an active ingredient in a pharmaceutically acceptable carrier. In one embodiment the prophylactic agent of the invention is an aqueous suspension or solution containing the antigen binding protein in a form ready for injection. In one embodiment the suspension or solution is buffered at physiological pH. In one embodiment the compositions for parenteral administration will comprise a solution of the antigen binding protein of the invention or a cocktail thereof dissolved in a pharmaceutically acceptable carrier. In one embodiment the carrier is an aqueous carrier. A variety of aqueous carriers may be employed, e.g., 0.9% saline, 0.3% glycine, and the like. These solutions may be made sterile and generally free of particulate matter. These solutions may be sterilized by conventional, well known sterilization techniques (e.g., filtration). The compositions may contain pharmaceutically acceptable auxiliary substances as required to approximate physiological conditions such as pH adjusting and buffering agents, etc. The concentration of the antigen binding protein of the invention in such pharmaceutical formulation can vary widely, i.e., from less than about 0.5%, usually at or at least about 1% to as much as about 15 or 20% by weight and will be selected primarily based on fluid volumes, viscosities, etc., according to the particular mode of administration selected.

Thus, a pharmaceutical composition of the invention for intravenous infusion could be made up to contain about 250 ml of sterile Ringer's solution, and about 1 to about 30 or 5 mg to about 25 mg of an antigen binding protein of the invention per ml of Ringer's solution. Actual methods for preparing parenterally administrable compositions are well known or will be apparent to those skilled in the art and are described in more detail in, for example, Remington's Pharmaceutical Science, 15th ed., Mack Publishing Company, Easton, Pennsylvania. For the preparation of intravenously administrable antigen binding protein formulations of the invention see Lasmar U and Parkins D "The formulation of Biopharmaceutical products", Pharma. Sci.Tech.today, page 129-137, Vol.3 (3rd April 2000); Wang, W "Instability, stabilisation and formulation of liquid protein pharmaceuticals", Int. J. Pharm 185 (1999) 129-188; Stability of Protein Pharmaceuticals Part A and B ed Ahern T.J., Manning M.C., New York, NY: Plenum Press (1992); Akers,M.J. "Excipient-Drug interactions in Parenteral Formulations", J.Pharm Sci 91 (2002) 2283-2300; Imamura, K et al "Effects of types of sugar on stabilization of Protein in the dried state", J Pharm Sci 92 (2003) 266-274; Izutsu, Kkojima, S. "Excipient crystalinity and its protein-structure-stabilizing effect during freeze-drying", J Pharm. Pharmacol, 54 (2002) 1033-1039;

Johnson, R, "Mannitol-sucrose mixtures-versatile formulations for protein peroxidise19g19n", J. Pharm. Sci, 91 (2002) 914-922; and Ha,E Wang W, Wang Y.j. "Peroxide formation in polysorbate 80 and protein stability", J. Pharm Sci, 91, 2252-2264,(2002)..

In one embodiment the therapeutic agent of the invention, when in a pharmaceutical preparation, is present in unit dose forms. The appropriate therapeutically effective dose will be determined readily by those of skill in the art. Suitable doses may be calculated for patients according to their weight, for example suitable doses may be in the range of about 0.1 to about 20mg/kg, for example about 1 to about 20mg/kg, for example about 10 to about 20mg/kg or for example about 1 to about 15mg/kg, for example about 10 to about 15mg/kg. To effectively treat conditions such as Multiple myeloma, SLE or IPT in a human, suitable doses may be within the range of about 0.1 to about 1000 mg, for example about 0.1 to about 500mg, for example about 500mg, for example about 0.1 to about 100mg, or about 0.1 to about 80mg, or about 0.1 to about 60mg, or about 0.1 to about 40mg, or for example about 1 to about 100mg, or about 1 to about 50mg, of an antigen binding protein of this invention, which may be administered parenterally, for example subcutaneously, intravenously or intramuscularly. Such dose may, if necessary, be repeated at appropriate time intervals selected as appropriate by a physician.

The antigen binding proteins described herein can be lyophilized for storage and reconstituted in a suitable carrier prior to use. This technique has been shown to be effective with conventional immunoglobulins and art-known peroxidise and reconstitution techniques can be employed.

In another aspect of the invention there is provided an antigen binding protein as herein described for use in a medicament.

In one aspect of the disclosure there is provided an antigen binding protein according to the invention as herein described for use in the treatment of rheumatoid arthitis, Type 1 Diabetes Mellitus, multiple sclerosis or psoriasis wherein said method comprises the step of administering to said patient a therapeutically effective amount of the antigen binding protein as described herein.

In one embodiment of the present disclosure, methods are provided for treating cancer in a human comprising administering to said human an antigen binding protein that specifically binds to BCMA. In some instances the antigen binding protein is part of an immunoconjugate.

In another aspect of the present invention there is provided an antigen binding protein according to the invention as herein described for use in the treatment of a B-cell mediated or plasma cell mediated disease or antibody mediated disease or disorder selected from Multiple Myeloma (MM), chronic lymphocytic leukemia (CLL), Non-secretory multiple myeloma, Smoldering multiple myeloma, Monoclonal gammopathy of undetermined significance (MGUS), Solitary plasmacytoma (Bone, Extramedullary), Lymphoplasmacytic lymphoma (LPL), Waldenstrom's Macroglobulinemia, Plasma cell leukemia,, Primary Amyloidosis (AL), Heavy chain disease, Systemic lupus erythematosus (SLE), POEMS syndrome / osteosclerotic myeloma, Type I and II cryoglobulinemia, Light chain deposition disease, Goodpasture's syndrome, Idiopathic thrombocytopenic purpura (ITP), Acute glomerulonephritis, Pemphigus and Pemphigoid disorders, and Epidermolysis bullosa acquisita; or any Non-Hodgkin's Lymphoma B-cell leukemia or Hodgkin's lymphoma (HL) with BCMA expression or any diseases in which patients develop neutralising antibodies to recombinant protein replacement therapy wherein said method comprises the step of administering to said patient a therapeutically effective amount of the antigen binding protein as described herein.

B-cell disorders can be divided into defects of B-cell development/immunoglobulin production (immunodeficiencies) and excessive/uncontrolled proliferation (lymphomas, leukemias). As used herein, B-cell disorder refers to both types of diseases, and methods are provided for treating B-cell disorders with an antigen binding protein.

In a particular aspect, the disease or disorder is selected from the group consisting of Multiple Myeloma (MM), Chronic Lymphocytic Leukaemia (CLL), Solitary Plasmacytoma (Bone, Extramedullary), Waldenstrom's Macroglobulinemia.

In one aspect of the present invention the disease is Multiple Myeloma, Smoldering Multiple Myeloma (SMM) or Solitary Plasmacytoma (Bone, Extramedullary).

In one aspect of the present invention the disease is Multiple Myeloma.

In one aspect of the present disclosure the disease is Systemic lupus erythematosus (SLE)

In one aspect of the present disclosure the disease is Idiopathic thrombocytopenic purpura (ITP) Use of the antigen binding protein as described herein in the manufacture of a medicament for the treatment of diseases and disorders as described herein is also provided.

In another aspect of the disclosure there is provided the use of the antigen binding protein as described herein for use in the treatment or prophylaxis of an antibody mediated or plasma cell mediated disease or disorder selected from rheumatoid arthitis, Type 1 Diabeted Mellitus, multiple sclerosis or psoriasis.

In another aspect of the disclosure there is provided the use of the antigen binding protein as described herein for use in the treatment or prophylaxis of an antibody mediated or plasma cell mediated disease or disorder selected from Multiple Myeloma (MM), chronic lymphocytic leukemia (CLL), Monoclonal gammopathy of undetermined significance (MGUS), Smoldering multiple myeloma (SMM), Solitary Plasmacytoma (Bone, Extramedullary), Waldenstrom's

Macroglobulinemia , Primary Amyloidosis (AL), Heavy chain disease, Systemic lupus erythematosus (SLE), POEMS syndrome / osteosclerotic myeloma, Type I and II cryoglobulinemia, Light chain deposition disease, Goodpastures syndrome, Idiopathic thrombocytopenic purpura (ITP), Acute glomerulonephritis, Pemphigus and Pemphigoid disorders and Epidermolysis bullosa acquisita, any Non-Hodgkin Lymphoma and Leukemia with BCMA expression or any diseases in which patients develop neutralising antibodies to recombinant protein replacement therapy wherein said method comprises the step of administering to said patient a therapeutically effective amount of the antigen binding protein as described herein.

In one aspect, the invention provides a pharmaceutical composition comprising an antigen binding protein of the present invention or a functional fragment thereof and a pharmaceutically acceptable carrier for treatment or prophylaxis of rheumatoid arthitis, Type 1 Diabetes Mellitus, multiple sclerosis or psoriasis or an antibody mediated or plasma cell mediated disease or disorder selected from selected from Multiple Myeloma (MM), chronic lymphocytic leukemia (CLL), Monoclonal gammopathy of undetermined significance (MGUS), Smoldering multiple myeloma (SMM), Solitary Plasmacytoma (Bone, Extramedullary), Waldenstrom's Macroglobulinemia , Primary Amyloidosis (AL), Heavy chain disease, Systemic lupus erythematosus (SLE), POEMS syndrome / osteosclerotic myeloma, Type I and II cryoglobulinemia, Light chain deposition disease, Goodpastures syndrome, Idiopathic thrombocytopenic purpura (ITP), Acute glomerulonephritis, Pemphigus and Pemphigoid disorders and Epidermolysis bullosa acquisita, any Non-Hodgkin Lymphoma and Leukemia with BCMA expression or any diseases in which patients develop neutralising antibodies to recombinant protein replacement therapy wherein said method comprises the step of administering to said patient a therapeutically effective amount of the antigen binding protein as described herein.

In another embodiment of the present disclosure there is provided a method of treating a human patient afflicted with rheumatoid arthitis, Type 1 Diabetes Mellitus, multiple sclerosis or psoriasis or an antibody mediated or plasma cell mediated disorder or disease which method comprises the step of administering a therapeutically effective amount of the antigen binding protein according to the invention as described herein.

In another aspect of the present disclosure there is provided an antigen binding protein according to the invention as herein described for use in the treatment of an antibody mediated or plasma cell mediated disease or disorder selected from Multiple Myeloma (MM), Chronic Lymphocytic Leukaemia (CLL)Monoclonal gammopathy of undetermined significance (MGUS), Smoldering multiple myeloma (SMM), Solitary Plasmacytoma (Bone, Extramedullary), Waldenstrom's Macroglobulinemia , Primary Amyloidosis (AL), Heavy chain disease, Systemic lupus erythematosus (SLE), POEMS syndrome / osteosclerotic myeloma, Type I and II cryoglobulinemia, Light chain deposition disease, Goodpastures syndrome, Idiopathic thrombocytopenic purpura (ITP), Acute glomerulonephritis, Pemphigus and Pemphigoid disorders and Epidermolysis bullosa acquisita, any Non-Hodgkin Lymphoma and Leukemia with BCMA expression or any diseases in which patients develop neutralising antibodies to recombinant protein replacement therapy wherein said method comprises the step of administering a pharmaceutical composition comprising an antigen binding protein according to the invention herein in combination with a pharmaceutically acceptable carrier.

In a further embodiment there is provided a method of treating a human patient afflicted with Multiple Myeloma (MM).

Definitions

As used herein, the terms "cancer," "neoplasm," and "tumor" are used interchangeably and, in either the singular or plural form, refer to cells that have undergone a malignant transformation that makes them pathological to the host organism. Primary cancer cells can be readily distinguished from noncancerous cells by well-established techniques, particularly histological examination. The definition of a cancer cell, as used herein, includes not only a primary cancer cell, but any cell derived from a cancer cell ancestor. This includes metastasized cancer cells, and in vitro cultures and cell lines derived from cancer cells. When referring to a type of cancer that normally manifests as a solid tumor, a "clinically detectable" tumor is one that is detectable on the basis of tumor mass; e.g., by procedures such as computed tomography (CT) scan, magnetic resonance imaging (MRI), X-ray, ultrasound or palpation on physical examination, and/or which is detectable because of the expression of one or more cancer-specific antigens in a sample obtainable from a patient. Tumors may be a hematopoietic (or hematologic or hematological or blood-related) cancer, for example, cancers derived from blood cells or immune cells, which may be referred to as "liquid tumors." Specific examples of clinical conditions based on hematologic tumors include leukemias such as chronic myelocytic leukemia, acute myelocytic leukemia, chronic lymphocytic leukemia and acute lymphocytic leukemia; plasma cell malignancies such as multiple myeloma, MGUS and Waldenstrom's macroglobulinemia; lymphomas such as non-Hodgkin's lymphoma, Hodgkin's lymphoma; and the like.

The cancer may be any cancer in which an abnormal number of blast cells or unwanted cell proliferation is present or that is diagnosed as a hematological cancer, including both lymphoid and myeloid malignancies. Myeloid malignancies include, but are not limited to, acute myeloid (or myelocytic or myelogenous or myeloblastic) leukemia (undifferentiated or differentiated), acute promyeloid (or promyelocytic or promyelogenous or promyeloblastic) leukemia,

acute myelomonocytic (or myelomonoblastic) leukemia, acute monocytic (or monoblastic) leukemia, erythroleukemia and megakaryocytic (or megakaryoblastic) leukemia. These leukemias may be referred together as acute myeloid (or myelocytic or myelogenous) leukemia (AML). Myeloid malignancies also include myeloproliferative disorders (MPD) which include, but are not limited to, chronic myelogenous (or myeloid) leukemia (CML), chronic myelomonocytic leukemia (CMML), essential thrombocythemia (or thrombocytosis), and polcythemia vera (PCV). Myeloid malignancies also include myelodysplasia (or myelodysplastic syndrome or MDS), which may be referred to as refractory anemia (RA), refractory anemia with excess blasts (RAEB), and refractory anemia with excess blasts in transformation (RAEBT); as well as myelofibrosis (MFS) with or without agnogenic myeloid metaplasia.

Hematopoietic cancers also include lymphoid malignancies, which may affect the lymph nodes, spleens, bone marrow, peripheral blood, and/or extranodal sites. Lymphoid cancers include B-cell malignancies, which include, but are not limited to, B-cell non-Hodgkin's lymphomas (B-NHLs). B-NHLs may be indolent (or low-grade), intermediate-grade (or aggressive) or high-grade (very aggressive). Indolent B-cell lymphomas include follicular lymphoma (FL); small lymphocytic lymphoma (SLL); marginal zone lymphoma (MZL) including nodal MZL, extranodal MZL, splenic MZL and splenic MZL with villous lymphocytes; lymphoplasmacytic lymphoma (LPL); and mucosaassociated-lymphoid tissue (MALT or extranodal marginal zone) lymphoma. Intermediate-grade B-NHLs include mantle cell lymphoma (MCL) with or without leukemic involvement, diffuse large cell lymphoma (DLBCL), follicular large cell (or grade 3 or grade 3B) lymphoma, and primary mediastinal lymphoma (PML). High-grade B-NHLs include Burkitt's lymphoma (BL), Burkitt-like lymphoma, small non-cleaved cell lymphoma (SNCCL) and lymphoblastic lymphoma. Other B-NHLs include immunoblastic lymphoma (or immunocytoma), primary effusion lymphoma, HIV associated (or AIDS related) lymphomas, and post-transplant lymphoproliferative disorder (PTLD) or lymphoma. B-cell malignancies also include, but are not limited to, chronic lymphocytic leukemia (CLL), prolymphocytic leukemia (PLL), Waldenstrom's macroglobulinemia (WM), hairy cell leukemia (HCL), large granular lymphocyte (LGL) leukemia, acute lymphoid (or lymphocytic or lymphoblastic) leukemia, and Castleman's disease. NHL may also include T-cell non-Hodgkin's lymphoma s(T-NHLs), which include, but are not limited to T-cell non-Hodgkin's lymphoma not otherwise specified (NOS), peripheral T-cell lymphoma (PTCL), anaplastic large cell lymphoma (ALCL), angioimmunoblastic lymphoid disorder (AILD), nasal natural killer (NK) cell / T-cell lymphoma, gamma/delta lymphoma, cutaneous T cell lymphoma, mycosis fungoides, and Sezary syndrome.

Hematopoietic cancers also include Hodgkin's lymphoma (or disease) including classical Hodgkin's lymphoma, nodular sclerosing Hodgkin's lymphoma, mixed cellularity Hodgkin's lymphoma, lymphocyte predominant (LP) Hodgkin's lymphoma, nodular LP Hodgkin's lymphoma,and lymphocyte depleted Hodgkin's lymphoma. Hematopoietic cancers also include plasma cell diseases or cancers such as multiple myeloma (MM) including smoldering MM, monoclonal gammopathy of undetermined (or unknown or unclear) significance (MGUS), plasmacytoma (bone, extramedullary), lymphoplasmacytic lymphoma (LPL), Waldenstrom's Macroglobulinemia, plasma cell leukemia, and primary amyloidosis (AL). Hematopoietic cancers may also include other cancers of additional hematopoietic cells, including polymorphonuclear leukocytes (or neutrophils), basophils, eosinophils, dendritic cells, platelets, erythrocytes and natural killer cells. Tissues which include hematopoietic cells referred herein to as "hematopoietic cell tissues" include bone marrow; peripheral blood; thymus; and peripheral lymphoid tissues, such as spleen, lymph nodes, lymphoid tissues associated with mucosa (such as the gut-associated lymphoid tissues), tonsils, Peyer's patches and appendix, and lymphoid tissues associated with other mucosa, for example, the bronchial linings.

The term "antigen binding protein" as used herein refers to antibodies, antibody fragments and other protein constructs which are capable of binding to and neutralising human BCMA.

The terms Fv, Fc, Fd, Fab, or F(ab)2 are used with their standard meanings (see, e.g., Harlow et al., Antibodies A Laboratory Manual, Cold Spring Harbor Laboratory, (1988)).

The term "antibody" is used herein in the broadest sense and specifically covers monoclonal antibodies (including full length monoclonal antibodies), polyclonal antibodies, multispecific antibodies (e.g. bispecific antibodies)

The term "monoclonal antibody" as used herein refers to an antibody obtained from a population of substantially homogenous antibodies i.e. the individual antibodies comprising the population are identical except for possible naturally occurring mutations that may be present in minor amounts. Monoclonal antibodies are highly specific being directed against a single antigenic binding site. Furthermore, in contrast to polyclonal antibody preparations which typically include different antibodies directed against different determinants (epitopes), each monoclonal antibody is directed against a single determinant on the antigen.

A "chimeric antibody" refers to a type of engineered antibody in which a portion of the heavy and/ or light chain is identical with or homologous to corresponding sequences in antibodies derived from a particular donor antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (US Patent No. 4, 816,567 *and* Morrison et al. Proc. Natl. Acad. Sci. USA 81:6851-6855) (1984*)).*

A "humanised antibody" refers to a type of engineered antibody having its CDRs derived from a non-human donor immunoglobulin, the remaining immunoglobulin-derived parts of the molecule being derived from one (or more)

human immunoglobulin(s). In addition, framework support residues may be altered to preserve binding affinity (see, e.g., Queen et al., Proc. Natl Acad Sci USA, 86:10029-10032 (1989), Hodgson et al., Bio/Technology, 9:421 (1991)). A suitable human acceptor antibody may be one selected from a conventional database, e.g., the KABAT® database, Los Alamos database, and Swiss Protein database, by homology to the nucleotide and amino acid sequences of the donor antibody. A human antibody characterized by a homology to the framework regions of the donor antibody (on an amino acid basis) may be suitable to provide a heavy chain constant region and/or a heavy chain variable framework region for insertion of the donor CDRs. A suitable acceptor antibody capable of donating light chain constant or variable framework regions may be selected in a similar manner. It should be noted that the acceptor antibody heavy and light chains are not required to originate from the same acceptor antibody. The prior art describes several ways of producing such humanised antibodies - see for example EP-A-0239400 and EP-A-054951.

For nucleic acids, the term "substantial identity" indicates that two nucleic acids, or designated sequences thereof, when optimally aligned and compared, are identical, with appropriate nucleotide insertions or deletions, in at least about 80% of the nucleotides, at least about 90% to about 95%, or at least about 98% to about 99.5% of the nucleotides. Alternatively, substantial identity exists when the segments will hybridize under selective hybridization conditions, to the complement of the strand. "Identity," means, for polynucleotides and polypeptides, as the case may be, the comparison calculated using an algorithm provided in (1) and (2) below:

(1) Identity for polynucleotides is calculated by multiplying the total number of nucleotides in a given sequence by the integer defining the percent identity divided by 100 and then subtracting that product from said total number of nucleotides in said sequence, or:

$$nn \leq xn - (xn \bullet y),$$

wherein nn is the number of nucleotide alterations, xn is the total number of nucleotides in a given sequence, y is 0.95 for 95%, 0.97 for 97% or 1.00 for 100%, and • is the symbol for the multiplication operator, and wherein any non-integer product of xn and y is rounded down to the nearest integer prior to subtracting it from xn. Alterations of a polynucleotide sequence encoding a polypeptide may create nonsense, missense or frameshift mutations in this coding sequence and thereby alter the polypeptide encoded by the polynucleotide following such alterations.

(2) Identity for polypeptides is calculated by multiplying the total number of amino acids by the integer defining the percent identity divided by 100 and then subtracting that product from said total number of amino acids, or:

$$na \leq xa - (xa \bullet y),$$

wherein na is the number of amino acid alterations, xa is the total number of amino acids in the sequence, y is 0.95 for 95%, 0.97 for 97% or 1.00 for 100%, and • is the symbol for the multiplication operator, and wherein any non-integer product of xa and y is rounded down to the nearest integer prior to subtracting it from xa

For nucleotide and amino acid sequences, the term "identical" indicates the degree of identity between two nucleic acid or amino acid sequences when optimally aligned and compared with appropriate insertions or deletions.

"Isolated" means altered "by the hand of man" from its natural state, has been changed or removed from its original environment, or both. For example, a polynucleotide or a polypeptide naturally present in a living organism is not "isolated," but the same polynucleotide or polypeptide separated from the coexisting materials of its natural state is "isolated", including but not limited to when such polynucleotide or polypeptide is introduced back into a cell, even if the cell is of the same species or type as that from which the polynucleotide or polypeptide was separated.

Throughout the present specification and the accompanying claims the term "comprising" and "comprises" incorporates "consisting of" and "consists of". That is, these words are intended to convey the possible inclusion of other elements or integers not specifically recited, where the context allows.

The term "specifically binds" as used throughout the present specification in relation to antigen binding proteins of the invention means that the antigen binding protein binds human BCMA (hBCMA) with no or insignificant binding to other human proteins. The term however does not exclude the fact that antigen binding proteins of the invention may also be cross-reactive with other forms of BCMA, for example primate BCMA. For example in one embodiment the antigen binding protein does not bind to TACI or BAFF-R.

The term "inhibits" as used throughout the present specification in relation to antigen binding proteins of the invention means that the biological activity of BCMA is reduced in the presence of the antigen binding proteins of the present invention in comparison to the activity of BCMA in the absence of such antigen binding proteins. Inhibition may be due but not limited to one or more of blocking ligand binding, preventing the ligand activating the receptor, and/ or

down regulating the BCMA. Inhibits can also refer to an antigen binding protein binding to BCMA and causing cell apoptosis or ADCC.The antibodies of the invention may neutralise the activity of the BCMA ligands BAFF and/or APRIL binding to BCMA. Levels of neutralisation can be measured in several ways, for example by use of the assays as set out in the examples below, for example in 4.4 in an H929 cell NFkB signalling assay. The BCMA ligands BAFF and APRIL are able to induce NFkB signalling and downstream events following binding to BCMA. The neutralisation of BCMA in this assay is measured by assessing the ability of anti-BCMA monoclonal antibodies to inhibit BAFF or APRIL driven NFkB induction.

If an antibody or antigen binding fragment thereof is capable of neutralisation then this is indicative of inhibition of the interaction between human BAFF or APRIL and BCMA. Antibodies which are considered to have neutralising activity against human BCMA would have an IC50 of less than 30 micrograms/ml, or less than 20 micrograms/ml, or less than 10 micrograms/ml, or less than 5 micrograms/ml or less than 1 micrograms/ml or less than 0.1 micrograms/ml in the H929 stimulation assay as set out in Example 4.4

"CDRs" are defined as the complementarity determining region amino acid sequences of an antibody which are the hypervariable domains of immunoglobulin heavy and light chains. There are three heavy chain and three light chain CDRs (or CDR regions) in the variable portion of an immunoglobulin. Thus, "CDRs" as used herein may refer to all three heavy chain CDRs, or all three light chain CDRs (or both all heavy and all light chain CDRs, if appropriate).

CDRs provide the majority of contact residues for the binding of the antibody to the antigen or epitope. CDRs of interest in this invention are derived from donor antibody variable heavy and light chain sequences, and include analogs of the naturally occurring CDRs, which analogs also share or retain the same antigen binding specificity and/or neutralizing ability as the donor antibody from which they were derived.

The CDR sequences of antibodies can be determined by the Kabat numbering system (Kabat et al; (Sequences of proteins of Immunological Interest NIH, 1987), alternatively they can be determined using the Chothia numbering system (Al-Lazikani et al., (1997) JMB 273,927-948), the contact definition method (MacCallum R.M., and Martin A.C.R. and Thornton J.M, (1996), Journal of Molecular Biology, 262 (5), 732-745) or any other established method for numbering the residues in an antibody and determining CDRs known to the skilled man in the art

Other numbering conventions for CDR sequences available to a skilled person include "AbM" (University of Bath) and "contact" (University College London) methods. The minimum overlapping region using at least two of the Kabat, Chothia, AbM and contact methods can be determined to provide the "minimum binding unit". The minimum binding unit may be a sub-portion of a CDR.

Table A below represents one definition using each numbering convention for each CDR or binding unit. The Kabat numbering scheme is used in Table X to number the variable domain amino acid sequence. It should be noted that some of the CDR definitions may vary depending on the individual publication used.

**Table A**

| | Kabat CDR | Chothia CDR | AbM CDR | Contact CDR | Minimum binding unit |
|---|---|---|---|---|---|
| H1 | 31-35/35A/35B | 26-32/33/34 | 26-35/35A/35B | 30-35/35A/35B | 31-32 |
| H2 | 50-65 | 52-56 | 50-58 | 47-58 | 52-56 |
| H3 | 95-102 | 95-102 | 95-102 | 93-101 | 95-101 |
| L1 | 24-34 | 24-34 | 24-34 | 30-36 | 30-34 |
| L2 | 50-56 | 50-56 | 50-56 | 46-55 | 50-55 |
| L3 | 89-97 | 89-97 | 89-97 | 89-96 | 89-96 |

Throughout this specification, amino acid residues in antibody sequences are numbered according to the Kabat scheme. Similarly, the terms "CDR", "CDRL1", "CDRL2", "CDRL3", "CDRH1", "CDRH2", "CDRH3" follow the Kabat numbering system as set forth in Kabat et al; Sequences of proteins of Immunological Interest NIH, 1987.

The terms "Variant" refers to at least one, two or three amino acid changes in the sequence. These amino acid changes may be deletion, substitution or addition but are preferably substitution. In one such embodiment the substitutions are conservative substitutions.

In an alternative embodiment the variant sequence contains at least one substitution whilst retaining the canonical of the antigen binding protein.

The complementarity determining regions (CDRs) L1, L2, L3, H1 and H2 tend to structurally exhibit one of a finite number of main chain conformations. The particular canonical structure class of a CDR is defined by both the length of the CDR and by the loop packing, determined by residues located at key positions in both the CDRs and the framework regions (structurally determining residues or SDRs). Martin and Thornton (1996; J Mol Biol 263:800-815)

have generated an automatic method to define the "key residue" canonical templates. Cluster analysis is used to define the canonical classes for sets of CDRs, and canonical templates are then identified by analysing buried hydrophobics, hydrogen-bonding residues, and e.g. conserved glycines. The CDRs of antibody sequences can be assigned to canonical classes by comparing the sequences to the key residue templates and scoring each template using identity or similarity matrices.

The terms "VH" and "VL" are used herein to refer to the heavy chain variable domain and light chain variable domain respectively of an antibody.

As used herein the term "domain" refers to a folded protein structure which has tertiary structure independent of the rest of the protein. Generally, domains are responsible for discrete functional properties of proteins and in many cases may be added, removed or transferred to other proteins without loss of function of the remainder of the protein and/or of the domain. An "antibody single variable domain" is a folded polypeptide domain comprising sequences characteristic of antibody variable domains. It therefore includes complete antibody variable domains and modified variable domains, for example, in which one or more loops have been replaced by sequences which are not characteristic of antibody variable domains, or antibody variable domains which have been truncated or comprise N- or C-terminal extensions, as well as folded fragments of variable domains which retain at least the binding activity and specificity of the full-length domain.

The phrase "immunoglobulin single variable domain" refers to an antibody variable domain (VH, VHH, VL) that specifically binds an antigen or epitope independently of a different V region or domain. An immunoglobulin single variable domain can be present in a format (e.g., homo- or hetero-multimer) with other, different variable regions or variable domains where the other regions or domains are not required for antigen binding by the single immunoglobulin variable domain (i.e., where the immunoglobulin single variable domain binds antigen independently of the additional variable domains). A "domain antibody" or "dAb" is the same as an "immunoglobulin single variable domain" which is capable of binding to an antigen as the term is used herein. An immunoglobulin single variable domain may be a human antibody variable domain, but also includes single antibody variable domains from other species such as rodent (for example, as disclosed in WO 00/29004), nurse shark and Camelid VHH dAbs. Camelid VHH are immunoglobulin single variable domain polypeptides that are derived from species including camel, llama, alpaca, dromedary, and guanaco, which produce heavy chain antibodies naturally devoid of light chains. Such VHH domains may be humanised according to standard techniques available in the art, and such domains are still considered to be "domain antibodies" according to the invention. As used herein "VH includes camelid VHH domains. NARV are another type of immunoglobulin single variable domain which were identified in cartilaginous fish including the nurse shark. These domains are also known as Novel Antigen Receptor variable region (commonly abbreviated to V(NAR) or NARV). For further details see Mol. Immunol. 44, 656-665 (2006) and US20050043519A.

The term "Epitope-binding domain" refers to a domain that specifically binds an antigen or epitope independently of a different V region or domain, this may be a domain antibody (dAb), for example a human, camelid or shark immunoglobulin single variable domain or it may be a domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEl and GroES; 30eroxidise30g (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to a ligand other than the natural ligand.

CTLA-4 (Cytotoxic T Lymphocyte-associated Antigen 4) is a CD28-family receptor expressed on mainly CD4+ T-cells. Its extracellular domain has a variable domain-like Ig fold. Loops corresponding to CDRs of antibodies can be substituted with heterologous sequence to confer different binding properties. CTLA-4 molecules engineered to have different binding specificities are also known as Evibodies. For further details see Journal of Immunological Methods 248 (1-2), 31-45 (2001)

Lipocalins are a family of extracellular proteins which transport small hydrophobic molecules such as steroids, bilins, retinoids and lipids. They have a rigid β-sheet secondary structure with a numer of loops at the open end of the conical structure which can be engineered to bind to different target antigens. Anticalins are between 160-180 amino acids in size, and are derived from lipocalins. For further details see Biochim Biophys Acta 1482: 337-350 (2000), US7250297B1 and US20070224633

An affibody is a scaffold derived from Protein A of Staphylococcus aureus which can be engineered to bind to antigen. The domain consists of a three-helical bundle of approximately 58 amino acids. Libraries have been generated by randomisation of surface residues. For further details see Protein Eng. Des. Sel. 17, 455-462 (2004) and EP1641818A1

Avimers are multidomain proteins derived from the A-domain scaffold family. The native domains of approximately 35 amino acids adopt a defined disulphide bonded structure. Diversity is generated by shuffling of the natural variation exhibited by the family of A-domains. For further details see Nature Biotechnology 23(12), 1556 - 1561 (2005) and Expert Opinion on Investigational Drugs 16(6), 909-917 (June 2007)

A Transferrin is a monomeric serum transport glycoprotein. Transferrins can be engineered to bind different target antigens by insertion of peptide sequences in a permissive surface loop. Examples of engineered transferrins scaffolds include the Trans-body. For further details see J. Biol. Chem 274, 24066-24073 (1999).

Designed Ankyrin Repeat Proteins (DARPins) are derived from Ankyrin which is a family of proteins that mediate attachment of integral membrane proteins to the cytoskeleton. A single ankyrin repeat is a 33 residue motif consisting of two α-helices and a β-turn. They can be engineered to bind different target antigens by randomising residues in the first α-helix and a β-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028A1.

Fibronectin is a scaffold which can be engineered to bind to antigen. Adnectins consists of a backbone of the natural amino acid sequence of the $10^{th}$ domain of the 15 repeating units of human fibronectin type III (FN3). Three loops at one end of the β-sandwich can be engineered to enable an Adnectin to specifically recognize a therapeutic target of interest. For further details see Protein Eng. Des. Sel. 18, 435-444 (2005), US20080139791, WO2005056764 and US6818418B1.

Peptide aptamers are combinatorial recognition molecules that consist of a constant scaffold protein, typically thioredoxin (TrxA) which contains a constrained variable peptide loop inserted at the active site. For further details see Expert Opin. Biol. Ther. 5, 783-797 (2005).

Microbodies are derived from naturally occurring microproteins of 25-50 amino acids in length which contain 3-4 cysteine bridges - examples of microproteins include KalataB1 and conotoxin and knottins. The microproteins have a loop which can be engineered to include upto 25 amino acids without affecting the overall fold of the microprotein. For further details of engineered knottin domains, see WO2008098796.

Other epitope binding domains include proteins which have been used as a scaffold to engineer different target antigen binding properties include human γ-crystallin and human ubiquitin (affilins), kunitz type domains of human protease inhibitors, PDZ-domains of the Ras-binding protein AF-6, scorpion toxins (charybdotoxin), C-type lectin domain (tetranectins) are reviewed in Chapter 7 - Non-Antibody Scaffolds from Handbook of Therapeutic Antibodies (2007, edited by Stefan Dubel) and Protein Science 15:14-27 (2006). Epitope binding domains of the present invention could be derived from any of these alternative protein domains.

As used herein, the term "antigen-binding site" refers to a site on a protein which is capable of specifically binding to antigen, this may be a single domain, for example an epitope-binding domain, or it may be paired VH/VL domains as can be found on a standard antibody. In some embodiments of the invention single-chain Fv (ScFv) domains can provide antigen-binding sites.

The terms "mAbdAb" and dAbmAb" are used herein to refer to antigen-binding proteins of the present invention. The two terms can be used interchangeably, and are intended to have the same meaning as used herein.

The term "antigen binding protein" as used herein refers to antibodies, antibody fragments for example a domain antibody (dAb), ScFv, Fab, Fab2, and other protein constructs. Antigen binding molecules may comprise at least one Ig variable domain, for example antibodies, domain antibodies (dAbs), Fab, Fab', F(ab')2, Fv, ScFv, diabodies, mAbdAbs, affibodies, heteroconjugate antibodies or bispecific antibodies. In one embodiment the antigen binding molecule is an antibody. In another embodiment the antigen binding molecule is a dAb, i.e. an immunoglobulin single variable domain such as a VH, VHH or VL that specifically binds an antigen or epitope independently of a different V region or domain. Antigen binding molecules may be capable of binding to two targets, i.e. they may be dual targeting proteins. Antigen binding molecules may be a combination of antibodies and antigen binding fragments such as for example, one or more domain antibodies and/or one or more ScFvs linked to a monoclonal antibody. Antigen binding molecules may also comprise a non-Ig domain for example a domain which is a derivative of a scaffold selected from the group consisting of CTLA-4 (Evibody); lipocalin; Protein A derived molecules such as Z-domain of Protein A (Affibody, SpA), A-domain (Avimer/Maxibody); Heat shock proteins such as GroEl and GroES; 32eroxidise32g (trans-body); ankyrin repeat protein (DARPin); peptide aptamer; C-type lectin domain (Tetranectin); human γ-crystallin and human ubiquitin (affilins); PDZ domains; scorpion toxinkunitz type domains of human protease inhibitors; and fibronectin (adnectin); which has been subjected to protein engineering in order to obtain binding to OSM. As used herein "antigen binding protein" will be capable of antagonising and/or neutralising human OSM. In addition, an antigen binding protein may inhibit and or block OSM activity by binding to OSM and preventing a natural ligand from binding and/or activating the gp130 receptor.

The term "Effector Function" as used herein is meant to refer to one or more of Antibody dependant cell mediated cytotoxic activity (ADCC) , Complement-dependant cytotoxic activity (CDC) mediated responses, Fc-mediated phagocytosis and antibody recycling via the FcRn receptor. For IgG antibodies, effector functionalities including ADCC and ADCP are mediated by the interaction of the heavy chain constant region with a family of Fcy receptors present on the surface of immune cells. In humans these include FcγRI (CD64), FcγRII (CD32) and FcγRIII (CD16). Interaction between the antigen binding protein bound to antigen and the formation of the Fc/ Fcy complex induces a range of effects including cytotoxicity, immune cell activation, phagocytosis and release of inflammatory cytokines.

The interaction between the constant region of an antigen binding protein and various Fc receptors (FcR) is believed to mediate the effector functions of the antigen binding protein. Significant biological effects can be a consequence of effector functionality, in particular, antibody-dependent cellular cytotoxicity (ADCC), fixation of complement (complement dependent cytotoxicity or CDC), and halflife/clearance of the antigen binding protein. Usually, the ability to mediate effector function requires binding of the antigen binding protein to an antigen and not all antigen binding proteins will mediate every effector function.

Effector function can be measured in a number of ways including for example via binding of the FcγRIII to Natural Killer cells or via FcγRI to monocytes/macrophages to measure for ADCC effector function. For example an antigen binding protein of the present invention can be assessed for ADCC effector function in a Natural Killer cell assay. Examples of such assays can be found in Shields et al, 2001 The Journal of Biological Chemistry, Vol. 276, p6591-6604; Chappel et al, 1993 The Journal of Biological Chemistry, Vol 268, p25124-25131; Lazar et al, 2006 PNAS, 103; 4005-4010.

Examples of assays to determine CDC function include that described in 1995 J Imm Meth 184:29-38.

Some isotypes of human constant regions, in particular IgG4 and IgG2 isotypes, essentially lack the functions of a) activation of complement by the classical pathway; and b) antibody-dependent cellular cytotoxicity. Various modifications to the heavy chain constant region of antigen binding proteins may be carried out depending on the desired effector property. IgG1 constant regions containing specific mutations have separately been described to reduce binding to Fc receptors and therefore reduce ADCC and CDC (Duncan et al. Nature 1988, 332; 563-564; Lund et al. J. Immunol. 1991, 147; 2657-2662; Chappel et al. PNAS 1991, 88; 9036-9040; Burton and Woof, Adv. Immunol. 1992, 51;1-84; Morgan et al., Immunology 1995, 86; 319-324; Hezareh et al., J. Virol. 2001, 75 (24); 12161-12168).

In one embodiment of the present invention there is provided an antigen binding protein comprising a constant region such that the antigen binding protein has reduced ADCC and/or complement activation or effector functionality. In one such embodiment the heavy chain constant region may comprise a naturally disabled constant region of IgG2 or IgG4 isotype or a mutated IgG1 constant region. Examples of suitable modifications are described in EP0307434. One example comprises the substitutions of alanine residues at positions 235 and 237 (EU index numbering).

Human IgG1 constant regions containing specific mutations or altered glycosylation on residue Asn297 have also been described to enhance binding to Fc receptors. In some cases these mutations have also been shown to enhance ADCC and CDC (Lazar et al. PNAS 2006, 103; 4005-4010; Shields et al. J Biol Chem 2001, 276; 6591-6604; Nechansky et al. Mol Immunol, 2007, 44; 1815-1817).

In one embodiment of the present invention, such mutations are in one or more of positions selected from 239, 332 and 330 (IgG1), or the equivalent positions in other IgG isotypes. Examples of suitable mutations are S239D and I332E and A330L. In one embodiment the antigen binding protein of the invention herein described is mutated at positions 239 and 332, for example S239D and I332E or in a further embodiment it is mutated at three or more positions selected from 239 and 332 and 330, for example S239D and I332E and A330L. (EU index numbering).

In an alternative embodiment of the present invention, there is provided an antigen binding protein comprising a heavy chain constant region with an altered glycosylation profile such that the antigen binding protein has enhanced effector function. For example, wherein the antigen binding protein has enhanced ADCC or enhanced CDC or wherein it has both enhanced ADCC and CDC effector function. Examples of suitable methodologies to produce antigen binding proteins with an altered glycosylation profile are described in WO2003011878, WO2006014679 and EP1229125, all of which can be applied to the antigen binding proteins of the present invention.

The present invention also provides a method for the production of an antigen binding protein according to the invention comprising the steps of:

a) culturing a recombinant host cell comprising an expression vector comprising the isolated nucleic acid as described herein, wherein the FUT8 gene encoding alpha-1,6-fucosyltransferase has been inactivated in the recombinant host cell; and
b) recovering the antigen binding protein.

Such methods for the production of antigen binding proteins can be performed, for example, using the POTELLIGENT™ technology system available from BioWa, Inc. (Princeton, NJ) in which CHOK1SV cells lacking a functional copy of the FUT8 gene produce monoclonal antibodies having enhanced antibody dependent cell mediated cytotoxicity (ADCC) activity that is increased relative to an identical monoclonal antibody produced in a cell with a functional FUT8 gene. Aspects of the POTELLIGENT™ technology system are described in US7214775, US6946292, WO0061739 and WO0231240.Those of ordinary skill in the art will also recognize other appropriate systems.

In one embodiment of the present invention there is provided an antigen binding protein comprising a chimaeric heavy chain constant region for example an antigen binding protein comprising a chimaeric heavy chain constant region with at least one CH2 domain from IgG3 such that the antigen binding protein has enhanced effector function, for example wherein it has enhanced ADCC or enhanced CDC, or enhanced ADCC and CDC functions,. In one such embodiment, the antigen binding protein may comprise one CH2 domain from IgG3 or both CH2 domains may be from IgG3.

Also provided is a method of producing an antigen binding protein according to the invention comprising the steps of:

a) culturing a recombinant host cell comprising an expression vector comprising an isolated nucleic acid as described herein wherein the expression vector comprises a nucleic acid sequence encoding an Fc domain having both IgG1 and IgG3 Fc domain amino acid residues; and
b) recovering the antigen binding protein.

Such methods for the production of antigen binding proteins can be performed, for example, using the COMPLEGENT™ technology system available from BioWa, Inc. (Princeton, NJ) and Kyowa Hakko Kogyo (now, Kyowa Hakko Kirin Co., Ltd.) Co., Ltd. In which a recombinant host cell comprising an expression vector in which a nucleic acid sequence encoding a chimeric Fc domain having both IgG1 and IgG3 Fc domain amino acid residues is expressed to produce an antigen binding protein having enhanced complement dependent cytotoxicity (CDC) activity that is increased relative to an otherwise identical antigen binding protein lacking such a chimeric Fc domain. Aspects of the COMPLEGENT™ technology system are described in WO2007011041 and US20070148165. In an alternative embodiment CDC activity may be increased by introducing sequence specific mutations into the Fc region of an IgG chain. Those of ordinary skill in the art will also recognize other appropriate systems.

It will be apparent to those skilled in the art that such modifications may not only be used alone but may be used in combination with each other in order to further enhance effector function.

In one such embodiment of the present invention there is provided an antigen binding protein comprising a heavy chain constant region which comprises a mutated and chimaeric heavy chain constant region for example wherein an antigen binding protein comprising at least one CH2 domain from IgG3 and one CH2 domain from IgG1, wherein the IgG1 CH2 domain has one or more mutations at positions selected from 239 and 332 and 330 (for example the mutations may be selected from S239D and I332E and A330L) such that the antigen binding protein has enhanced effector function, for example wherein it has one or more of the following functions, enhanced ADCC or enhanced CDC, for example wherein it has enhanced ADCC and enhanced CDC. In one embodiment the IgG1 CH2 domain has the mutations S239D and I332E.

In an alternative embodiment of the present invention there is provided an antigen binding protein comprising a chimaeric heavy chain constant region and which has an altered glycosylation profile. In one such embodiment the heavy chain constant region comprises at least one CH2 domain from IgG3 and one CH2 domain from IgG1 and has an altered glycosylation profile such that the ratio of fucose to mannose is 0.8:3 or less, for example wherein the antigen binding protein is defucosylated so that said antigen binding protein has an enhanced effector function in comparison with an equivalent antigen binding protein with an immunoglobulin heavy chain constant region lacking said mutations and altered glycosylation profile, for example wherein it has one or more of the following functions, enhanced ADCC or enhanced CDC, for example wherein it has enhanced ADCC and enhanced CDC In an alternative embodiment the antigen binding protein has at least one IgG3 CH2 domain and at least one heavy chain constant domain from IgG1 wherein both IgG CH2 domains are mutated in accordance with the limitations described herein.

In one aspect of the invention there is provided a method of producing an antigen binding protein according to the invention described herein comprising the steps of:

a) culturing a recombinant host cell containing an expression vector containing an isolated nucleic acid as described herein, said expression vector further comprising a Fc nucleic acid sequence encoding a chimeric Fc domain having both IgG1 and IgG3 Fc domain amino acid residues, and wherein the FUT8 gene encoding alpha-1,6-fucosyltransferase has been inactivated in the recombinant host cell;and
b) recovering the antigen binding protein.

Such methods for the production of antigen binding proteins can be performed, for example, using the ACCRETAMAB™ technology system available from BioWa, Inc. (Princeton, NJ) which combines the POTELLIGENT™ and COMPLEGENT™ technology systems to produce an antigen binding protein having both ADCC and CDC enhanced activity that is increased relative to an otherwise identical monoclonal antibody lacking a chimeric Fc domain and which has fucose on the oligosaccharide

In yet another embodiment of the present invention there is provided an antigen binding protein comprising a mutated and chimeric heavy chain constant region wherein said antigen binding protein has an altered glycosylation

profile such that the antigen binding protein has enhanced effector function, for example wherein it has one or more of the following functions, enhanced ADCC or enhanced CDC. In one embodiment the mutations are selected from positions 239 and 332 and 330, for example the mutations are selected from S239D and I332E and A330L. In a further embodiment the heavy chain constant region comprises at least one CH2 domain from IgG3 and one Ch2 domain from IgG1. In one embodiment the heavy chain constant region has an altered glycosylation profile such that the ratio of fucose to mannose is 0.8:3 or less for example the antigen binding protein is defucosylated, so that said antigen binding protein has an enhanced effector function in comparison with an equivalent non-chimaeric antigen binding protein or with an immunoglobulin heavy chain constant region lacking said mutations and altered glycosylation profile.

Immunoconjugates

Also provided is an immunoconjugate (interchangeably referred to as "antibody-drug conjugates," or "ADCs")comprising an antigen binding protein according to the invention as herein described including, but not limited to, an antibody conjugated to one or more cytotoxic agents, such as a chemotherapeutic agent, a drug, a growth inhibitory agent, a toxin (e.g., a protein toxin, an enzymatically active toxin of bacterial, fungal, plant, or animal origin, or fragments thereof), or a radioactive isotope (i.e., a radioconjugate).

Immunoconjugates have been used for the local delivery of cytotoxic agents, i.e., drugs that kill or inhibit the growth or proliferation of cells, in the treatment of cancer (Lambert, J. (2005) Curr. Opinion in Pharmacology 5:543-549; Wu et al. (2005) Nature Biotechnology 23(9):1137-1146; Payne, G. (2003) i 3:207-212; Syrigos and Epenetos (1999) Anticancer Research 19:605-614; Niculescu-Duvaz and Springer (1997) Adv. Drug Deliv. Rev. 26:151-172; U.S. Pat. No. 4,975,278). Immunoconjugates allow for the targeted delivery of a drug moiety to a tumor, and intracellular accumulation therein, where systemic administration of unconjugated drugs may result in unacceptable levels of toxicity to normal cells as well as the tumor cells sought to be eliminated (Baldwin et al., Lancet (Mar. 15, 1986) pp. 603-05; Thorpe (1985) "Antibody Carriers Of Cytotoxic Agents In Cancer Therapy: A Review," in Monoclonal Antibodies '84: Biological And Clinical Applications (A. Pinchera et al., eds) pp. 475-506. Both polyclonal antibodies and monoclonal antibodies have been reported as useful in these strategies (Rowland et al., (1986) Cancer Immunol. Immunother. 21:183-87). Drugs used in these methods include daunomycin, doxorubicin, methotrexate, and vindesine (Rowland et al., (1986) supra). Toxins used in antibody-toxin conjugates include bacterial toxins such as diphtheria toxin, plant toxins such as ricin, small molecule toxins such as geldanamycin (Mandler et al (2000) J. Nat. Cancer Inst. 92(19):1573-1581; Mandleret al (2000) Bioorganic & Med. Chem. Letters 10:1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342).

In one embodiment, the present invention includes immunoconjugates having the following general structure:

$$ABP - ((Linker)_n - Ctx)_m$$

Wherein ABP is an antigen binding protein
Linker is either absent or any a cleavable or non-cleavable linker described herein
Ctx is any cytotoxic agent described herein
n is 0, 1, 2, or 3 and
m is 1, 2, 3, 4, 5, 6, 7, 8, 9 or 10.

Examples of antibodies linked by an MC linker with auristatins such as MMAE and MMAF are depicted in the following structures:

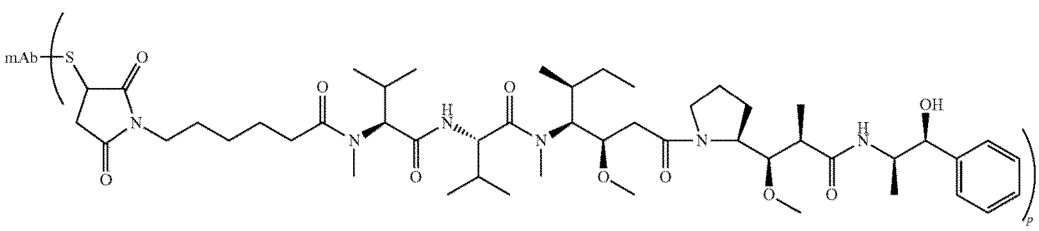

or

L-MC-MMAE

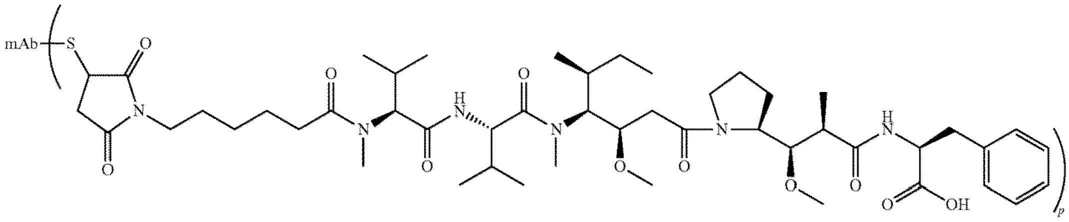

L-MC-MMAF

In certain embodiments, an immunoconjugate comprises an antigen binding protein, including but not limited to, an antibody and a chemotherapeutic agent or other toxin. Chemotherapeutic agents useful in the generation of immunoconjugates are described herein. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. See, e.g., WO 93/21232 published Oct. 28, 1993. A variety of radionuclides are available for the production of radioconjugated antibodies. Examples include $^{211}At$, $^{212}Bi$, $^{131}I$, $^{131}In$, $^{90}Y$, and $^{186}Re$.

Antigen binding proteins of the present invention may also be conjugated to one or more toxins, including, but not limited to, a calicheamicin, maytansinoids, dolastatins, aurostatins, a trichothecene, and CC1065, and the derivatives of these toxins that have toxin activity. Suitable cytotoxic agents include, but are not limited to, an auristatin including dovaline-valine-dolaisoleunine-dolaproinephenylalanine (MMAF) and monomethyl auristatin E (MMAE) as well as ester forms of MMAE, a DNA minor groove binding agent, a DNA minor groove alkylating agent, an enediyne, a lexitropsin, a duocarmycin, a taxane, including paclitaxel and docetaxel, a puromycin, a dolastatin, a maytansinoid, and a vinca alkaloid. Specific cytotoxic agents include topotecan, morpholino-doxorubicin, rhizoxin, cyanomorpholino-doxorubicin, dolastatin-10, echinomycin, combretatstatin, chalicheamicin, maytansine, DM-1, DM-4, netropsin. Other suitable cytotoxic agents include anti-tubulin agents, such as an auristatin, a vinca alkaloid, a podophyllotoxin, a taxane, a baccatin derivative, a cryptophysin, a maytansinoid, a combretastatin, or a dolastatin. Antitubulin agent include dimethylvaline-valine-dolaisoleuine-dolaproine-phenylalanine-p-phenylened- iamine (AFP), MMAF, MMAE, auristatin E, vincristine, vinblastine, vindesine, vinorelbine, VP-16, camptothecin, paclitaxel, docetaxel, epothilone A, epothilone B, nocodazole, colchicines, colcimid, estramustine, cemadotin, discodermolide, maytansine, DM-1, DM-4 or eleutherobin.

Antibody drug conjugates were produced by conjugating the small molecule anti-tubulin agent monomethylauristatin E (MMAE) or monomethylauristatin F (MMAF) to the antibodies. In the case of MMAE the linker consists of a thiol-reactive maleimide, a caproyl spacer, the dipeptide valine-citrulline, and p-aminobenzyloxycarbonyl, a self-immolative fragmenting group. In the case of MMAF a protease-resistant maleimidocaproyl linker is used. The conjugation process leads to heterogeneity in drug-antibody attachment, varying in both the number of drugs bound to each antibody molecule (mole ratio [MR]), and the site of attachment. The most prevalent species is the material with an MR = 4; less prevalent are materials with MR of 0, 2, 6, and 8. The overall average drug-to-antibody MR is approximately 4.

Production of Immunoconjugates

The points of attachment are cysteines produced by mild reduction of the interchain disulfides of the antibody which is carried out whilst antibodies are immobilised on Protein G affinity resin (thus enabling the use of large reagent excesses without intermediate purifications). While immobilized, a large excess of TCEP will fully reduce the interchain disulfides but has no impact upon the binding of the antibody to the resin.

The number of thiols per antibody generated by this procedure depends upon the source and isotype of the antibodies. For example, human (and mouse-human chimeric) IgG1s have 4 reducible disulfides, and thus generate 8 thiols upon full reduction, whereas murine IgG1s have 5 reducible disulfides and produce 10 thiols. If ADCs with the maximal drug loading (e.g., 10 drugs per antibody for the murine IgG1s) are desired, then the maleimido-drug-linker can simply be added to the immobilized antibodies in sufficient excess to ensure complete conjugation. However, ADCs with fewer drugs per antibody can also be prepared from fully reduced antibodies by including a biologically inert capping agent such as N-ethyl maleimide (NEM) which occupies some of the available thiols on the antibody. When the maleimido-drug-linker and the capping agent are added simultaneously to the fully reduced antibody and in large excess (at least 3-fold), the two maleimide electrophiles compete for the limiting number of available thiols. In this fashion, the drug loading is determined by the relative thiol reaction rates of the drug-linker and capping agent, and thus can be considered to be under kinetic control. The relative reaction rates of maleimido-drug-linkers do vary significantly, and thus the molar ratio of drug-linker to NEM present in a reaction mix must be determined empirically to arrive at a panel of ADCs with a

desired level of drug loading. The mole fraction of the drug linkers SGD-1006 (vcMMAE) and SGD-1269 (mcMMAF) in NEM mixtures which yield ADCs with approximately 4 drugs per antibody are summarized in Table 2 for common human and murine IgG isotypes.

Auristatins and Dolastatins

In some embodiments, the immunoconjugate comprises an antigen binding protein or antibody conjugated to dolastatins or dolostatin peptidic analogs and derivatives, the auristatins (U.S. Pat. Nos. 5,635,483; 5,780,588). Dolastatins and auristatins have been shown to interfere with microtubule dynamics, GTP hydrolysis, and nuclear and cellular division (Woyke et al. (2001) Antimicrob. Agents and Chemother. 45(12):3580-3584) and have anticancer (U.S. Pat. No. 5,663,149) and antifungal activity (Pettit et al. (1998) Antimicrob. Agents Chemother. 42:2961-2965). The dolastatin or auristatin (which are pentapeptide derivatives of dolastatins) drug moiety may be attached to the antibody through the N (amino) terminus or the C (carboxyl) terminus of the peptidic drug moiety (WO 02/088172).

Exemplary auristatin embodiments include the N-terminus linked monomethylauristatin drug moieties DE and DF, disclosed in "Monomethylvaline Compounds Capable of Conjugation to Ligands," U.S. Patent No. 7,498,298,. As used herein, the abbreviation "MMAE" refers to monomethyl auristatin E. As used herein the abbreviation "MMAF" refers to dovaline-valine-dolaisoleuine-dolaproine-phenylalanine.

Typically, peptide-based drug moieties can be prepared by forming a peptide bond between two or more amino acids and/or peptide fragments. Such peptide bonds can be prepared, for example, according to the liquid phase synthesis method (see E. Schroder and K. Lubke, "The Peptides," volume 1, pp 76-136, 1965, Academic Press) that is well known in the field of peptide chemistry. The auristatin/dolastatin drug moieties may be prepared according to the methods of: U.S. Pat. No. 5,635,483; U.S. Pat. No. 5,780,588; Pettit et al. (1989) J. Am. Chem. Soc. 111:5463-5465; Pettit et al. (1998) Anti-Cancer Drug Design 13:243-277; Pettit, G. R., et al. Synthesis, 1996, 719-725; and Pettit et al. (1996) J. Chem. Soc. Perkin Trans. 15:859-863. See also Doronina (2003) Nat Biotechnol 21(7):778-784; "Monomethylvaline Compounds Capable of Conjugation to Ligands," U.S. Patent No. 7,498,298, filed Nov. 5, 2004, (disclosing, e.g., linkers and methods of preparing monomethylvaline compounds such as MMAE and MMAF conjugated to linkers). Biologically active organic compounds which act as cytotoxic agents, specifically pentapeptides, are disclosed in US Patent Nos. 6,884,869; 7,498,298; 7,098,308; 7,256,257; and 7,423,116.. Monoclonal antibodies linked with MMAE adn MMAF as well as various derivatives of auristatins and methods of making them are described in US Patent NO. 7,964,566.

Examples of auristatins include MMAE and MMAF the structures of which are shown below:

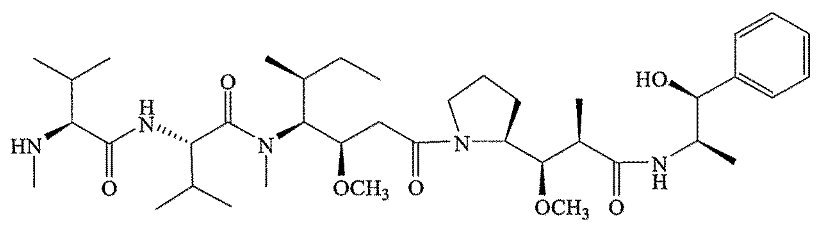

MMAE

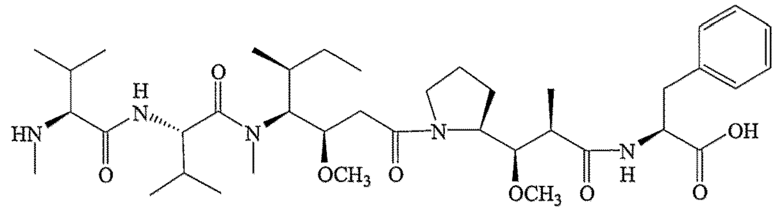

**MMAF**

Maytansine and Maytansinoids

Maytansinoids are mitototic inhibitors which act by inhibiting tubulin polymerization. Maytansine was first isolated from the east African shrub Maytenus serrata (U.S. Pat. No. 3,896,111). Subsequently, it was discovered that certain microbes also produce maytansinoids, such as maytansinol and C-3 maytansinol esters (U.S. Pat. No. 4,151,042). Highly cytotoxic maytansinoid drugs drugs can be prepared from ansamitocin precursors produced by fermentation of microorganisms such as Actinosynnema. Methods for isolating ansamitocins are described in US Patent No. 6,573,074.

Synthetic maytansinol and derivatives and analogues thereof are disclosed, for example, in U.S. Pat. Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

Antibody-maytansinoid conjugates are prepared by chemically linking an antibody to a maytansinoid molecule without significantly diminishing the biological activity of either the antibody or the maytansinoid molecule. See, e.g., U.S. Pat. No. 5,208,020. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Pat. No. 5,208,020 and in the other patents and nonpatent publications referred to hereinabove. Maytansinoids are maytansinol and maytansinol analogues modified in the aromatic ring or at other positions of the maytansinol molecule, such as various maytansinol esters. Methods for preparing matansinoids for linkage with antibodies are disclosed in US Patent Nos. 6,570,024 and 6,884,874.

Calicheamicin

The calicheamicin family of antibiotics is capable of producing double-stranded DNA breaks at subpicomolar concentrations. For the preparation of conjugates of the calicheamicin family, see U.S. Pat. Nos. 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, .gamma.1I, .alpha.2I, .alpha.3I, N-acetyl-.gamma.1I, PSAG and .theta.!1 (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

Other Cytotoxic Agents

Other antitumor agents that can be conjugated to the antibodies include BCNU, streptozoicin, vincristine and 5-fluorouracil, the family of agents known collectively LL-E33288 complex described in U.S. Pat. Nos. 5,053,394, 5,770,710, as well as esperamicins (U.S. Pat. No. 5,877,296).

Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published Oct. 28, 1993.

The present invention further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated antibodies. Examples include At211, I131, I125, Y90, Re186, Re188, Sm153, Bi212, P32, Pb212 and radioactive isotopes of Lu. When the conjugate is used for detection, it may comprise a radioactive atom for scintigraphic studies, for example tc99m or I123, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as tc99m or 1123, Re186, Re188 and In111 can be attached via a cysteine residue in the peptide. Yttrium-90 can be attached via a lysine residue. The IODOGEN method (Fraker et al. (1978) Biochem. Biophys. Res. Commun. 80: 49-57) can be used to incorporate iodine-123. "Monoclonal Antibodies in Immunoscintigraphy" (Chatal, CRC Press 1989) describes other methods in detail.

Preparation of ADCs

In antibody drug conjugates, the antibody can be conjugated directly to the cytotoxic agent or via a linker. Suitable linkers include, for example, cleavable and non-cleavable linkers. A cleavable linker is typically susceptible to cleavage under intracellular conditions. Suitable cleavable linkers include, for example, a peptide linker cleavable by an intracellular protease, such as lysosomal protease or an endosomal protease. In exemplary embodiments, the linker can be a dipeptide linker, such as a valine-citrulline (val-cit) or a phenylalanine-lysine (phe-lys) linker. Other suitable

linkers include linkers hydrolyzable at a pH of less than 5.5, such as a hydrazone linker. Additional suitable cleavable linkers include disulfide linkers.

Bristol-Myers Squibb has described particular lysosomal enzyme-cleavable antitumor drug conjugates. See, for example, U.S. Pat. No. 6,214,345. Seattle Genetics has published applications U.S. Pat. Appl. 2003/0096743 and U.S. Pat. Appl. 2003/0130189, which describe p-aminobenzylethers in drug delivery agents. The linkers described in these applications are limited to aminobenzyl ether compositions.

Conjugates of the antigen binding protein and cytotoxic agent may be made using a variety of bifunctional protein coupling agents such as N-succinimidyl-3-(2-pyridyldithio)propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis(p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene).

Additionally the linker may be composed of one or more linker components. Exemplary linker components include 6-maleimidocaproyl ("MC"), maleimidopropanoyl ("MP"), valine-citrulline ("val-cit"), alanine-phenylalanine ("ala-phe"), p-aminobenzyloxycarbonyl ("PAB"), N-Succinimidyl 4-(2-pyridylthio)pentanoate ("SPP"), N-Succinimidyl 4-(N-maleimidomethyl)cyclohexane-1 carboxylate ("SMCC"), and N-Succinimidyl (4-iodo-acetyl)aminobenzoate ("SIAB"). Additional linker components are known in the art and some are described herein. See also "Monomethylvaline Compounds Capable of Conjugation to Ligands," U.S. Patent No. US7,498,298, filed Nov. 5, 2004.

Linkers may also comprises amino acids and/or amino acid analogs. Amino acid linker components include a dipeptide, a tripeptide, a tetrapeptide or a pentapeptide. Exemplary dipeptides include: valine-citrulline (vc or val-cit), alanine-phenylalanine (af or ala-phe). Exemplary tripeptides include: glycine-valine-citrulline (gly-val-cit) and glycine-glycine-glycine (gly-gly-gly). Amino acid residues which comprise an amino acid linker component include those occurring naturally, as well as minor amino acids and non-naturally occurring amino acid analogs, such as citrulline. Amino acid linker components can be designed and optimized in their selectivity for enzymatic cleavage by a particular enzyme, for example, a tumor-associated protease, cathepsin B, C and D, or a plasmin protease.

Antigen binding proteins and antibodies may be made reactive for conjugation with linker reagents. Nucleophilic groups on antibodies include, but are not limited to: (i) N-terminal amine groups, (ii) side chain amine groups, e.g., lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by introducing one, two, three, four, or more cysteine residues (e.g., preparing mutant antibodies comprising one or more non-native cysteine amino acid residues).

Antigen binding proteins and antibodies may also be modified to introduce electrophilic moieties, which can react with nucleophilic substituents on the linker reagent or drug. The sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g., by borohydride reagents to form stable amine linkages. In one embodiment, reaction of the carbohydrate portion of a glycosylated antibody with either glactose oxidase or sodium meta-periodate may yield carbonyl (aldehyde and ketone) groups in the protein that can react with appropriate groups on the drug (Hermanson, Bioconjugate Techniques). In another embodiment, proteins containing N-terminal serine or threonine residues can react with sodium meta-periodate, resulting in production of an aldehyde in place of the first amino acid (Geoghegan & Stroh, (1992) Bioconjugate Chem. 3:138-146; U.S. Pat. No. 5,362,852). Such aldehydes can be reacted with a drug moiety or linker nucleophile.

Nucleophilic groups on a drug moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

In some embodiments, the linker is cleavable by a cleaving agent that is present in the intracellular environment (e.g., within a lysosome or endosome or caveolea). The linker can be, e.g., a peptidyl linker that is cleaved by an intracellular peptidase or protease enzyme, including, but not limited to, a lysosomal or endosomal protease. Typically, the peptidyl linker is at least two amino acids long or at least three amino acids long. Cleaving agents can include

cathepsins B and D and plasmin, all of which are known to hydrolyze dipeptide drug derivatives resulting in the release of active drug inside target cells (see, e.g., Dubowchik and Walker, 1999, Pharm. Therapeutics 83:67-123). Peptidyl linkers may be cleavable by enzymes that are present cells. For example, a peptidyl linker that is cleavable by the thiol-dependent protease cathepsin-B, which is highly expressed in cancerous tissue, can be used (e.g., a Phe-Leu or a Gly-Phe-Leu-Gly (SEQ ID NO:50) linker). Other such linkers are described, e.g., in U.S. Pat. No. 6,214,345. In specific embodiments, the peptidyl linker cleavable by an intracellular protease is a Val-Cit linker or a Phe-Lys linker (see, e.g., U.S. Pat. No. 6,214,345, which describes the synthesis of doxorubicin with the val-cit linker). One advantage of using intracellular proteolytic release of the therapeutic agent is that the agent is typically attenuated when conjugated and the serum stabilities of the conjugates are typically high.

In other embodiments, the cleavable linker is pH-sensitive, i.e., sensitive to hydrolysis at certain pH values. Typically, the pH-sensitive linker hydrolyzable under acidic conditions. For example, an acidlabile linker that is hydrolyzable in the lysosome (e.g., a hydrazone, semicarbazone, thiosemicarbazone, cis-aconitic amide, orthoester, acetal, ketal, or the like) can be used. (See, e.g., U.S. Pat. Nos. 5,122,368; 5,824,805; 5,622,929; Dubowchik and Walker, 1999, Pharm. Therapeutics 83:67-123; Neville et al., 1989, Biol. Chem. 264:14653-14661.) Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, e.g., a thioether attached to the therapeutic agent via an acylhydrazone bond (see, e.g., U.S. Pat. No. 5,622,929)).

In yet other embodiments, the linker is cleavable under reducing conditions (e.g., a disulfide linker). A variety of disulfide linkers are known in the art, including, for example, those that can be formed using SATA (N-succinimidyl-5-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alphamethyl-alpha-(2-pyridyl-dithio)toluene)- , SPDB and SMPT (See, e.g., Thorpe et al., 1987, Cancer Res. 47:5924-5931; Wawrzynczak et al., In Immunoconjugates: Antibody Conjugates in Radioimagery and Therapy of Cancer (C. W. Vogel ed., Oxford U. Press, 1987. See also U.S. Pat. No. 4,880,935.)

In yet other specific embodiments, the linker is a malonate linker (Johnson et al., 1995, Anticancer Res. 15:1387-93), a maleimidobenzoyl linker (Lau et al., 1995, Bioorg-Med-Chem. 3(10):1299-1304), or a 3'-N-amide analog (Lau et al., 1995, Bioorg-Med-Chem. 3(10):1305-12).

Typically, the linker is not substantially sensitive to the extracellular environment. As used herein, "not substantially sensitive to the extracellular environment," in the context of a linker, means that no more than about 20%, typically no more than about 15%, more typically no more than about 10%, and even more typically no more than about 5%, no more than about 3%, or no more than about 1% of the linkers, in a sample of ADC or ADC derivative, are cleaved when the ADC or ADC derivative present in an extracellular environment (e.g., in plasma). Whether a linker is not substantially sensitive to the extracellular environment can be determined, for example, by incubating independently with plasma both (a) the ADC or ADC derivative (the "ADC sample") and (b) an equal molar amount of unconjugated antibody or therapeutic agent (the "control sample") for a predetermined time period (e.g., 2, 4, 8, 16, or 24 hours) and then comparing the amount of unconjugated antibody or therapeutic agent present in the ADC sample with that present in control sample, as measured, for example, by high performance liquid chromatography.

In other, non-mutually exclusive embodiments, the linker promotes cellular internalization. In certain embodiments, the linker promotes cellular internalization when conjugated to the therapeutic agent (i.e., in the milieu of the linker-therapeutic agent moiety of the ADC or ADC derivate as described herein). In yet other embodiments, the linker promotes cellular internalization when conjugated to both the therapeutic agent and the antigen binding protein or antibody or derivative thereof (i.e., in the milieu of the ADC or ADC derivative as described herein).

A variety of linkers that can be used with the present compositions and methods are described in WO 2004010957 entitled "Drug Conjugates and Their Use for Treating Cancer, An Autoimmune Disease or an Infectious Disease" filed Jul. 31, 2003, and U.S. Provisional Application No. 60/400,403, entitled "Drug Conjugates and their use for treating cancer, an autoimmune disease or an infectious disease", filed Jul. 31, 2002.

Alternatively, a fusion protein comprising the antigen binding protein and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

In yet another embodiment, the antibody may be conjugated to a "receptor" (such as streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

The term "Non Human antibody or antibody fragment thereof' as used herein is meant to refer to antibodies or fragments thereof which originate from any species other than human wherein human includes chimeric antibodies.

The term "donor antibody" refers to an antibody (monoclonal, and/or recombinant) which contributes the amino acid sequences of its variable domains, CDRs, or other functional fragments or analogs thereof to a first immunoglobulin partner, so as to provide the altered immunoglobulin coding region and resulting expressed altered antibody with the

antigenic specificity and neutralizing activity characteristic of the donor antibody.

The term "acceptor antibody" refers to an antibody (monoclonal and/or recombinant) heterologous to the donor antibody, which contributes all (or any portion, but preferably all) of the amino acid sequences encoding its heavy and/or light chain framework regions and/or its heavy and/or light chain constant regions to the first immunoglobulin partner. The human antibody is the acceptor antibody. The term "Human acceptor sequence" as used herein is meant to refer to a framework of an antibody or antibody fragment thereof comprising the amino acid sequence of a VH or VL framework derived from a human antibody or antibody fragment thereof or a human consensus sequence framework into which CDR's from a non-human species may be incorporated.

The term "incorporation" of CDR's or hypervariable regions as used herein encompasses any means by which the non-human CDR's are situated with the human acceptor framework. It will be appreciated that this can be achieved in various ways, for example, nucleic acids encoding the desired amino acid sequence can be generated by mutating nucleic acids encoding the non-human variable domain sequence so that the framework residues thereof are changed to human acceptor framework residues, or by mutating nucleic acid encoding the human variable domain sequence so that the CDR's are changed to non-human residues, or by synthesizing nucleic acids encoding the desired sequence. In one embodiment the final sequence is generated in silico.

The present invention is illustrated by the following examples.

## Examples

### Example 1 Monoclonal Antibody Generation and Selection

1.1 Immunisation strategies

The anti human BCMA mAb murine parental CA8 was identified from hybridomas derived from mice immunized with full length human BCMA. A BALB/c mouse was immunized i.p. with 25 μg of recombinant (rBCMA) protein combined with CFA. The mouse was boosted three times at one-month intervals with 25 μg of full length rBCMA protein + 10 μg monophosphoryl lipid A-stable emulsion (MPL-SE) (Corixa Corporation, Seattle, WA) and given a pre-fusion boost of 30 μg rBCMA protein i.v. 3 days prior to fusion. Hybridomas were either generated and cloned using the ClonaCell-HY hybridoma cloning kit (StemCell Technologies, Vancouver, BC) or using a conventional method. In the conventional method, B cells from the spleens of the immunized animals were fused with Sp2/0 myeloma cells in the presence of PEG (Sigma-Aldrich, St. Louis, MO). After overnight recovery, fused cells were plated at limiting dilution in 96-well plates and subjected to hypoxanthine-aminopterinthymidine selection. Hybridoma culture supernatants were examined for the presence of anti-BCMA antibodies by ELISA and flow cytometry-.

The anti human BCMA mAb murine parental S307118G03 was identified from hybridomas derived from SJL mice immunized with recombinant human BCMA/TNFRSF17-Fc chimera (R&D 193-Fc) using the RIMMS method (Rapid immunisation multiple sites). At Day 0, 5ug protein per mouse was emulsified in AS02a adjuvant at 2 sites on back (over haunches and over shoulders) and subjacent to the major lymph nodes at 4 sites on front. On day 6 and day 11 2.5ug protein per mouse in RIBI adjuvant was injected subjacent to the major lymph nodes at 4 sites on front. On day 14 the animals were sacrificed. The lymph nodes and spleen were excised, disrupted and a PEG1500 induced somatic cell fusion performed using a 3:1 ratio with mouse myeloma cells X63 AG8 653.GFP.Bcl-2.11 (BioCat 112754; R17209/58). The fusion was plated out into 10 × 96 well plates and screened directly from these.

The anti human BCMA mAb murine parental S336105A07 was identified from hybridomas derived from identical immunisations. The lymph nodes and spleen were excised at day 14, disrupted, and a Cytopulse electrofusion was performed using a 1:1 ratio with mouse myeloma cells X63 AG8 653.GFP.Bcl-2.11 (BioCat 112754; R17209/58). The fusion was plated out into omnitrays containing semi solid medium prior to picking into 10 × 96 well plates and was screened directly from these 5 days later.

The anti human BCMA murine parental mAbs S332121F02 and S332126E04 were identified from hybridomas derived from SJL mice immunized with recombinant Fc fusion of the extracellular domain of human BCMA (4-53)BCMA using the RIMMS method (Rapid immunisation). At Day 0, 5ug protein per mouse was emulsified in AS02a adjuvant at 2 sites on back (over haunches and over shoulders) and subjacent to the major lymph nodes at 4 sites on front. On day 6 5ug recombinant cyno BCMA-Fc protein per mouse in RIBI adjuvant was injected subjacent to the major lymph nodes at 4 sites on front. On day 11 2.5ug recombinant human BCMA-Fc and 2.5ug recombinant cyno BCMA-Fc per mouse in RIBI adjuvant was injected subjacent to the major lymph nodes at 4 sites on front. On day 14 the animals were sacrificed and cells treated as for S307118G03.

The anti human BCMA murine parental mAb S322110D07 was identified from hybridomas derived from SJL mice immunised with recombinant Fc fusion of the extracellular domain of human BCMA (4-53) in complex with recombinant human April (R&D 5860-AP/CF) premixed at 1:1 molar ratio. The mice were immunized i.p. with 5ug April/Cyno BCMA-Fc complex in PBS, suspended in RIBI adjuvant, 100ul dose per mouse and boosted 3 times at 3-4 week intervals

with 2.5ug April/Cyno BCMA-Fc complex in PBS, suspended in RIBI adjuvant, 100ul dose per mouse injected via intraperitoneal route and given a pre-fusion boost of the same immunogen 1 day prior to fusion and treated as for S307118G03.

The anti human BCMA mAb murine parental S335115G01 and S335122F05 were identified from hybridomas derived from SJL mice immunized with a mixture of recombinant Fc fusion of the extracellular domain of human BCMA (4-53) and recombinant Fc fusion of the extracellular domain of cyno BCMA (4-52) using the RIMMS method (Rapid immunisation multiple sites). At Day 0, 2, 5ug of each protein per mouse was emulsified in AS02a adjuvant and injected at 2 sites on the back (over haunches and over shoulders) and subjacent to the major lymph nodes at 4 sites on front. On day 6 and day 11 2.5ug of each protein per mouse in RIBI adjuvant was injected subjacent to the major lymph nodes at 4 sites on front. On day 14 the animals were sacrificed. The lymph nodes and spleen were excised, disrupted and a Cytopulse electrofusion was performed using a 1:1 ratio with mouse myeloma cells X63 AG8 653.GFP.Bcl-2.11 (BioCat 112754; R17209/58). The fusion was plated out into omnitrays containing semi solid medium prior to picking into 32 × 96 well plates and was screened directly from these 5 days later.

**Example 2 Humanisation.**

2.1 Cloning of CA8 Hybridoma Variable Regions

Total RNA was extracted from CA8 hybridoma cells, heavy and light variable domain cDNA sequence was then generated by reverse transcription and polymerase chain reaction (RT-PCR). The forward primer for RT-PCR was a mixture of degenerate primers specific for murine immunoglobulin gene leader-sequences and the reverse primer was specific for the antibody constant regions. Reverse primers specific for IgG1, IgG2a and IgG2b were used in this case as the isotype was unknown. To design the primers, DNA multiple sequence alignments of the leader sequences of the mouse $V_H$ and $V_k$ genes were generated.

2.2 Cloning of chimeric CA8

The DNA expression constructs encoding the chimeric antibody were prepared *de novo* by build-up of overlapping oligonucleotides including restriction sites for cloning into mammalian expression vectors as well as a human signal sequence. *HindIII* and *SpeI* restriction sites were introduced to frame the VH domain containing the signal sequence for cloning into mammalian expression vectors containing the human γ1 constant region. *HindIII* and *BsiWI* restriction sites were introduced to frame the VL domain containing the signal sequence for cloning into mammalian expression vector containing the human kappa constant region.

2.3 Cloning of the humanised CA8 variants

The DNA expression constructs encoding the humanised antibody variants were prepared *de novo* by build-up of overlapping oligonucleotides including restriction sites for cloning into mammalian expression vectors as well as a human signal sequence. *HindIII* and *SpeI* restriction sites were introduced to frame the VH domain containing the signal sequence for cloning into mammalian expression vectors containing the human γ1 constant region. *HindIII* and *BsiVIII* restriction sites were introduced to frame the VL domain containing the signal sequence for cloning into mammalian expression vector containing the human kappa constant region.

2.4 Expression of the recombinant CA8 antibodies (including antibody quantification)

Expression plasmids encoding the heavy and light chains respectively were transiently co-transfected into HEK 293 6E cells and expressed at small scale to produce antibody. Antibodies were quantified by ELISA. ELISA plates were coated with anti human IgG (Sigma I3382) at 1mg/ml and blocked with blocking solution (4% BSA in Tris buffered saline). Various dilutions of the tissue culture supernatants were added and the plate was incubated for 1 hour at room temperature. Dilutions of a known standard antibody were also added to the plate. The plate was washed in TBST and binding was detected by the addition of a peroxidise labelled anti human kappa light chain antibody (Sigma A7164) at a dilution of 1/1000 in blocking solution. The plate was incubated for 1 hour at room temp before washing in TBST. The plate was developed by addition of OPD substrate (Sigma P9187) and colour development stopped by addition of 2M H2SO4. Absorbance was measured at 490nm and a standard curve plotted using data for the known standard dilutions. The standard curve was used to estimate the concentration of antibody in the tissue culture supernatants. Larger scale antibody preparations were purified using protein A and concentrations were measured using a Nanodrop (Thermo Scientific).

Table 1. Design of CA8 variable heavy and light humanised variants

| Humanised VH | Template | Backmutations (Kabat#) |
|---|---|---|
| J0 | Straight graft of CA8 VH CDRs onto IGHV1_69 + JH1 minigene | None |
| J1 | J0 | G27Y. S30T |
| J2 | J1 | A93T |
| J3 | J2 | A24G. K73T |
| J4 | J3 | M4RI. V67A. |
| J5 | J3 | N99D |
| J6 | J0 | N99D |
| J7 | J1 | N99D |
| J8 | J2 | N99D |
| J9 | J4 | N99D |
| M0 | Straight graft of CA8 VL CDRs onto IGKV1_39 + JK2 minigene | None |
| M1 | M0 | F71Y |
| M2 | M1 | M4L. K45E. |

2.5 Defucosylated antibody production

To generate defucosylated antibodies the heavy and light chains respectively were co-transfected into CHO DG44 MS705 BioWa cells and expressed at scale to produce antibody. Briefly, 30μg DNA was linearised overnight with Not1, the DNA was ethanol precipitated and re-dissolved in TE buffer. From culture, 2.4X107 BioWa DG44 cells were obtained and washed in 14ml of warmed PBS-sucrose. The cells were spun and the pellet resuspended in 1.6ml of PBS-sucrose. Half (0.8ml) of aforementioned cells, suspended in PBS-sucrose, were added to a BioRad cuvette with the 30μg of linearised DNA (in 50μl TE buffer). A BioRad GenePulserwas programmed to 380V with a capacitance of 25μF and the cuvette was entered for electroporation. The resulting 850ul of electroporated cells and DNA were added to (80ml) warmed SFM512 medium (including phenol red, 2XHT (nucleosides), glutamax and Gibco supplement4). Finally, the resulting 80ml of cell suspension was transferred □(150μl/well) to each well of one of 4 X 96-well plates. After 48 hours, the medium was changed to nucleoside free by removing approximately 130μl of conditioned and replacing with 150μl of fresh selection medium SFM512 medium (including phenol red and glutamax). Every 3-4 days, 130-150μl of conditioned medium was removed and replaced with fresh, selection medium. Wells were monitored for colour change and assayed for IgG concentration as discussed previously.

2.6 Additional antibodies - Cloning of Hybridoma Variable Regions

Total RNA was extracted from S307118G03, S332121F02, S332126E04, S322110D07, S336105A07, S335115G01 and S335122F05 hybridoma cells. Heavy and light variable domain cDNA sequence was then generated by reverse transcription and polymerase chain reaction (RT-PCR). The forward primer for RT-PCR was a mixture of degenerate primers specific for murine immunoglobulin gene leader-sequences and the reverse primer was specific for the antibody constant regions, in this case isotype IgG2a. Primers were designed based on a strategy described by Jones and Bendig (Bio/Technology 9:88, 1991). RT-PCR was carried out for both V-region sequences to enable subsequent verification of the correct V-region sequences. DNA sequence data was obtained for the V-region products generated by the RT-PCR.

2.7 Additional antibodies - Cloning of the chimeras

The DNA expression constructs encoding the chimeric antibodies were prepared de novo by infusion advantage PCR cloning (Clonetech) of the V-gene PCR products into mammalian expression vectors. This cloning method enabled fusion the murine variable regions to human IgG1 H chain and kappa L chain constant regions.

2.8 S307118G03 - Cloning of the humanized variants

Cloning was carried out as for paragraph 2.3.

2.9 S307118G03 Expression of the recombinant antibodies

Expression plasmids encoding the relevant heavy and light chains (listed in Table 8 below) were transiently co-transfected into HEK 293 6E cells and expressed at small scale to produce antibody. The antibodies were Protein A purified from the supernatants and quantified using the Nanodrop spectrophotometer. 8 below) were transiently co-transfected into HEK 293 6E cells and expressed at small scale to produce antibody. The antibodies were Protein A purified from the supernatants and quantified using the Nanodrop spectrophotometer.

**Example 3 Conjugation of antibodies to vcMMAE and mcMMAF to form antibody drug conjugates (ADC)**

**Table B Chemical structures of drug-linkers**

| |
|---|
| 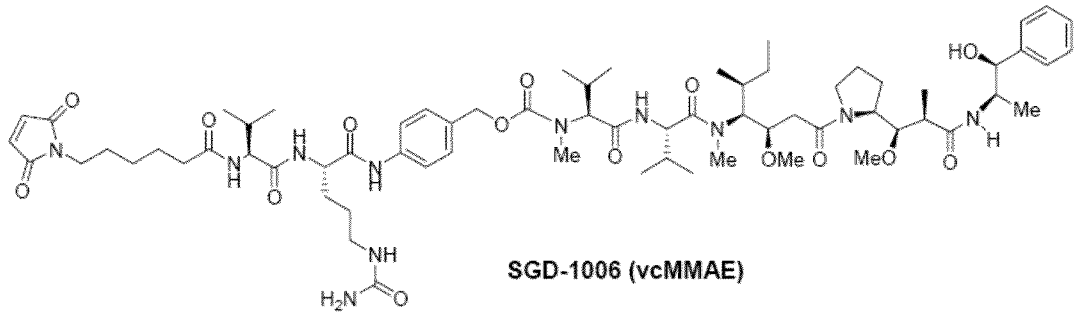 **SGD-1006 (vcMMAE)** |
| 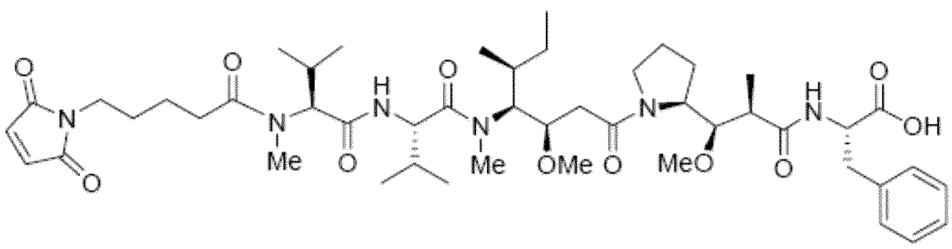 **SGD-1269 (mcMMAF)** |

Gammabind Plus Protein G Sepharose (GE Healthcare) resin slurry (75 uL) was added to a each well of a deep well (2 mL capacity) filter plate. The antibodies to be conjugated were grouped by species and isotype and up to 0.5 mg of each antibody transferred to each well of the plate. Each antibody was transferred to two separate wells to facilitate the preparation of two conjugates, with the drug-linkers SGD-1006 and SGD-1269. The filter plate was then shaken at 1200 RPM for 2 hours at 5 °C to bind the antibodies to the resin. The filter plate was then centrifuged at 500x g for 3 minutes to ensure complete pulldown of all fluids and resin to the bottom of the each well.

The bound antibodies were then reduced by adding 500 uL of 10 mM TCEP in 100 mM KPO4, 150 mM NaCl, pH 7, 1 mM EDTA and shaking for 30 minutes at 22 °C. Following reduction, the plate was again centrifuged to remove the TCEP solution and subsequently washed with PBS + 1 mM EDTA, 1 mL per well. The wash solution was removed

by centrifugation and the process repeated 3 times for a total of 4 washes. The bound and reduced antibodies were then conjugated using a mixture of NEM and drug linker prepared in accordance with the mole fractions indicated in Table 2.

**Table 2.**

| Antibody (species / isotype) | Reducible Disulfides | SGD-1006 mole fraction | SGD-1269 mole fraction |
|---|---|---|---|
| Human IgG1* | 4 | 0.675 | 0.688 |
| Murine IgG1 | 5 | 0.500 | 0.586 |
| Murine IgG2a | 5 | 0.500 | 0.586 |
| Murine IgG2b | 6 | 0.463 | 0.481 |
| * also for murine / human IgG1 chimerics | | | |

Separate mixtures of NEM and drug linker were thus prepared for each antibody species / isotype using 10 mM DMSO stock solutions of SGD-1006, SGD-1269 (See Table B) and NEM. When mixed at the appropriate ratio the total maleimide concentration was therefore still 10 mM, and this value was used to calculate the volume of maleimide solution to be added to each well. For example for a murine IgG1 with 5 reducible disulfides (10 available thiols when reduced) 0.5 mg of antibody at 150 kD is 3.33 nmol corresponding to 33.3 nmol of thiol. A 3-fold excess is therefore 100 nmol of total maleimide or 10 μL of the 10 mM drug linker / NEM mix. For the SGD-1269 conjugate this mix would then be prepared with 5.86 μL of SGD-1269 and 4.14 μL of NEM. The maleimide mix would then be diluted into 500 μL of PBS prior to addition to the immobilized reduced antibody. In practice, since multiple antibodies of each isotype were conjugated simultaneously a single SGD-1269 / NEM mixed solution for each isotype was prepared by multiplying the number of wells containing that isotype by 10 μL per well then diluting into a volume of PBS equal to 500 μL times the number of wells. In like fashion a total of eight drug-linker / NEM mixes were prepared-four with SGD-1006 and four with SGD-1269-and diluted into PBS. These mixes were then added to the reduced antibodies (500 μL per well) and the plate was shaken for 30 minutes at 22 °C. The plate was then centrifuged as above to remove the excess reaction solution, and subsequently washed 4 times with PBS as before.

The bound ADCs were then eluted by adding 200 uL of 50 mM glycine pH 2.5 to each well and shaking the plate for 3 minutes at 1200 RPM. While shaking 20 uL of neutralization buffer (1M potassium phosphate, pH 7.4, 500 mM NaCl, 0.2% Tween-20) was added to each well of a 1 mL collection plate. The ADCs were then eluted into the collection plate by spinning at 1500x g for 6 minutes. The collection plate was then shaken briefly to ensure complete mixing of the neutralization buffer.

The concentration of each ADC was then determined with an absorbance plate reader by transferring the solutions into a UV assay plate (Costar model 3635, Corning) and measuring the optical density at 280 nm. An average IgG extinction coefficient of 1.45 mL mg-1 cm-1 was used to provide an adequate estimation of ADC concentration across the panel. To confirm successful conjugation, a reversed phase protein HPLC method (described below) was used to estimate the drug loading of the isotype controls. For the plate containing the humanization variants of CA8 this method was used to estimate the loading of all ADCs directly.

The reversed phase protein chromatography method for determining drug loading employs the PLRP-S polymeric stationary phase (Agilent Technologies). Since the antibodies were fully reduced during the conjugation process all of the antibody subunits elute from the column as single polypeptide chains allowing the subpopulations of light and heavy chain species with varying levels of drug loading to be evaluated separately. Thus, the analysis of these data allow for the calculation of the average light chain drug loading and the average heavy chain drug loading as independent factors which can then be combined to determine average antibody drug loading with the basic knowledge that each antibody is comprised of two light and two heavy chains. The chromatographic conditions were as follows: A PRLP-S column, 1000 Å, 50 × 2.1 mm, 8 um particle size (Agilent Technologies) with water + 0.05% TFA as mobile phase A and acetonitrile + 0.01% TFA as mobile phase B; elution with a linear gradient of 27% B to 42% B in 12.5 minutes.

Anti-BCMA antibodies were conjugated with SGD-1006 and SGD-1269 in three separate batches over a period of seven months. In the first batch a total of 29 antibodies were conjugated (resulting in 58 ADCs). The drug loading of each isotype control determined by PLRP chromatography and the data are summarized in Table 3.

**Table 3.**

| Isotype | SGD-1006 loading | SGD-1269 loading |
|---|---|---|
| cIgG1 (control P) | 4.23 | 4.35 |

(continued)

| Isotype | SGD-1006 loading | SGD-1269 loading |
|---|---|---|
| cIgG1 (control M) | 4.42 | 4.41 |
| mIgG1 | 4.26 | 4.04 |
| mIgG2a | 4.51 | 4.57 |
| mIgG2b | 4.39 | 4.18 |

For the second batch an additional 25 antibodies were conjugated (resulting in 50 ADCs). The drug loading of each isotype control was again determined by PLRP chromatography and the data are summarized in Table 4.

**Table 4.**

| Isotype | SGD-1006 loading | SGD-1269 loading |
|---|---|---|
| cIgG1 | 3.96 | 3.78 |
| mIgG1 | 3.95 | 3.32 |
| mIgG2a | 4.53 | 3.60 |
| mIgG2b | 4.32 | 3.49 |

In the third batch 30 antibodies were conjugated (resulting in 60 ADCs), including 13 humanized variants of CA8. In this final batch, the drug loading of all ADCs were determined and are summarized in the following two plate maps. (Table 5 & 6)

**Table 5.**

| | drug loading | | | | | | | | | |
|---|---|---|---|---|---|---|---|---|---|---|
| | 1 | 2 | 3 | 4 | 5 | 6 | 7 | 8 | 9 | 10 |
| A | 3.7 | 4.0 | 3.6 | 3.8 | 3.8 | 3.5 | 3.9 | 2.8 | 3.8 | 3.8 |
| B | 3.7 | 3.6 | 3.5 | 3.7 | 4.0 | 3.4 | 3.7 | 3.3 | 3.8 | 3.9 |
| C | 3.6 | 3.8 | 3.5 | 3.7 | 3.6 | 3.3 | 3.8 | 4.7 | 3.8 | 3.7 |
| D | 3.4 | 3.6 | 3.6 | 3.9 | 3.9 | 3.4 | 3.2 | 4.8 | 3.8 | 3.9 |
| E | 3.9 | | 3.8 | 3.9 | 3.4 | 3.6 | | 3.3 | 3.7 | 3.4 |
| F | 3.7 | | | 4.0 | 3.6 | 3.5 | | | 3.8 | 3.7 |
| G | 3.6 | | | 3.6 | | 3.4 | | | 3.7 | |
| H | | | | 3.6 | | | | | 3.6 | |
| | SGD-1006 (vc-MMAE) ADCs | | | | | SGD-1269 (mc-MMAF) ADCs | | | | |
| | | | | | | | | | | |
| | 3.7 | 3.8 | 3.6 | 3.8 | | 3.4 | 3.7 | 3.8 | 3.7 | |
| | 4.1% | 5.1% | 3.4% | 4.8% | | 2.8% | 8.5% | 24.1% | 3.4% | |

**Table 6.**

| | mIgG1 | mIgG2a | mIgG2b | humanized | | mIgG1 | mIgG2a | mIgG2b | humamed | |
|---|---|---|---|---|---|---|---|---|---|---|
| | 1 | 2 | 3 | 4 | 5 | 6 | 7 | 8 | 9 | 10 |
| A | control | control | control | CA8 J6MO | CA8 J8M2 | control | control | control | CA8 J6M0 | CA8 J8M2 |
| B | 5336106D07 | 5336105A07 | S336107G0S | CAS J6M1 | CA8 J9M0 | 5336106D07 | 5336105A07 | S336107G08 | CAS J6M1 | CAS J9M0 |
| C | 5335115G03 | 5335122F05 | 5336104A09 | CA8 J6M2 | CA8 J9M1 | 5335115G03 | 5335122F05 | 5336104A09 | CA8 J6M2 | CA8 J9M1 |
| D | 5335115G01 | 5335128A12 | 5335107H11 | CA8 J7M0 | CA8 J9M2 | 5335115G01 | 5335128A12 | 5335107H11 | CA8 J7M0 | CA8 J9M2 |
| E | 533510δE08 | | 5335119E11 | CA8 J7M1 | CA8 Fc ENH | S335106EOS | | 5335119E11 | CA8 T7M1 | CA8 Fc ENH |
| F | 5335132E01 | | | CA8 J7M2 | GRITS28785 | S335132E01 | | | CA8 J7M2 | GRITS28785 |
| G | 5341106G02 | | | CAS J8M0 | | S341106G02 | | | CAS JSM0 | |
| H | | | | CA8 J8M1 | | | | | CA8 J8M1 | |
| | SGD-1 006 (vc-MMAE) ADCs | | | | | SGD-12 269 (mc-MMAF) ADCs | | | | |

Mean drug loading and %CV are indicated for each isotype series at the bottom. An uncharacteristically large variability in drug loading was observed for the SGD-1269 ADCs prepared with mIgG2b antibodies; the reason for this is unclear. Also, the Fc-enhanced CA8 antibodies yielded somewhat lower drug loading levels than the other CA8 human variants; to address this, additional Fc-enhanced CA8 was conjugated in a solution-phase reaction to better match the drug loading achieved for the other antibodies.

**Example 4 - Binding Data**

4.1 FMAT binding assay to show binding of Chimeric CA8 to cells expressing human or cyno BCMA.

Cryopreserved transfected human, cyno BCMA and mock transfected HEK293 cells were recovered from LN2 storage. Assay wells were prepared with human chimeric CA8 antibody, at a range of different concentrations, mixed with human BCMA HEK293, cyno BCMA HEK293 and mock transfected cells respectively. Anti-human IgG FMAT Blue secondary conjugate was added for detection of human chimeric CA8. The assay plates were left for a minimum of 90 minutes before the result was read on the ABI8200 (FMAT) plate reader.
This showed that the CA8 antibody in chimeric form binds well to both human and cyno BCMA proteins expressed on HEK293 cells.
Results are shown in Figure 1.

4.2 ELISA experiment showing binding of chimeric CA8 to recombinant BCMA protein

Chimeric CA8 antibodies were tested for binding to human BCMA and cyno BCMA expressed as Fc fusions. Human BCMA-Fc and cyno BCMA-Fc were coated to ELISA plates and the plates were blocked using BSA to reduce non specific binding. CA8 chimeric antibodies were added in a concentration range from 5ug/ml to 0.1ug/ml to the human and cyno BCMA coated ELISA plates. Any bound human chimeric CA8 antibody was detected using anti-human IgG HRP conjugated secondary antibody as appropriate. HRP substrate (TMB) was added to develop the ELISA. This showed that CA8 antibody binds to recombinant human and cyno BCMA in an ELISA assay.
Results are shown in Figure 2.

4.3 Biacore experiment to show CA8 antibody binding to BCMA and TACI proteins to determine cross reactivity with TACI protein.

CA8 chimera antibody was injected and captured on protein A. (A protein A derivitised sensorchip was used). Residual protein A binding was blocked with an injection of a high concentration of human IgG solution. BCMA-Fc, TACI-Fc or BAFF-R-Fc solutions were then tested for binding to the antibody. The 3 proteins were injected in sequence and binding events were measured. The surface was regenerated between injection of each protein.
Sensorgrams were analysed in the Biaevaluation program. Double reference subtraction was done to remove instrument noise and any non-specific binding from the sensorgram curves.
This showed that CA8 was specific for binding to BCMA binding and not to TACI and BAFFR.
Binding of the CA8 antibody to BCMA-Fc, TACI-Fc and BAFF-R-Fc was plotted out as shown in Figure 3.

4.4 Cell binding and neutralisation data

4.4.1 Binding of murine anti BCMA antibodies to multiple myeloma cells and BCMA expressing cells

Multiple myeloma cell line H929 and ARH77-hBCMA 10B5 BCMA expressing transfectant cells were stained with murine S332211D07, S3332121 F02 or S332126E04 or murine isotype control at 5 μg/mL. Multiple myeloma cell line H929 was stained with murine S307118G03. Cells were incubated for 20 mins at room temperature (RT) and then washed with FACS buffer (PBS + 0.5% BSA +0.1% sodium azide) to remove unbound antibody. Cells were incubated with a secondary PE labelled antimouse IgG antibody for 15 minutes at RT and then washed with FACS buffer to remove unbound antibody. Cells were analysed by FACS to detect antibody bound to the cells.
The results (Figure 4) showed that all 4 murine antibodies bound to the H929 multiple myeloma cell line and the three antibodies tested on ARH77 BCMA transfected cells bound to these.

4.4.2 Binding curve of chimeric CA8 to multiple myeloma cells as determined by FACS

A panel of multiple myeloma cell lines were used to determine the binding of chimeric CA8. Cell lines H929, OPM-2, JJN-3 and U266 were stained with either chimeric CA8 or irrelevant antibody (Synagis) at varying concentrations

for 20 minutes at RT. Cells were then washed with FACS buffer (PBS + 0.5% BSA + 0.1% sodium azide) to remove unbound antibody. Cells were incubated with a secondary PE labelled anti-human IgG antibody for 15 minutes at RT and then washed with FACS buffer to remove unbound antibody. Cells were analysed by FACS and mean fluorescence intensity (MFI) values measured to determine binding.

Results showed that chimeric CA8 bound to multiple myeloma cell lines H929, OPM-2, JJN-3 & U266 in a dose dependent manner (Figure 5).

4.4.3 Binding of humanised CA8 to BCMA transfected cells as determined by FACS

ARH77-hBCMA 10B5 BCMA expressing transfectant cells or H929 cells were stained with either chimeric CA8 or humanised variants of CA8 designated J6M0, J6M1, J6M2, J9M0, J9M1, J9M2 at varying concentrations for 20 minutes at RT. Cells were then washed with FACS buffer (PBS + 0.5% BSA + 0.1% sodium azide) to remove unbound antibody. Cells were incubated with a secondary PE labelled anti-human IgG antibody for 15 minutes at RT and then washed with FACS buffer to remove unbound antibody. Cells were analysed by FACS and mean fluorescence intensity (MFI) values measured to determine binding.

Results showed that chimeric CA8 and all antibodies tested apart from J9M2 bound to ARH77-hBCMA 10B5 BCMA expressing transfectant cells and H929 cells in a dose dependent manner (Figure 6).

4.5 Demonstration of ability of CA8 and the humanised version J6MO to neutralise binding of BAFF or APRIL to recombinant BCMA.

The aim of this assay was to assess the ability of antibody CA8, and humanised version J6M0 in both wild type and afucosylated (Potelligent) form, at various concentrations, to neutralise the binding ability of either BCMA ligand, BAFF or APRIL.

96 well flat bottomed plates were coated overnight with 1μg/mL solution of recombinant human BCMA Fc 4-53 in PBS. Following a wash step using 0.05% TWEEN20, plates were blocked with 2% Bovine Serum Albumin solution in PBS for 1 hour at room temperature. Plates were washed as before and 40μL of each antibody (murine IgG, murine CA8, and chimeric CA8), starting at 10μg/mL, titrated at 1 in 2 in duplicate was added to the relevant wells and incubated for 1hour at room temperature. 40μL of 2% BSA was added to the relevant control wells. 10μL of either recombinant human BAFF (2149-BF/CF, R&D Systems) or recombinant human APRIL (5860-AP/CF, R&D Systems) was added at 30ng/mL and 750ng/mL respectively, giving a final concentration of 6ng/mL and 150ng/mL respectively in each well. Equivalent volume of 2% BSA was added to the relevant control wells. Plates were allowed to incubate for 2 hours at room temperature, after which they were washed as before. Biotinylated anti-human ligand (BAFF BAF124 or APRIL BAF884, R&D Systems) was added to the relevant wells at 50ng/mL and incubated for 1 hour. Following a wash step, 50μL of a 1:4000 dilution of Streptavidin-HRP (Amersham RPN4401) was added to each well and incubated for 30 minutes at room temperature. The wash process was repeated again followed by the addition of 100μL of Tetramethylbenzidine substrate solution (T8665, Sigma) into each well. Plates were incubated for 20-25 minutes at room temperature, wrapped in foil. The reaction was stopped with the addition of 100μL of 1M $H_2SO_4$. Optical density was determined at 450nm using Spectromax reader. See Figure 7A and B.

In a plate based assay for neutralisation of binding of BAFF or APRIL to BCMA, the EC50 values calculated for chimeric CA8 were 0.695μg/mL and 0.773μg/mL respectively. The values for the humanised J6M0 were 0.776ng/ml and 0.630ng/ml. The vaues for the J6M0 potelligent version were 0.748 and 0.616ng/ml respectively.

4.6 Effect of chimerised CA8 and humanised J6MO BCMA antibody on BAFF or APRIL induced phosphorylation of NFkB in H929 cells.

In one set of experiments, H-929 cells were plated at 75,000cells/well in a 96 well plate in serum free medium. The chimeric CA8 antibody was added 24 hours later to give final well concentrations up to 200ug/ml. Ten minutes later, BAFF or APRIL ligand were added to the cells to give final well concentrations of 0.6 or 0.3ug/ml respectively. After 30 minutes the cells were lysed and phosphorylated NfkappaB levels measured using a MSD pNFkappaB assay.

The chimeric BCMA antibody CA8 neutralised both BAFF and APRIL induced NfkappaB cell signalling in H-929 cells. It was particularly potent at neutralising BAFF induced NfkappaB cell signalling in this cell type with a mean IC50 of 10nM, compared to 257nM for APRIL induced NfkappaB cell signalling.

Meaned data for 2 experiments

IC50s were 10nM for BAFF induced NfkappaB neutralisation and 257nM for APRIL induced NfkappaB neutralisation (mean of 2 independent experiments) are shown in Table 7.

**Table 7**

| | **BAFF induced IC50** | | **APRIL induced IC50** | |
|---|---|---|---|---|
| | ug/ml | **nM** | ug/ml | **nM** |
| BCMA antibody CA8 | **1.5** | **10** | **38.5** | **256.7** |

A further set of experiments were carried out to aim to understand why there was such a discrepancy between the potency in neutralisation of APRIL and BAFF in the cell based system. Following the discovery of the soluble form of BCMA the experimental design was changed to include a step where the H929 cells were washed prior to the assay to reduce the interference from the antibody binding to soluble BCMA. H-929 cells were washed 3 times to remove any sBCMA and resuspended in serum free medium. J6M0 potelligent antibody was added to a 96 well plate to give a final well concentrations up to 100ug/ml along with BAFF or APRIL ligand to give a final well concentration of 0.6 or 0.2 ug/ml respectively. H-929 cells were then plated at 7.5×104cells/well in serum free medium. 30 minutes later the cells were lysed and phosphorylated NFkappaB levels measured using a MSD pNFkappaB assay. This is data from one experiment. Each data point is the mean/sd of two replicates. The data from this experiment is shown in Figure 7c. The IC50s for inhibition of BAFF and APRIL signalling were determined as 0.91ug/ml and 2.43ug/ml respectively.

4.7 ProteOn analysis of anti-BCMA CA8 chimeric and humanised constructs

The initial screen of CA8 chimeric and humanised variants was carried out on the ProteOn XPR36 (Biorad). The method was as follows; Protein A was immobilised on a GLC chip (Biorad, Cat No: 176-5011) by primary amine coupling, CA8 variants were then captured on this surface and recombinant human BCMA (in house or commercial US Biological, B0410) materials (run 2 only)) passed over at 256, 64, 16, 4, 1nM with a 0nM injection (i.e. buffer alone) used to double reference the binding curves, the buffer used is the HBS-EP buffer. 50mM NaOH was used to regenerate the capture surface. The data was fitted to the 1:1 model using the analysis software inherent to the ProteOn XPR36.. Run 1 corresponds to the first screen of humanised CA8 variants (J0 to J5 series) and run 2 to the second screen of humanised CA8 variants (J5 to J9 series). Both runs were carried out at 25°C.

The data obtained from run1 are set out in Table 8 and data from run 2 are set in Table 9 Several molecules in the Run 2 (Table 09) failed to give affinity values measurable by ProteOn, this was due to the off-rate being beyond the sensitivity of the machine in this assay, this does however indicate that all these molecules bind tightly to recombinant human BCMA. From Run 1 the data indicates that some constructs did not show any binding to recombinant cyno BCMA.

**Table 8**: Run 1-Kinetics analyses of anti-BCMA molecules against Recombinant Human BCMA

| | **Human in house BCMA** | | | **Cyno in house BCMA** | | |
|---|---|---|---|---|---|---|
| **Sample name** | **ka** | **kd** | **KD (nM)** | **ka** | **kd** | **KD (nM)** |
| CA8 humanised J5M0 | 2.16E+05 | 1.88E-05 | 0.087 | 3.25E+05 | 8.14E-06 | 0.025 |
| CA8 humanised J5M2 | 2.67E+05 | 3.21E-05 | 0.12 | 4.30E+05 | 4.70E-05 | 0.109 |
| CA8 humanised J5M1 | 2.97E+05 | 4.32E-05 | 0.145 | 4.81E+05 | 5.41E-05 | 0.112 |
| CA8 humanised J4M1 | 2.54E+05 | 7.04E-05 | 0.278 | 3.50E+05 | 7.10E-05 | 0.203 |
| CA8 humanised J4M2 | 2.51E+05 | 7.06E-05 | 0.281 | 3.44E+05 | 6.15E-05 | 0.179 |
| CA8 humanised J0M2 | 2.25E+05 | 6.97E-05 | 0.31 | 3.26E+05 | 1.84E-04 | 0.563 |
| CA8 humanised J3M2 | 2.66E+05 | 9.64E-05 | 0.362 | 3.69E+05 | 5.87E-05 | 0.159 |
| CA8 humanised J0M1 | 2.31E+05 | 8.60E-05 | 0.373 | 3.32E+05 | 1.67E-04 | 0.503 |
| CA8 humanised J0M0 | 2.45E+05 | 1.06E-04 | 0.435 | 3.58E+05 | 2.32E-04 | 0.648 |
| CA8 humanised J3M1 | 2.85E+05 | 1.25E-04 | 0.438 | 4.04E+05 | 7.93E-05 | 0.196 |
| CA8 humanised J2M2 | 2.05E+05 | 9.87E-05 | 0.482 | 2.98E+05 | 3.17E-05 | 0.106 |
| CA8 Chimera | 2.41E+05 | 1.25E-04 | 0.519 | 3.82E+05 | 1.74E-04 | 0.457 |
| CA8 humanised J2M1 | 2.04E+05 | 1.72E-04 | 0.842 | 2.96E+05 | 6.46E-05 | 0.218 |

(continued)

| | Human in house BCMA | | | Cyno in house BCMA | | |
|---|---|---|---|---|---|---|
| **Sample name** | **ka** | **kd** | **KD (nM)** | **ka** | **kd** | **KD (nM)** |
| CA8 humanised J4M0 | 2.42E+05 | 2.20E-04 | 0.906 | 3.34E+05 | 2.89E-04 | 0.866 |
| CA8 humanised J1M2 | 2.15E+05 | 2.46E-04 | 114 | 3.19E+05 | 9.67E-05 | 0.303 |
| CA8 humanised J3M0 | 2.08E+05 | 2.85E-04 | 1.37 | 2.93E+05 | 1.54E-04 | 0.526 |
| CA8 humanised J1M1 | 2.27E+05 | 3.43E-04 | 1.51 | 3.33E+05 | 1.47E-04 | 0.442 |
| CA8 humanised J2M0 | 1.95E+05 | 3.77E-04 | 194 | 2.81E+05 | 1.51E-04 | 0.538 |
| CA8 humanised J1M0 | 1.78E+05 | 5.02E-04 | 2.82 | 2.47E+05 | 2.10E-04 | 0.849 |
| S307118G03 Chimera | 4.75E+05 | 1.95E-03 | 4.11 | No | Analysable | Binding |
| S307118G03 humanised H3L1 | 4.69E+05 | 2.28E-03 | 4.86 | No | Analysable | Binding |
| S307118G03 humanised H3L0 | 2.86E+05 | 1.52E-03 | 5.31 | No | Analysable | Binding |
| S307118G03 humanised H2L0 | 3.78E+05 | 2.41E-03 | 6.36 | No | Analysable | Binding |
| S307118G03 humanised H2L1 | 3.38E+05 | 2.15E-03 | 6.37 | No | Analysable | Binding |
| S307118G03 humanised H4L1 | No | Analysable | Binding | No | Analysable | Binding |

**Table 9.** Run 2-Kinetics analyses of anti-BCMA molecules against Recombinant Human BCMA

For antibodies J8M0, J9M0, J8M1, J9M2, J7M2, J5M0, J7M1, J7M0, J8M2, J9M1, J5M2, J5M1 the off rate was beyond the sensitivity of the assay hence no data shown.

| | **Human in house BCMA** | | | **commercial human BCMA** | | | **Cyno in house BCMA** | | |
|---|---|---|---|---|---|---|---|---|---|
| **Sample Name** | **ka** | **kd** | **KD (nM)** | **ka** | **kd** | **KD (nM)** | **ka** | **kd** | **KD (nM)** |
| CA8 Chimera | 2.51E+05 | 1.03E-04 | 0.412 | 7.05E+05 | 9.79E-05 | 0.139 | 5.89E+04 | 1.21E-04 | 2.060 |
| CA8 humanised J6M1 | 2.17E+05 | 2.70E-05 | 0.124 | 5.92E+05 | 3.75E-05 | 0.063 | 4.88E+04 | 2.58E-04 | 5.300 |
| CA8 humanised J6M0 | 2.40E+05 | 7.40E-05 | 0.308 | 6.23E+05 | 5.37E-05 | 0.086 | 5.64E+04 | 3.18E-04 | 5.630 |
| CA8 humanised J6M2 | 2.01E+05 | 4.06E-05 | 0.202 | 5.63E+05 | 3.97E-05 | 0.071 | 4.41E+04 | 3.02E-04 | 6.860 |
| S307118G03 H5L0 | No Analysable Binding | | | V weak signal | | | No | Analysable | Binding |
| S307118G03 H5L1 | No Analvsable Binding | | | V weak signal | | | No | Analysable | Binding |
| S307118G03Chimera | 4.79E+05 | 1.65E-03 | 3.44 | 1.55E+06 | 1.48E-03 | 0.956 | No | Analysable | Binding |

4.8 BIAcore analysis of anti-BCMA CA8 chimeric and humanised constructs (J7 to J9 series)

Protein A was immobilised on a CM5 chip (GE Healthcare, Cat No: BR-1005-30) by primary amine coupling and this surface was then used to capture the antibody molecules. Recombinant human BCMA (US Biological, B0410) was used as analyte at 256nM, 64nM, 16nM, 4nM and 1nM. Regeneration of the capture surface was carried out using 50mM NaOH. All binding curves were double referenced with a buffer injection (i.e. 0nM) and the data was fitted to the using the 1:1 model inherent to T100 evaluation software. The run was carried out at 37°C, using HBS-EP as the running buffer.

The results showed the molecules tested with the exception of J9M2 bind to recombinant human BCMA, with similar affinity as the chimeric molecule. Data generated from this experiment are presented in table 10.

**Table 10:** Kinetics analysis of anti-BCMA humanised molecules against Recombinant Human BCMA

| | **Human commercial BCMA** | | | **Cyno in house BCMA** | | |
|---|---|---|---|---|---|---|
| **Sample name** | **ka** | **kd** | **KD (nM)** | **ka** | **kd** | **KD (nM)** |
| CA8 humanised J9M1 | 1.96E+07 | 3.50E-04 | 0.018 | 6.77E+05 | 2.99E-04 | 0.442 |
| CA8 humanised J9M0 | 4.95E+06 | 1.74E-04 | 0.035 | 7.03E+05 | 3.24E-04 | 0.46 |
| CA8 Chimera | 3.27E+07 | 1.18E-03 | 0.036 | 1.15E+06 | 3.49E-04 | 0.305 |
| CA8 humanised J8M1 | 2.66E+06 | 1.34E-04 | 0.05 | 2.82E+05 | 3.62E-04 | 1.284 |
| CA8 humanised J8M0 | 2.44E+06 | 1.26E-04 | 0.052 | 3.89E+05 | 4.18E-04 | 1.076 |
| CA8 humanised J7M1 | 2.35E+06 | 1.31E-04 | 0.056 | 3.70E+05 | 3.91E-04 | 1.057 |
| CA8 humanised J8M2 | 2.63E+06 | 1.50E-04 | 0.057 | 3.83E+05 | 5.06E-04 | 1.324 |
| CA8 humanised J7M2 | 2.37E+06 | 1.35E-04 | 0.057 | 3.46E+05 | 4.47E-04 | 1.293 |
| CA8 humanised J7M0 | 2.36E+06 | 1.51E-04 | 0.064 | 3.21 E+05 | 3.67E-04 | 1.143 |
| CA8 humanised J9M2 | No | Analysable | Binding | 4.88E+05 | 2.52E-04 | 0.515 |

4.9 BIAcore analysis of anti-BCMA CA8 chimeric and humanised constructs J6M0 and J9M0

Protein A was immobilised on a CM5 chip (GE Healthcare, Cat No: BR-1005-30) by primary amine coupling and this surface was then used to capture the antibody molecules. Recombinant human BCMA (US Biological, B0410) was used as analyte at 256nM, 64nM, 16nM, 4nM and 1nM. Regeneration of the capture surface was carried out using 50mM NaOH. All binding curves were double referenced with a buffer injection (i.e. 0nM) the data was fitted to the using the 1:1 model inherent to T100 evaluation software. The run was carried out at 25°C and 37°C for experiment 1 and only 37°C for experiment 2 using HBS-EP as the running buffer.

The both runs identified J9M0 as the best molecule in term of overall affinity to human BCMA. Data generated from this experiment are presented in table 11.

**Table 11** Kinetics analyses of anti-BCMA humanised molecules against Human BCMA

| | **Human commercial BCMA** | | | | | | | | |
|---|---|---|---|---|---|---|---|---|---|
| | **25°C** | | | **37°C** | | | | | |
| | **Experiment 1** | | | **Experiment 1** | | | **Experiment 2** | | |
| **Sample** | **ka** | **kd** | **KD (nM)** | **ka** | **kd** | **KD (nM)** | **ka** | **kd** | **KD (nM)** |
| J9M0 | 1.59E+06 | 3.38E-05 | 0.021 | 3.75E+06 | 1.58E-04 | 0.042 | 3.62E+06 | 1.89E-04 | 0.052 |
| J6M0 | 1.01E+06 | 1.22E-04 | 0.121 | 2.12E+06 | 1.48E-03 | 0.698 | 3.78E+06 | 1.88E-03 | 0.498 |
| Chimera CA8 | 1.88E+06 | 2.63E-04 | 0.140 | 1.72E+07 | 8.72E-04 | 0.051 | 1.88E+07 | 1.04E-03 | 0.055 |

4.10. ProteOn analysis of new anti-BCMA chimeric constructs

The initial screen of the new chimeric variants from the second batch of hybridomas was carried out on the ProteOn XPR36 (Biorad). The method was as follows; Protein A was immobilised on a GLM chip (Biorad, Cat No: 176-5012) by primary amine coupling, anti-BCMA variants were then captured on this surface and recombinant human BCMA (in house material) passed over at 256, 64, 16, 4, 1nM with a 0nM injection (i.e. buffer alone) used to double reference the binding curves, the buffer used is the HBS-EP buffer. Regeneration of the capture surface was carried out using 50mM NaOH. The data was fitted to the 1:1 model using the analysis software inherent to the ProteOn XPR36. The run was carried out at 25°C.

Data generated from this experiment are presented in table 12.

**Table 12:** Kinetics analyses of anti-BCMA humanised molecules against Human BCMA

| | In house human BCMA | | |
|---|---|---|---|
| **Sample name** | **ka** | **kd** | **KD (nM)** |
| S332110D07 | 3.11E+05 | 3.77E-03 | 12.100 |
| S332121F02 | 3.73E+05 | 6.45E-03 | 17.300 |

**Example 5 Cell Killing Assays.**

5.1 ADCC potencies of chimeric CA8 and defucosylated chimeric CA8 version in ARH77 cells expressing BCMA

Human natural killer (NK) cells were incubated with europium labelled ARH77 BCMA transfected target cells (10B5) in the presence of varying concentrations of antibody at an E:T ratio of 5:1 for 2 hours. Europium release from the target cells was measured and specific lysis calculated.

Result: Chimeric CA8 and defucosylated chimeric CA8 killed BCMA expressing target cells via ADCC. The defucosylated chimeric antibody showed more potent ADCC activity, as measured by a higher percent lysis achieved with all the target cells tested and a ten-fold lower $EC_{50}$ on the high BCMA expressing target cell line 10B5, compared to the parent chimeric antibody. See Figure 8A and 8B.

5.2 ADCC activity of CA8 humanised antibodies using ARH77 BCMA expressing target cells and PBMC as effectors

Human PBMC were incubated with europium labelled ARH77 BCMA transfected target cells (10B5) in the presence of varying concentrations of humanised versions of CA8 antibody (5ug/ml to 0.005ug/ml) at an E:T ratio of 5:1 for 2 hours. Europium release from the target cells was measured and specific lysis calculated.

Result:

Result: All the J5, J6, J7 J8 & J9 series of humanised variants of CA8 showed ADCC activity against the ARH77 high BCMA expressing cell line 10B5 in a dose dependent manner. ADCC was at a similar level as that found in the experiments using chimeric CA8 molecule. See Figure 9.

5.3 ADCC potencies of chimeric S322110F02, S322110D07 and S307118G03 and humanised S307118G03 H3L0 against ARH77 10B5 cells expressing BCMA with purified NK cells as effector cells

Human natural killer (NK) target cells were incubated with europium labelled ARH77 BCMA transfected target cells (10B5) in the presence of varying concentrations of antibody at an E:T ratio of 5:1 for 2 hours. Europium release from the target cells was measured and specific lysis calculated. Result: all 4 antibodies tested showed ADCC activity against ARH77 10B5 cells. See Figure 10.

5.4 Antibody-Drug Conjugate (ADC) activity of Chimeric CA8 ADCs.

Measuring ADC activity of chimeric CA8 antibody, chimeric CA8-mcMMAF antibody drug conjugates and chimeric CA8-vcMMAE antibody drug conjugates against human multiple myeloma cell lines. Muliple Myeloma cell lines were treated with chimeric CA8 antibody-drug conjugates to determine the ADC concentrations required for growth inhibition and death.

The antibody drug conjugates tested were added to wells containing multiple myeloma cells at concentrations ranging from 1ug/ml to 5ng/ml. The plates were incubated at 37°C for 96 hours at which point viable cells were quantitated using Cell titre Glo. The unconjugated chimeric CA8 antibody showed no significant growth inhibitory activity at the antibody concentrations that were tested. The chimeric CA8-mcMMAF antibody-drug conjugate showed greater growth inhibitory activity than the chimeric CA8-vcMMAE antibody-drug conjugate in all 4 of the multiple myeloma cell lines that were tested. See Figure 11 and Table 13

**Table 13** $IC_{50}$ values represented in ng/mL for the chimeric CA8-vcMMAE and the chimeric CA8-mcMMAF antibody-drug conjugates in 4 different multiple myeloma cell lines

| Multiple Myeloma cell lines | $IC_{50}$ (ng/mL) | |
|---|---|---|
| | CA8 chimera - vcMMAE | CA8 chimera - mcMMAF |
| NCI-H929 | 29.5 | 8.8 |
| U266-B1 | 18.9 | 9.7 |
| JJN3 | 21.8 | 12.4 |
| OPM2 | 92.7 | 58.1 |

5.5 Measuring cell cycle arrest activity of chimeric CA8 antibody, chimeric CA8-mcMMAF antibody drug conjugates and chimeric CA8-vcMMAE antibody drug conjugates against human multiple myeloma cell line H929.

To determine the mechanism that chimeric CA8 Antibody Drug Conjugates (ADC's) cause growth inhibition in multiple myeloma cells, the cell cycle of NCI-H929 cells was monitored by measuring cellular DNA content through fixed cell propidium iodide staining at multiple timepoints following chimeric CA8 antibody and chimeric CA8 ADC treatment.

At the chimeric CA8 ADC concentration tested (50ng/mL), the chimeric CA8-mcMMAF ADC caused significant G2/M cell cycle arrest (4N DNA content) which peaked at 48 hours. At the later timepoints 48, 72 and 96 hours, treatment with the chimeric CA8-mcMMAF ADC resulted in accumulation of a cell population with sub-2N DNA content, which is representative of cell death. At the 50ng/mL concentration tested the chimeric CA8-vcMMAE ADC had no significant effect on G2/M cell cycle arrest or sub-G1 accumulation. See Figure 12.

5.6 Phospho-Histone-H3 (Thr11) staining as a marker for chimeric CA8-mcMMAF antibody drug conjugate and chimeric CA8-vcMMAE antibody drug conjugate induced mitotic arrest.

To determine if the accumulation of cells with 4N DNA content is a specific result of mitotic arrest induced by the chimeric CA8 ADCs NCI-H929 cells were stained with an anti-phospho-Histone H3 antibody following treatment with increasing concentrations of unconjugated chimeric CA8, chimeric CA8-vcMMAE or chimeric CA8-mcMMAFfor 48 hours.

Treatment with chimeric CA8 ADCs resulted in a dose-dependent accumulation of NCI-H929 cells that stained positive for 65eroxidi-Histone H3 (Thr11), a specific marker of mitotic cells. The chimeric CA8-mcMMAF ADC caused accumulation of 65eroxidi-Histone H3 positive cells at lower concentrations than the chimeric CA8-vcMMAE ADC. See Figure 13.

5.7 Measuring apoptosis in NCI-H929 cells in response to chimeric CA8 ADCs by staining for Annexin V.

To determine if the accumulation of cells with sub-2N DNA content is a specific result of apoptosis induced by the chimeric CA8 ADCs, NCI-H929 cells were stained with an anti-Annexin-V antibody following treatment with increasing concentrations of unconjugated chimeric CA8, chimeric CA8-vcMMAE or chimeric CA8-mcMMAFfor 48 hours. Treatment with chimeric CA8 ADCs resulted in a dose-dependent accumulation of NCI-H929 cells that stained positive for Annexin-V, a specific marker of apoptosis. The chimeric CA8-mcMMAF ADC caused accumulation of Annexin-V positive cells at lower concentrations than the chimeric CA8- vcMMAE ADC. See Figure 14.

5.8 Antibody-Drug Conjugate (ADC) activity of humanised variants of CA8 anti-BCMA antibody-drug conjugates.

Cells were plated in 96-well plates (4,000 cells per well in 100uL of RPMI + 10% FBS)

Naked antibody or ADC was added 6 hours after cell seeding and plates were incubated for 144 hours. Growth inhibition in the presence of the antibodies or ADCs was measured at 144 hours using Cell Titre glo. Data points represent

the mean of triplicate CellTiterGlo measurements. Error bars represent standard error.

Multiple Myeloma cell lines NCI-H929 and OPM2 were treated with humanized CA8 anti-BCMA antibody-drug conjugates to determine the ADC concentrations required for growth inhibition and death. The mcMMAF and vcMMAE antibody-drug conjugate forms of these antibodies showed significant growth inhibitory activity comparable to that found with the CA8 chimera. Variant J6M0 showed higher potency than the chimera and data is shown in figure 15 in H929 cells and OPM2 cells. The mcMMAF antibody-drug conjugate showed greater growth inhibitory activity than the vcMMAE antibody-drug conjugate for all antibodies in both cell lines tested. Results for all humanized variants are shown in Table 14.

**Table 14.** $IC_{50}$ values represented in ng/mL for the anti BCMA antibody-drug conjugates in NCI-H929 and U266-B1 cells

| | NCI-H929 mcMMAF | vcMMAE | OPM2 mcMMAF | vcMMAE |
|---|---|---|---|---|
| | Average IC50 (ng/mL) | Average IC50 (ng/mL) | Average IC50 (ng/mL) | Average IC50 (ng/mL) |
| CA8 chimera | 11.64 | 37.96 | 57.04 | 80.01 |
| CA8 J6M0 | 5.97 | 27.67 | 87.22 | 121.2 |
| CA8 J6M1 | 14.6 | 51.89 | 205.6 | 239.9 |
| CA8 J6M2 | 9.5 | 39.71 | 112.9 | 144.7 |
| CA8 J7M0 | 18.97 | 52.25 | 93.27 | 127.1 |
| CA8 J7M1 | 17.87 | 43.97 | 95.35 | 107.5 |
| CA8 J7M2 | 31.63 | 55.13 | 102.6 | 115.9 |
| CA8 J8M0 | 15.67 | 59.94 | 89.95 | 132 |
| CA8 J8M1 | 17.04 | 46.55 | 82.96 | 115.8 |
| CA8 J8M2 | 15.08 | 55.98 | 72.63 | 124.5 |
| CA8 J9M0 | 14.95 | 48.5 | 58.6 | 109.8 |
| CA8 J9M1 | 15.19 | 55.1 | 55.88 | 115 |
| CA8 J9M2 | 20.87 | 55.77 | 80.35 | 111.7 |

5.9 Antibody-Drug Conjugate (ADC) activity of other murine anti-BCMA antibody-drug conjugates.

Cells were plated in 96-well plates (4,000 cells per well in 100uL of RPMI + 10% FBS)

Antibody or ADC was added 6 hours after cell seeding and plates were incubated for 144 hours. Growth inhibition in the presence of the ADCs was measured at 144 hours using Cell Titre glo. The mean of triplicate CellTiterGlo measurements are shown. Table 15a and 15b are from experiments carried out at different times on different series of antibodies. Multiple Myeloma cell lines NCI-H929 and U266-B1 were used for antibodies in Table 15a.

The mcMMAF and vcMMAE antibody-drug conjugate forms of murine antibodies S322110D07, S332121 F02 and S332136E04 showed significant growth inhibitory activity. The mcMMAF antibody-drug conjugate showed greater growth inhibitory activity than the vcMMAE antibody-drug conjugate in all of the murine anti-BCMA antibodies tested where activity was seen. IC50 figures are shown in Table 15a. See Figure 16 for dose response curves for these three antibodies and also S107118G03. Error bars represent standard error. NCI-H929, U266-B1, JJN3 and OPM2 cells for antibodies in Table 15b were treated with a different series of murine anti-BCMA antibody-drug conjugates to determine the ADC concentrations required for growth inhibition and death. IC50 figures are shown in Table 15b. All 5 antibodies shown on the table had significant ADC activity.

**Table 15a.** $IC_{50}$ values represented in ng/mL for the anti BCMA antibody-drug conjugates in NCI-H929 and U266-B1 cells

| | IC50 (ng/mL) | | | |
|---|---|---|---|---|
| **Antibody** | **NCI-H929** | | -**U226-B1** | |
| | **-vcMMAE** | **-mcMMAF** | **- -vcMMAE** | **-mcMMAF** |
| **S322110D07 mIgG1** | **28.4** | **6.7** | **53.3** | **33.3** |
| **S332121F02 mIgG1** | **24.5** | **7** | **- 2.3** | **2.5** |
| **S332126E04 mIgG1** | **46.8** | **9.7** | **- 27.1** | **10.6** |

**Table 15b** $IC_{50}$ values represented in ng/mL for the anti BCMA antibody-drug conjugates in NCI-H929, U266-B1, JJN3 and OPM2 cells

| | NCI-H929 | | U266B1 | | JJN3 | | OPM2 |
|---|---|---|---|---|---|---|---|
| AveraqeIC50 (ng/mL) | vcMMAE | mcMMAF | vcMMAE | mcMMAF | vcMMAE | mcMMAF | vcMMAE |
| S335115G01 | 14.9 | 4.2 | 38.8 | 18.5 | 73.9 | 45.8 | 162.4 |
| S336105A07 | 17.8 | 5.1 | 21.4 | 9.3 | 54.2 | 23.2 | 95.5 |
| S335122F05 | 10.9 | 4.2 | 21.1 | 14.1 | 29.5 | 25.5 | 98.4 |
| S335106E08 | 19.2 | 7.9 | 36.8 | 32.6 | 189.8 | 214.1 | 243.9 |
| S335128A12 | 86.3 | 28.3 | 101.8 | 104.1 | >500 | >500 | >500 |

5.10 ADCC potency of conjugated, afucosylated J6M0 (Potelligent)

Afucosylated J6M0 conjugated to MMAE or MMAF was tested in ADCC assays using BCMA transfectants to ensure that its ADCC activity was not compromised by the conjugation. Europium labelled ARH77-10B5 cells were incubated with various J6M0 WT and Potelligent BCMA antibodies at concentrations up to 1 0000ng/ml for 30 minutes prior to the addition of PBMCs (PBMC: target cell ratio 50:1). Two hours later an aliquot of cell media was sampled and mixed with enhancement solution. After 30 minutes on a plate shaker, europium release was monitored on the Victor 2 1420 multi-label reader. Datapoints represent means of triplicate values. This data is representative of 2 experiments.

There were no significant differences in ADCC potency between the unconjugated and ADC forms of J6M0 Potelligent. In the same experiment a wild type version of J6M0 was included to show how the potency compares to the afucosylated version. As expected, defucosylation resulted in a lower EC50 and higher maximal lysis. No lysis was observed with the Fc disabled form of J6M0. (Figure 17)

5.11 ADCC potency of afucosylated J6M0 on MM cell lines

Human PBMC were incubated with multiple myeloma target cells at an E:T ratio of 50:1 in presence of varying concentrations of afucosylated (Potelligent) J6MO The percentage of target cells remaining in the effector + target cell mixture after 18 hours was measured by FACS using a fluorescently labelled anti-CD138 antibody to detect the target cells and the percent lysis calculated. This is representative of several experiments.

J6M0 Potelligent antibody showed ADCC activity against all five multiple myeloma target cell lines tested. This was important to test since earlier studies were carried out using transfected cells. Results are shown in Figure 18. Full dataset with multiple donors is shown in Table 16 The potencies were all in a similar range as those found with the transfectants. The ADCC activity was not directly related to BCMA surface expression on these cell lines.

Table 16 $EC_{50}$ values generated on 13 independent assays using 11 donors (designated A-K) across the five multiple myeloma cell lines.

| Donor | $EC_{50}$ (ng/ml ) | | | | |
|---|---|---|---|---|---|
| | H929 | RPMI 8226 | JJN-3 | OPM-2 | U266 |
| A | 1.43 | NA | 1.64 | NA | NA |
| R | 057 | NA | NA | NA | NA |
| C | 0.73 | NA | 1.01 | NA | NA |
| C | 1 8 1 | NA | NA | NA | NA |
| A | 2.05 | NA | NA | NA | NA |
| D | NA | 4.09 | NA | NA | NA |
| F | NA | NA | 14.4 | NA | NA |
| F | 2.18 | NA | NA | NA | NA |
| G | NA | NA | 26.3 | NA | NA |
| H | 4.79 | NA | 111.3 | NA | NA |

(continued)

| Donor | $EC_{50}$ (ng/ml ) | | | | |
|---|---|---|---|---|---|
| | H929 | RPMI 8226 | JJN-3 | OPM-2 | U266 |
| I | NA | NA | 40.1 | NA | NA |
| J | 2.19 | 20.4 | 4.89 | NA | NA |
| K | ND | ND | 4.52 | 4.15 | 9.04 |

**Example 6. Xenograft data**

6.1 Murine xenografts of human MM cell lines were tested to ensure that antibody potency detected in vitro can also be demonstrated in vivo. The cell line selected for xenograft studies was NCI-H929 which is sensitive to ADC and ADCC killing in vitro. Studies were carried out in immunocompromised CB.17 SCID mice which lack T and B cells but maintain NK cells to allow for ADCC activity. However it should be noted that although human IgG1 can engage murine Fc receptors, the Potelligent enhancement does not improve the affinity as it does with human Fc receptors.

6.2 Impact of unconjugated and MMAE or MMAF conjugated J6M0 on NCI-H929 tumour growth.

In order to independently analyze both the ADCC and ADC activities of J6M0 we tested J6M0 antibody in the presence and absence of MMAF or MMAE conjugation. By testing the unconjugated J6M0, any anti-tumour effects could be attributed to some combination of ADDC and functional inhibitory activity. Mice with NCI-H929 tumours that had reached a volume of 200 $mm^3$ on average were treated with a human IgG1 control or the J6M0 antibody (unconjugated, MMAE or MMAF) twice weekly at a dose of 50 ug or 100ug, for 2 weeks. Results from this study show that a 100 ug dose of the J6M0-MMAF conjugate resulted in elimination of tumours in those mice which have completed the dosing. The J6M0-MMAF mice were maintained for 40 days after the last dose with no recurrence of tumour occurring. These results from this experiment demonstrate that MMAF conjugation had increased anti-tumour activity over both unconjugated J6M0 antibody and J6M0-MMAE conjugate See Figure 19.

**Example 7 Evaluation of Soluble BCMA Levels from MM Patient Serum**

7.1 It is currently unknown whether BCMA is present extracellularly and can be detected in the blood. In this work, we determined the serum level of human BCMA from MM patients. Serum samples from 54 MM and plasma cell dyscrasia patients and 20 normal control samples were analyzed by ELISA. Human Subject Approval was obtained from Western Institutional Review Board.

7.2 Assessment of Serum Human BCMA Levels

Blood, from patients and normal controls in the clinic, were collected in serum collection tubes. MM patient samples were from a variety of stages (progressive disease, remission, relapsed, newly diagnosed, and others). The Blood samples were spun at 10,000 rpm for 10 minutes and serum transferred into sterile micro-centrifuge plastic tubes.

A Human BCMA/TNFRSF17 ELISA kit from R& D Systems (catalog # DY193E) which measures soluble human BCMA levels was used to detect BCMA following the standard protocol supplied with the kit.

Briefly, 96 well micro-plates were coated with 100ul per well capture antibody and incubated overnight at 4oC. The plates were washed three times with wash buffer (0.05% Tween 20 in PBS, pH 7.2) and blocked with 300ul of 1% BSA in PBS at room temperature for 2 hours. The plates were washed three times with washing buffer. 100ul of serum sample or standard was added into each well and incubated for 2 hours at room temperature. The plates were washed three times with washing buffer and then 100ul of the detection antibody was added to each well and incubated 2 hours at room temperature. 100ul of Streptavidin-HRP was added in each well after washing plates three times and incubated in dark room for 20 minutes. The plates were washed three times and added 50ul stop solution and then determined by micro-plate reader with 570nM wavelength.

A series of assays were carried out in order to determine the serum dilution factor appropriate for the levels of BCMA which were present. A dilution factor of 1:500 was found to be suitable for the majority of samples and is the dilution factor used in the data shown in Figure 20. The full data set is shown in Table 17.

Patient and normal control serum samples diluted and run in triplicates had BCMA levels determined. The serum levels of BCMA were significantly elevated in the sera from MM patients compared with normal controls in this study. When the disease subset was divided further there was a trend towards elevated serum levels of BCMA in the

sera from progressing MM patients compared with those in remission.. This is the first report identifying serum BCMA in any human disease and suggests that these levels may be a novel biomarker for monitoring disease status and therapeutic response of MM patients and for other patients with plasma cell mediated diseases.

Table 17. Figures represent serum concentration of soluble BCMA in ng/ml calculated from samples diluted at 1/50, 1/500 and 1/5000. P values were calculated using the one tailed T-Test and 95% significance values are below the table.

| 1-5000 | Normal | Myeloma: Progressive | Myeloma: Stable | Myeloma: Remission | Myeloma: Other | MGUS | Other Plasma Cell Dyscrasias |
|---|---|---|---|---|---|---|---|
| Mean | 14.130 | 500.804 | 154.762 | 151.201 | 94.457 | 84.912 | 22.838 |
| | | | | | | | |
| 1-500 Triplicate | Normal | Myeloma: Progressive | Myeloma: Stable | Myeloma: Remission | Myeloma: Other | MGUS | Other Plasma Cell Dyscrasias |
| Mean | 15.901 | 215.877 | 81.135 | 43.294 | 97.584 | 53.894 | 22.838 |
| | | | | | | | |
| 1-500 Single | Normal | Myeloma: Progressive | Myeloma: Stable | Myeloma: Remission | Myeloma: Other | MGUS | Other Plasma Cell Dyscrasias |
| Mean | 16.620 | 207.028 | 61.576 | 42.796 | 71.372 | 40.623 | 14.099 |
| 1-50 Trial 1 | Normal | Myeloma: Progressive | Myeloma: Stable | Myeloma: Remission | Myeloma: Other | MGUS | Other Plasma Cell Dyscrasias |
| Mean | 25.568 | 129.544 | 41.983 | 40.507 | 65.120 | 42.067 | 51.650 |
| | | | | | | | |
| 1-50 Trial 2 | Normal | Myeloma: Progressive | Myeloma: Stable | Myeloma: Remission | Myeloma: Other | MGUS | Other Plasma Cell Dyscrasias |
| Mean | 17.160 | 119.220 | 34.567 | 34.264 | 54.780 | 26.333 | 51.650 |
| P-Values (One Tailed T-Test, 95% Significance)<br>~1-500 Single<br>Normal vs Progressive: p=.0010*<br>Progressive vs Remission: p=.0146*<br>~1-500 Triplicate<br>Normal vs Progressive: p=.0004*<br>Progressive vs Remission: p=.0091*<br>~1-50 Trial 1<br>Normal vs Progressive: p=.0171*<br>Progressive vs Remission: p=.0777<br>~1-50 Trial 2<br>Normal vs Progressive: p=.0184*<br>Progressive vs Remission: p=.0876<br>* shows significance | | | | | | | |

Sequence Summary **(Table C)**

| Description | Amino acid sequence | Polynucleotide sequence |
|---|---|---|
| CA8 CDRH1 | SEQ.I.D.NO:1 | n/a |

(continued)

| Description | Amino acid sequence | Polynucleotide sequence |
|---|---|---|
| CA8 CDRH2 | SEQ.I.D.NO:2 | n/a |
| CA8 CDRH3 | SEQ.I.D.NO:3 | n/a |
| CA8 CDRL1 | SEQ.I.D.NO:4 | n/a |
| CA8 CDRL2 | SEQ.I.D.NO:S | n/a |
| CA8 CDRL3 | SEQ.I.D.NO:6 | n/a |
| CA8 $V_H$ domain (murine) | SEQ.I.D.NO:7 | SEQ.ID.NO:8 |
| CA8 $V_L$ domain (murine) | SEQ.I.D.NO:9 | SEQ.I.D.NO:10 |
| CA8 Humanised $V_H$ J0 | SEQ.I.D.NO:11 | SEQ.ID.NO:12 |
| CA8 Humanised $V_H$ J1 | SEQ.I.D.NO:13 | SEQ.I.D.NO:14 |
| CA8 Humanised $V_H$ J2 | SEQ.I.D.NO:15 | SEQ.I.D.NO:16 |
| CA8 Humanised $V_H$ J3 | SEQ.I.D.NO:17 | SEQ.I.D.NO:18 |
| CA8 Humanised $V_H$ J4 | SEQ.I.D.NO:19 | SEQ.I.D.NO:20 |
| CA8 Humanised $V_H$ J5 | SEQ.LD.NO:21 | SEQ.I.D.NO:22 |
| CA8 Humanised $V_H$ J6 | SEQ.I.D.NO:23 | SEQ.I.D.NO:24 |
| CA8 Humanised $V_H$ J7 | SEQ.I.D.NO:25 | SEQ.ID.NO:26 |
| CA8 Humanised $V_H$ J8 | SEQ.I.D.NO:27 | SEQ.I.D.NO:28 |
| CA8 Humanised $V_H$ J9 | SEQ.I.D.NO:29 | SEQ.I.D.NO:30 |
| CA8 Humanised $V_L$ M0 | SEQ.I.D. NO:31 | SEQ.I.D.NO:32 |
| CA8 Humanised $V_L$ M1 | SEQ.I.D. NO:33 | SEQ.I.D.NO:34 |
| CA8 Humanised $V_L$ M2 | SEQ.I.D. NO:35 | SEQ.ID.NO:36 |
| Human BCMA<br>CD33-hBCMA ECD (1-53) TEV-Fc | SEQ.I.D.NO:37 | SEQ.I.D.NO:38 |
| Human BCMA<br>CD33-hBCMA ECD (4-53) TEV-Fc | SEQ.I.D.NO:39 | SEQ.I.D.NO:40 |
| Cyno BCMA<br>CD33 cyno BCMA ECD (4-52) TEV-Fc | SEQ.I.D.NO:41 | SEQ.I.D.NO:42 |
| CA8 J0 Humanised heavy chain | SEQ.I.D.NO:43 | SEQ.I.D.NO:44 |
| CA8 J1 Humanised heavy chain | SEQ.I.D.NO:45 | SEQ.I.D.N0:46 |
| CA8 J2 Humanised heavy chain | SEQ.I.D.NO:47 | SEQ.I.D.NO:48 |
| CA8 J3 Humanised heavy chain | SEQ.I.D.NO:49 | SEQ.I.D.NO:50 |
| CA8 J4 Humanised heavy chain | SEQ.I.D.NO:51 | SEQ.I.D.NO:52 |
| CA8 J5 Humanised heavy chain | SEQ.I.D.NO:53 | SEQ.ID.NO:S4 |
| CA8 J6 Humanised heavy chain | SEQ.I.D.NO:55 | SEQ.I.D.NO:56 |
| CA8 J7 Humanised heavy chain | SEQ.I.D.NO:57 | SEQ.ID.NO:S8 |
| CA8 J8 Humanised heavy chain | SEQ.I.D.NO:59 | SEQ.ID.NO:60 |
| CA8 J9 Humanised heavy chain | SEQ.I.D.NO:61 | SEQ.I.D.NO:62 |
| CA8 M0 Humanised light chain | SEQ.I.D.NO:63 | SEQ.I.D.NO:64 |
| CA8 M1 Humanised light chain | SEQ.I.D.NO:65 | SEQ.ID.NO:66 |

(continued)

| Description | Amino acid sequence | Polynucleotide sequence |
|---|---|---|
| CA8 M2 Humanised light chain | SEQ.I.D.NO:67 | SEQ.ID.NO:68 |
| S307118G03 $V_H$ domain (murine) | SEQ.ID.NO:69 | SEQ.ID.NO:70 |
| S307118G03 $V_L$ domain (murine) | SEQ.ID.NO:71 | SEQ.I.D.NO:72 |
| S307118G03 heavy chain (chimeric) | SEQ.I.D.NO:73 | SEQ.ID.NO:74 |
| S307118G03 light chain(chimeric) | SEQ.I.D.NO:75 | SEQ.I.D.NO:76 |
| S307118G03 Humanised $V_H$ H0 | SEQ.ID.NO:77 | SEQ.ID.NO:78 |
| S307118G03 Humanised $V_H$ H1 | SEQ.ID.NO:79 | SEQ.I.D.NO:80 |
| S307118G03 humanised $V_H$ H2 | SEQ.ID.NO:81 | SEQ.I.D.NO:82 |
| S307118G03 humanised $V_H$ H3 | SEQ.ID.NO:83 | SEQ.I.D.NO:84 |
| S307118G03 humanised $V_H$ H4 | SEQ.ID.NO:8S | SEQ.I.D.NO:86 |
| S307118G03 humanised $V_H$ H5 | SEQ.ID.NO:87 | SEQ.I.D.NO:88 |
| S307118G03 humanised $V_L$ L0 | SEQ.I.D.NO:89 | SEQ.I.D.NO:90 |
| S307118G03 humanised $V_L$ L1 | SEQ.I.D.NO:91 | SEQ.I.D.NO:92 |
| S307118G03 CDRH1 | SEQ.ID.NO:93 | |
| S307118G03 CDRH2 | SEQ.ID.NO:94 | |
| S307118G03 CDRH3 | SEQ.I.D.NO:95 | |
| S307118G03 CDRL1 | SEQ.ID.NO:96 | |
| S307118G03 CDRL2 | SEQ.ID.NO:97 | |
| S307118G03 CDRL3 | SEQ.ID.NO:98 | |
| S307118G03 humanised H5 CDRH3 | SEQ.ID.NO:99 | |
| S307118G03 H0 Humanised heavy chain | SEQ.I.D.NO:100 | SEQ.I.D.NO:101 |
| S307118G03 H1 humanised heavy chain | SEQ.I.D.NO:102 | SEQ.ID.NO:103 |
| S307118G03 H2 humanised heavy chain | SEQ.I.D.NO:104 | SEQ.I.D.NO:105 |
| S307118G03 H3 humanised heavy chain | SEQ.I.D.NO:106 | SEQ.I.D.NO:107 |
| S307118G03 H4 humanised heavy chain | SEQ.I.D.NO:108 | SEQ.I.D.NO:109 |
| S307118G03 H5 humanised heavy chain | SEQ.I.D.NO:110 | SEQ.ID.NO:111 |
| S307118G03 L0 humanised light chain | SEQ.I.D.NO:112 | SEQ.ID.NO:113 |
| S307118G03 L1 humanised light chain | SEQ.I.D.NO:114 | SEQ.ID.NO:115 |
| S332121F02 murine variable heavy chain | SEQ.I.D.NO:116 | SEQ.ID.NO:117 |
| S332121F02 chimeric variable heavy chain | SEQ.I.D.NO:118 | SEQ.ID.NO:119 |
| S332121F02 murine variable light chain | SEQ.I.D.NO:120 | SEQ.ID.NO:121 |
| S332121F02 chimeric variable light chain | SEQ.I.D.NO:122 | SEQ.ID.NO:123 |
| S322110D07 murine variable heavy chain | SEQ.I.D.NO:124 | SEQ.ID.NO:12S |
| S322110D07 chimeric heavy chain | SEQ.I.D.NO:126 | SEQ.I.D.NO:127 |
| S322110D07 murine variable light chain | SEQ.I.D.NO:128 | SEQ.I.D.NO:129 |
| S322110D07 chimeric light chain | SEQ.I.D.NO:130 | SEQ.ID.NO:131 |
| S332126E04 murine variable heavy chain | SEQ.I.D.NO:132 | SEQ.ID.NO:133 |
| S332126E04 Chimeric heavy chain | SEQ.I.D.NO:134 | SEQ.I.D.NO:135 |

(continued)

| Description | Amino acid sequence | Polynucleotide sequence |
|---|---|---|
| S332126E04 murine variable light chain | SEQ.I.D.NO:136 | SEQ.ID.NO:137 |
| S332126E04 Chimeric light chain | SEQ.I.D.NO:138 | SEQ.ID.NO:139 |
| S336105A07 murine variable heavy chain | SEQ.I.D.NO:140 | SEQ.ID.NO:141 |
| S336105A07 Chimeric heavy chain | SEQ.I.D.NO:142 | SEQ.ID.NO:143 |
| S336105A07 murine variable light chain | SEQ.I.D.NO:144 | SEQ.I.D.NO:145 |
| S336105A07 chimeric light chain | SEQ.I.D.NO:146 | SEQ.ID.NO:147 |
| S335115G01 murine variable heavy chain | SEQ.I.D.NO:148 | SEQ.I.D.NO:149 |
| S335115G01 Chimeric heavy chain | SEQ.I.D.NO:150 | SEQ.I.D.NO:151 |
| S335115G01 murine variable light chain | SEQ.I.D.NO:152 | SEQ.I.D.NO:153 |
| S335115G01 Chimeric light chain | SEQ.I.D.NO:154 | SEQ.I.D.NO:155 |
| S335122F05 murine variable heavy chain | SEQ.I.D.NO:156 | SEQ.I.D.NO:158 |
| S335122F05 Chimeric heavy chain | SEQ.I.D.NO:158 | SEQ.I.D.NO:159 |
| S335122F05 murine variable light chain | SEQ.I.D.NO:160 | SEQ.ID.NO:161 |
| S335122F05 Chimeric light chain | SEQ.I.D.NO:162 | SEQ.ID.NO:163 |
| S332121F02 CDRH1 | SEQ.I.D.NO: 164 | |
| S332121F02 CDRH2 | SEQ.I.D.NO: 165 | |
| S332121F02 CDRH3 | SEQ.I.D.NO: 166 | |
| S332121F02 CDRL1 | SEQ.I.D.NO: 167 | |
| S332121F02 CDRL2 | SEQ.I.D.NO: 168 | |
| S332121F02 CDRL3 | SEQ.I.D.NO: 169 | |
| S322110D07 CDRH1 | SEQ.I.D.NO: 170 | |
| S322110D07 CDRH2 | SEQ.I.D.NO: 171 | |
| S322110D07 CDRH3 | SEQ.I.D.NO: 172 | |
| S322110D07CDRL1 | SEQ.I.D.NO: 173 | |
| S322110D07 CDRL2 | SEQ.I.D.NO: 174 | |
| S322110D07 CDRL3 | SEQ.I.D.NO: 175 | |
| S332126E04CDRH1 | SEQ.I.D.NO: 176 | |
| S332126E04 CDRH2 | SEQ.I.D.NO: 177 | |
| S332126E04 CDRH3 | SEQ.I.D.NO: 178 | |
| S332126E04 CDRL1 | SEQ.I.D.NO: 179 | |
| S332126E04 CDRL2 | SEQ.I.D.NO: 180 | |
| S332126E04 CDRL3 | SEQ.I.D.NO: 181 | |
| S336105A07 CDRH1 | SEQ.I.D.NO: 182 | |
| S336105A07 CDRH2 | SEQ.I.D.NO: 183 | |
| S336105A07 CDRH3 | SEQ.I.D.NO: 184 | |
| S336105A07 CDRL1 | SEQ.I.D.NO: 185 | |
| S336105A07 CDRL2 | SEQ.I.D.NO: 186 | |
| S336105A07 CDRL3 | SEQ.I.D.NO: 187 | |

(continued)

| Description | Amino acid sequence | Polynucleotide sequence |
|---|---|---|
| S335115G01 CDRH1 | SEQ.I.D.NO: 188 | |
| S335115G01 CDRH2 | SEQ.I.D.NO: 189 | |
| S335115G01 CDRH3 | SEQ.I.D.NO: 190 | |
| S335115G01 CDRL1 | SEQ.I.D.NO: 191 | |
| S335115G01 CDRL2 | SEQ.I.D.NO: 192 | |
| S335115G01 CDRL3 | SEQ.I.D.NO: 193 | |
| S335122F05 CDRH1 | SEQ.I.D.NO: 194 | |
| S335122F05 CDRH2 | SEQ.I.D.NO: 195 | |
| S335122F05 CDRH3 | SEQ.I.D.NO: 196 | |
| S335122F05 CDRL1 | SEQ.I.D.NO: 197 | |
| S335122F05 CDRL2 | SEQ.I.D.NO: 198 | |
| S335122F05 CDRL3 | SEQ.I.D.NO: 199 | |

SEQUENCE LISTING

SEQ ID 1 - CA8 CDRH1
NYWMH
SEQ ID 2 - CA8 CDRH2
ATYRGHSDTYYNQKFKG
SEQ ID 3 - CA8 CDRH3
GAIYNGYDVLDN
SEQ ID 4 - CA8 CDRL1
SASQDISNYLN
SEQ ID 5 - CA8 CDRL2
YTSNLHS
SEQ ID 6 - CA8 CDRL3
QQYRKLPWT
SEQ ID 7 - CA8 $V_H$ domain (murine)

EVQLQQSGAVLARPGASVKMSCKGSGYTFTNYWMHWVKQRPGQGLEWIGATYRGHSDTYYNQKF
KGKAKLTAVTSTSTAYMELSSLTNEDSAVYYCTRGAIYNGYDVLDNWGQGTLVTVSS

SEQ ID 8 - CA8 $V_H$ domain (murine) (Polynucleotide)

GAGGTGCAGCTGCAGCAGAGCGGCGCCGTGCTGGCCAGGCCCGGAGCTAGCGTGAAGATGAG
CTGCAAGGGCAGCGGCTACACCTTCACCAACTACTGGATGCACTGGGTGAAACAGAGGCCCGG
CCAGGGACTGGAGTGGATCGGCGCCACCTACAGGGGCCACAGCGACACCTACTACAACCAGAA
GTTCAAGGGCAAGGCCAAGCTGACCGCCGTGACCTCAACCAGCACCGCCTACATGGAACTGAG
CAGCCTGACCAACGAGGACAGCGCCGTCTATTACTGCACCAGGGGCGCCATCTACAACGGCTA
CGACGTGCTGGACAATTGGGGCCAGGGAACACTAGTGACCGTGTCCAGC

SEQ ID 9 - CA8 $V_L$ domain (murine)

DIQLTQTTSSLSASLGDRVTISCSASQDISNYLNWYQQKPDGTVELVIYYTSNLHSGVPSRFSGSGSG TDYSLTIGYLEPEDVATYYCQQYRKLPWTFGGGSKLEIKR

SEQ ID 10 - CA8 $V_L$ domain (murine) (Polynucleotide)

GATATCCAGCTGACCCAGACCACAAGCAGCCTGAGCGCCTCCCTGGGCGACAGGGTGACCATT AGCTGCAGCGCCAGCCAGGACATCAGCAACTACCTGAACTGGTACCAGCAGAAGCCCGACGGC ACCGTGGAGCTCGTGATCTACTACACCTCCAACCTGCACAGCGGCGTGCCCAGCAGGTTCTCTG

GCAGCGGCAGCGGCACCGACTACAGCCTGACCATCGGCTATCTGGAGCCCGAGGACGTCGCCA CCTACTACTGCCAGCAGTACAGGAAGCTGCCCTGGACCTTCGGCGGAGGCTCTAAGCTGGAGA TTAAGCGT

SEQ ID 11 - CA8 Humanised $V_H$ J0

QVQLVQSGAEVKKPGSSVKVSCKASGGTFSNYWMHWVRQAPGQGLEWMGATYRGHSDTYYNQK FKGRVTITADKSTSTAYMELSSLRSEDTAVYYCARGAIYNGYDVLDNWGQGTLVTVSS

SEQ ID 12 - CA8 Humanised $V_H$ J0 (Polynucleotide)

CAGGTGCAGCTGGTCCAGAGCGGCGCCGAAGTGAAGAAGCCCGGCAGCTCCGTGAAAGTGAG CTGCAAGGCCAGCGGCGGCACCTTCAGCAACTACTGGATGCACTGGGTGAGGCAGGCCCCCG GACAGGGCCTGGAGTGGATGGGCGCCACCTACAGGGGCCACAGCGACACCTACTACAACCAGA AGTTCAAGGGCCGGGTGACCATCACCGCCGACAAGAGCACCAGCACCGCCTACATGGAACTGA GCAGCCTCAGGAGCGAGGACACCGCTGTGTATTACTGCGCCAGGGGCGCCATCTACAACGGCT ACGACGTGCTGGACAACTGGGGCCAGGGCACACTAGTGACCGTGTCCAGC

SEQ ID 13 - CA8 Humanised $V_H$ J1

QVQLVQSGAEVKKPGSSVKVSCKASGYTFTNYWMHWVRQAPGQGLEWMGATYRGHSDTYYNQK FKGRVTITADKSTSTAYMELSSLRSEDTAVYYCARGAIYNGYDVLDNWGQGTLVTVSS

SEQ ID 14 - CA8 Humanised $V_H$ J1 (Polynucleotide)

CAGGTGCAGCTGGTCCAGAGCGGCGCCGAAGTGAAGAAGCCCGGCAGCTCCGTGAAAGTGAG CTGCAAGGCCAGCGGCTACACCTTCACCAACTACTGGATGCACTGGGTGAGGCAGGCCCCCGG ACAGGGCCTGGAGTGGATGGGCGCCACCTACAGGGGCCACAGCGACACCTACTACAACCAGAA GTTCAAGGGCCGGGTGACCATCACCGCCGACAAGAGCACCAGCACCGCCTACATGGAACTGAG CAGCCTCAGGAGCGAGGACACCGCTGTGTATTACTGCGCCAGGGGCGCCATCTACAACGGCTA CGACGTGCTGGACAACTGGGGCCAGGGCACACTAGTGACCGTGTCCAGC

SEQ ID 15 - CA8 Humanised $V_H$ J2

QVQLVQSGAEVKKPGSSVKVSCKASGYTFTNYWMHWVRQAPGQGLEWMGATYRGHSDTYYNQK FKGRVTITADKSTSTAYMELSSLRSEDTAVYYCTRGAIYNGYDVLDNWGQGTLVTVSS

SEQ ID 16 - CA8 Humanised $V_H$ J2 (Polynucleotide)

CAGGTGCAGCTGGTCCAGAGCGGCGCCGAAGTGAAGAAGCCCGGCAGCTCCGTGAAAGTGAG
CTGCAAGGCCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACAAGAGCACCAGCACCGCCTACATGGAACTGAG
CAGCCTCAGGAGCGAGGACACCGCTGTGTATTACTGCACCAGGGGCGCCATCTACAACGGCTA
CGACGTGCTGGACAACTGGGGCCAGGGCACACTAGTGACCGTGTCCAGC

SEQ ID 17 - CA8 Humanised $V_H$ J3

QVQLVQSGAEVKKPGSSVKVSCKGSGYTFTNYWMHWVRQAPGQGLEWMGATYRGHSDTYYNQK
FKGRVTITADTSTSTAYMELSSLRSEDTAVYYCTRGAIYNGYDVLDNWGQGTLVTVSS

SEQ ID 18 - CA8 Humanised $V_H$ J3 (Polynucleotide)

CAGGTGCAGCTGGTCCAGAGCGGCGCCGAAGTGAAGAAGCCCGGCAGCTCCGTGAAAGTGAG
CTGCAAGGGCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACACGAGCACCAGCACCGCCTACATGGAACTGAG
CAGCCTCAGGAGCGAGGACACCGCTGTGTATTACTGCACCAGGGGCGCCATCTACAACGGCTA
CGACGTGCTGGACAACTGGGGCCAGGGCACACTAGTGACCGTGTCCAGC

SEQ ID 19 - CA8 Humanised $V_H$ J4

QVQLVQSGAEVKKPGSSVKVSCKGSGYTFTNYWMHWVRQAPGQGLEWIGATYRGHSDTYYNQKF
KGRATLTADTSTSTAYMELSSLRSEDTAVYYCTRGAIYNGYDVLDNWGQGTLVTVSS

SEQ ID 20 - CA8 Humanised $V_H$ J4 (Polynucleotide)

CAGGTGCAGCTGGTCCAGAGCGGCGCCGAAGTGAAGAAGCCCGGCAGCTCCGTGAAAGTGAG
CTGCAAGGGCAGCGGCTACACCTTCACCAACTACTGGATGCACTGGGTGAGGCAGGCCCCCGG
ACAGGGCCTGGAGTGGATCGGCGCCACCTACAGGGGCCACAGCGACACCTACTACAACCAGAA
GTTCAAGGGCCGGGCGACCCTCACCGCCGACACGAGCACCAGCACCGCCTACATGGAACTGAG
CAGCCTCAGGAGCGAGGACACCGCTGTGTATTACTGCACCAGGGGCGCCATCTACAACGGCTA
CGACGTGCTGGACAACTGGGGCCAGGGCACACTAGTGACCGTGTCCAGC

SEQ ID 21 - CA8 Humanised $V_H$ J5

QVQLVQSGAEVKKPGSSVKVSCKGSGYTFTNYWMHWVRQAPGQGLEWMGATYRGHSDTYYNQK
FKGRVTITADTSTSTAYMELSSLRSEDTAVYYCTRGAIYDGYDVLDNWGQGTLVTVSS

SEQ ID 22 - CA8 Humanised $V_H$ J5 (Polynucleotide)

CAGGTGCAGCTGGTCCAGAGCGGCGCCGAAGTGAAGAAGCCCGGCAGCTCCGTGAAAGTGAG
CTGCAAGGGCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACACGAGCACCAGCACCGCCTACATGGAACTGAG
CAGCCTCAGGAGCGAGGACACCGCTGTGTATTACTGCACCAGGGGCGCCATCTACGACGGCTA
CGACGTGCTGGACAACTGGGGCCAGGGCACACTAGTGACCGTGTCCAGC

SEQ ID 23 - CA8 Humanised $V_H$ J6

QVQLVQSGAEVKKPGSSVKVSCKASGGTFSNYWMHWVRQAPGQGLEWMGATYRGHSDTYYNQK
FKGRVTITADKSTSTAYMELSSLRSEDTAVYYCARGAIYDGYDVLDNWGQGTLVTVSS

SEQ ID 24 - CA8 Humanised $V_H$ J6 (Polynucleotide)

CAGGTGCAGCTGGTCCAGAGCGGCGCCGAAGTGAAGAAGCCCGGCAGCTCCGTGAAAGTGAG
CTGCAAGGCCAGCGGCGGCACCTTCAGCAACTACTGGATGCACTGGGTGAGGCAGGCCCCCG
GACAGGGCCTGGAGTGGATGGGCGCCACCTACAGGGGCCACAGCGACACCTACTACAACCAGA
AGTTCAAGGGCCGGGTGACCATCACCGCCGACAAGAGCACCAGCACCGCCTACATGGAACTGA
GCAGCCTCAGGAGCGAGGACACCGCTGTGTATTACTGCGCCAGGGGCGCCATCTACGACGGCT
ACGACGTGCTGGACAACTGGGGCCAGGGCACACTAGTGACCGTGTCCAGC

SEQ ID 25 - CA8 Humanised $V_H$ J7

QVQLVQSGAEVKKPGSSVKVSCKASGYTFTNYWMHWVRQAPGQGLEWMGATYRGHSDTYYNQK
FKGRVTITADKSTSTAYMELSSLRSEDTAVYYCARGAIYDGYDVLDNWGQGTLVTVSS

SEQ ID 26 - CA8 Humanised $V_H$ J7 (Polynucleotide)

CAGGTGCAGCTGGTCCAGAGCGGCGCCGAAGTGAAGAAGCCCGGCAGCTCCGTGAAAGTGAG
CTGCAAGGCCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACAAGAGCACCAGCACCGCCTACATGGAACTGAG
CAGCCTCAGGAGCGAGGACACCGCTGTGTATTACTGCGCCAGGGGCGCCATCTACGACGGCTA
CGACGTGCTGGACAACTGGGGCCAGGGCACACTAGTGACCGTGTCCAGC

SEQ ID 27 - CA8 Humanised $V_H$ J8

QVQLVQSGAEVKKPGSSVKVSCKASGYTFTNYWMHWVRQAPGQGLEWMGATYRGHSDTYYNQK
FKGRVTITADKSTSTAYMELSSLRSEDTAVYYCTRGAIYDGYDVLDNWGQGTLVTVSS

SEQ ID 28 - CA8 Humanised $V_H$ J8 (Polynucleotide)

CAGGTGCAGCTGGTCCAGAGCGGCGCCGAAGTGAAGAAGCCCGGCAGCTCCGTGAAAGTGAG
CTGCAAGGCCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACAAGAGCACCAGCACCGCCTACATGGAACTGAG
CAGCCTCAGGAGCGAGGACACCGCTGTGTATTACTGCACCAGGGGCGCCATCTACGACGGCTA
CGACGTGCTGGACAACTGGGGCCAGGGCACACTAGTGACCGTGTCCAGC

SEQ ID 29 - CA8 Humanised $V_H$ J9

QVQLVQSGAEVKKPGSSVKVSCKGSGYTFTNYWMHWVRQAPGQGLEWIGATYRGHSDTYYNQKF
KGRATLTADTSTSTAYMELSSLRSEDTAVYYCTRGAIYDGYDVLDNWGQGTLVTVSS

SEQ ID 30 - CA8 Humanised $V_H$ J9 Polnucleotide

CAGGTGCAGCTGGTCCAGAGCGGCGCCGAAGTGAAGAAGCCCGGCAGCTCCGTGAAAGTGAG
CTGCAAGGGCAGCGGCTACACCTTCACCAACTACTGGATGCACTGGGTGAGGCAGGCCCCCGG

ACAGGGCCTGGAGTGGATCGGCGCCACCTACAGGGGCCACAGCGACACCTACTACAACCAGAA
GTTCAAGGGCCGGGCGACCCTCACCGCCGACACGAGCACCAGCACCGCCTACATGGAACTGAG
CAGCCTCAGGAGCGAGGACACCGCTGTGTATTACTGCACCAGGGGCGCCATCTACGACGGCTA
CGACGTGCTGGACAACTGGGGCCAGGGCACACTAGTGACCGTGTCCAGC

SEQ ID 31 - CA8 Humanised $V_L$ M0

DIQMTQSPSSLSASVGDRVTITCSASQDISNYLNWYQQKPGKAPKLLIYYTSNLHSGVPSRFSGSGS
GTDFTLTISSLQPEDFATYYCQQYRKLPWTFGQGTKLEIKR

SEQ ID 32 - CA8 Humanised $V_L$ M0 (Polynucleotide)

GACATCCAGATGACCCAGAGCCCTAGCTCACTGAGCGCCAGCGTGGGCGACAGGGTGACCATT
ACCTGCTCCGCCAGCCAGGACATCAGCAACTACCTGAACTGGTACCAGCAGAAGCCCGGCAAG
GCCCCCAAGCTGCTGATCTACTACACCTCCAACCTGCACTCCGGCGTGCCCAGCAGGTTCAGCG
GAAGCGGCAGCGGCACCGATTTCACCCTGACCATCTCCAGCCTGCAGCCCGAGGACTTCGCCA
CCTACTACTGCCAGCAGTACAGGAAGCTCCCCTGGACTTTCGGCCAGGGCACCAAACTGGAGAT
CAAGCGT

SEQ ID 33 - CA8 Humanised $V_L$ M1

DIQMTQSPSSLSASVGDRVTITCSASQDISNYLNWYQQKPGKAPKLLIYYTSNLHSGVPSRFSGSGS
GTDYTLTISSLQPEDFATYYCQQYRKLPWTFGQGTKLEIKR

SEQ ID 34 - CA8 Humanised $V_L$ M1 (Polynucleotide)

GACATCCAGATGACCCAGAGCCCTAGCTCACTGAGCGCCAGCGTGGGCGACAGGGTGACCATT
ACCTGCTCCGCCAGCCAGGACATCAGCAACTACCTGAACTGGTACCAGCAGAAGCCCGGCAAG
GCCCCCAAGCTGCTGATCTACTACACCTCCAACCTGCACTCCGGCGTGCCCAGCAGGTTCAGCG
GAAGCGGCAGCGGCACCGATTACACCCTGACCATCTCCAGCCTGCAGCCCGAGGACTTCGCCA
CCTACTACTGCCAGCAGTACAGGAAGCTCCCCTGGACTTTCGGCCAGGGCACCAAACTGGAGAT
CAAGCGT

SEQ ID 35 - CA8 Humanised $V_L$ M2

DIQLTQSPSSLSASVGDRVTITCSASQDISNYLNWYQQKPGKAPELVIYYTSNLHSGVPSRFSGSGSG
TDYTLTISSLQPEDFATYYCQQYRKLPWTFGQGTKLEIKR

SEQ ID 36 - CA8 Humanised $V_L$ M2 (Polynucleotide)

GACATCCAGCTGACCCAGAGCCCTAGCTCACTGAGCGCCAGCGTGGGCGACAGGGTGACCATT
ACCTGCTCCGCCAGCCAGGACATCAGCAACTACCTGAACTGGTACCAGCAGAAGCCCGGCAAG
GCCCCCGAGCTGGTGATCTACTACACCTCCAACCTGCACTCCGGCGTGCCCAGCAGGTTCAGC
GGAAGCGGCAGCGGCACCGATTACACCCTGACCATCTCCAGCCTGCAGCCCGAGGACTTCGCC
ACCTACTACTGCCAGCAGTACAGGAAGCTCCCCTGGACTTTCGGCCAGGGCACCAAACTGGAGA
TCAAGCGT

SEQ ID 37 - Human BCMA CD33-hBCMA ECD (1-53) TEV-Fc

MPLLLLLPLLWAGALAMLQMAGQCSQNEYFDSLLHACIPCQLRCSSNTPPLTCQRYCNASVTNSVKG
TNSGENLYFQGDPKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK

SEQ ID 38 - Human BCMA CD33-hBCMA ECD (1-53) TEV-Fc (Polynucleotide)

ATGCCGCTGCTGCTACTGCTGCCCCTGCTGTGGGCAGGGGCGCTAGCTATGCTGCAGATGGCC
GGCCAGTGCAGCCAGAACGAGTACTTCGACAGCCTGCTGCACGCCTGCATCCCCTGCCAGCTG
AGATGCAGCAGCAACACACCTCCTCTGACCTGCCAGAGATACTGCAACGCCAGCGTGACCAACA
GCGTGAAGGGCACCAACTCCGGAGAGAACCTGTACTTCCAAGGGGATCCCAAATCTTGTGACAA
AACTCACACATGCCCACCGTGCCCAGCACCTGAACTCCTGGGGGGACCGTCAGTCTTCCTCTTC
CCCCCAAAACCCAAGGACACCCTCATGATCTCCCGGACCCCTGAGGTCACATGCGTGGTGGTG
GACGTGAGCCACGAAGACCCTGAGGTCAAGTTCAACTGGTACGTGGACGGCGTGGAGGTGCAT
AATGCCAAGACAAAGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTCTCCAACAAAGCCC
TCCCAGCCCCCATCGAGAAAACCATCTCCAAAGCCAAAGGGCAGCCCCGAGAGCCACAGGTGT
ACACCCTGCCCCCATCCCGGGATGAGCTGACCAAGAACCAGGTCAGCCTGACCTGCCTGGTCA
AAGGCTTCTATCCCAGCGACATCGCCGTGGAGTGGGAGAGCAATGGGCAGCCGGAGAACAACT
ACAAGACCACGCCTCCCGTGCTGGACTCCGACGGCTCCTTCTTCCTCTACAGCAAGCTCACCGT
GGACAAGAGCAGGTGGCAGCAGGGGAACGTCTTCTCATGCTCCGTGATGCATGAGGCTCTGCA
CAACCACTACACGCAGAAGAGCCTCTCCCTGTCTCCGGGTAAA

SEQ ID 39- Human BCMA CD33-hBCMA ECD (4-53) TEV-Fc

MPLLLLLPLLWAGALAMAGQCSQNEYFDSLLHACIPCQLRCSSNTPPLTCQRYCNASVTNSVKGTNS
GENLYFQGDPKSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID 40 - Human BCMA CD33-hBCMA ECD (4-53) TEV-Fc (Polynucleotide)

ATGCCGCTGCTGCTACTGCTGCCCCTGCTGTGGGCAGGGGCGCTAGCTATGGCCGGCCAGTGC
AGCCAGAACGAGTACTTCGACAGCCTGCTGCACGCCTGCATCCCCTGCCAGCTGAGATGCAGC
AGCAACACACCTCCTCTGACCTGCCAGAGATACTGCAACGCCAGCGTGACCAACAGCGTGAAGG
GCACCAACTCCGGAGAGAACCTGTACTTCCAAGGGGATCCCAAATCTTGTGACAAAACTCACAC
ATGCCCACCGTGCCCAGCACCTGAACTCCTGGGGGGACCGTCAGTCTTCCTCTTCCCCCCAAAA

CCCAAGGACACCCTCATGATCTCCCGGACCCCTGAGGTCACATGCGTGGTGGTGGACGTGAGC
CACGAAGACCCTGAGGTCAAGTTCAACTGGTACGTGGACGGCGTGGAGGTGCATAATGCCAAG
ACAAAGCCGCGGGAGGAGCAGTACAACAGCACGTACCGTGTGGTCAGCGTCCTCACCGTCCTG
CACCAGGACTGGCTGAATGGCAAGGAGTACAAGTGCAAGGTCTCCAACAAAGCCCTCCCAGCC
CCCATCGAGAAAACCATCTCCAAAGCCAAAGGGCAGCCCCGAGAGCCACAGGTGTACACCCTG
CCCCCATCCCGGGATGAGCTGACCAAGAACCAGGTCAGCCTGACCTGCCTGGTCAAAGGCTTCT
ATCCCAGCGACATCGCCGTGGAGTGGGAGAGCAATGGGCAGCCGGAGAACAACTACAAGACCA
CGCCTCCCGTGCTGGACTCCGACGGCTCCTTCTTCCTCTACAGCAAGCTCACCGTGGACAAGAG
CAGGTGGCAGCAGGGGAACGTCTTCTCATGCTCCGTGATGCATGAGGCTCTGCACAACCACTAC
ACGCAGAAGAGCCTCTCCCTGTCTCCGGGTAAA

SEQ ID 41- Cynomolgous BCMA CD33 cyno BCMA ECD (4-52) TEV-Fc

MPLLLLLPLLWAGALAMARQCSQNEYFDSLLHDCKPCQLRCSSTPPLTCQRYCNASMTNSVKGMNS
GENLYFQGDPKSCDKTHTCPPCPAPELLGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALPAPIEKTISKAKG
QPREPQVYTLPPSRDELTKNQVSLTCLVKGFYPSDIAVEWESNGQPENNYKTTPPVLDSDGSFFLYS
KLTVDKSRWQQGNVFSCSVMHEALHNHYTQKSLSLSPGK

SEQ ID 42 - Cynomolgous BCMA CD33 cyno BCMA ECD (4-52) TEV-Fc (Polynucleotide)

ATGCCGCTGCTGCTACTGCTGCCCCTGCTGTGGGCAGGGGCGCTAGCTATGGCCAGACAGTGC
AGCCAGAACGAGTACTTCGACAGCCTGCTGCACGACTGCAAGCCCTGCCAGCTGAGATGCAGC
AGCACACCTCCTCTGACCTGCCAGAGATACTGCAACGCCAGCATGACCAACAGCGTGAAGGGCA
TGAACTCCGGAGAGAACCTGTACTTCCAAGGGGATCCCAAATCTTGTGACAAAACTCACACATGC
CCACCGTGCCCAGCACCTGAACTCCTGGGGGGACCGTCAGTCTTCCTCTTCCCCCCAAAACCCA
AGGACACCCTCATGATCTCCCGGACCCCTGAGGTCACATGCGTGGTGGTGGACGTGAGCCACG
AAGACCCTGAGGTCAAGTTCAACTGGTACGTGGACGGCGTGGAGGTGCATAATGCCAAGACAAA
GCCGCGGGAGGAGCAGTACAACAGCACGTACCGTGTGGTCAGCGTCCTCACCGTCCTGCACCA
GGACTGGCTGAATGGCAAGGAGTACAAGTGCAAGGTCTCCAACAAAGCCCTCCCAGCCCCCATC
GAGAAAACCATCTCCAAAGCCAAAGGGCAGCCCCGAGAGCCACAGGTGTACACCCTGCCCCCA
TCCCGGGATGAGCTGACCAAGAACCAGGTCAGCCTGACCTGCCTGGTCAAAGGCTTCTATCCCA
GCGACATCGCCGTGGAGTGGGAGAGCAATGGGCAGCCGGAGAACAACTACAAGACCACGCCTC
CCGTGCTGGACTCCGACGGCTCCTTCTTCCTCTACAGCAAGCTCACCGTGGACAAGAGCAGGTG
GCAGCAGGGGAACGTCTTCTCATGCTCCGTGATGCATGAGGCTCTGCACAACCACTACACGCAG
AAGAGCCTCTCCCTGTCTCCGGGTAAA

SEQ ID 43- CA8 J0 Humanised heavy chain

QVQLVQSGAEVKKPGSSVKVSCKASGGTFSNYWMHWVRQAPGQGLEWMGATYRGHSDTYYNQK
FKGRVTITADKSTSTAYMELSSLRSEDTAVYYCARGAIYNGYDVLDNWGQGTLVTVSSASTKGPSVF
PLAPSSKSTSGGTAALGCLVKDYFPEPVTVSWNSGALTSGVHTFPAVLQSSGLYSLSSVVTVPSSSL

GTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID 44 - CA8 J0 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGCCAGCGGCGGCACCTTCAGCAACTACTGGATGCACTGGGTGAGGCAGGCCCCCG
GACAGGGCCTGGAGTGGATGGGCGCCACCTACAGGGGCCACAGCGACACCTACTACAACCAGA
AGTTCAAGGGCCGGGTGACCATCACCGCCGACAAGAGCACCAGCACCGCCTACATGGAACTGA
GCAGCCTCAGGAGCGAGGACACCGCTGTGTATTACTGCGCCAGGGGCGCCATCTACAACGGCT
ACGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG
GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC
AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 45- CA8 J1 Humanised heavy chain

QVQLVQSGAEVKKPGSSVKVSCKASGYTFTNYWMHWVRQAPGQGLEWMGATYRGHSDTYYNQK
FKGRVTITADKSTSTAYMELSSLRSEDTAVYYCARGAIYNGYDVLDNWGQGTLVTVSSASTKGPSVF
PLAPSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID 46 - CA8 J1 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGCCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACAAGAGCACCAGCACCGCCTACATGGAACTGAG
CAGCCTCAGGAGCGAGGACACCGCTGTGTATTACTGCGCCAGGGGCGCCATCTACAACGGCTA
CGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG
GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC
AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 47 - CA8 J2 Humanised heavy chain

QVQLVQSGAEVKKPGSSVKVSCKASGYTFTNYWMHWVRQAPGQGLEWMGATYRGHSDTYYNQK
FKGRVTITADKSTSTAYMELSSLRSEDTAVYYCTRGAIYNGYDVLDNWGQGTLVTVSSASTKGPSVF
PLAPSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID 48 - CA8 J2 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGCCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACAAGAGCACCAGCACCGCCTACATGGAACTGAG
CAGCCTCAGGAGCGAGGACACCGCTGTGTATTACTGCACCAGGGGCGCCATCTACAACGGCTA
CGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG

GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC
AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 49- CA8 J3 Humanised heavy chain

QVQLVQSGAEVKKPGSSVKVSCKGSGYTFTNYWMHWVRQAPGQGLEWMGATYRGHSDTYYNQK
FKGRVTITADTSTSTAYMELSSLRSEDTAVYYCTRGAIYNGYDVLDNWGQGTLVTVSSASTKGPSVF
PLAPSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID 50 - CA8 J3 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGGCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACACGAGCACCAGCACCGCCTACATGGAACTGAG
CAGCCTCAGGAGCGAGGACACCGCTGTGTATTACTGCACCAGGGGCGCCATCTACAACGGCTA
CGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG
GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC

AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 51 - CA8 J4 Humanised heavy chain

QVQLVQSGAEVKKPGSSVKVSCKGSGYTFTNYWMHWVRQAPGQGLEWIGATYRGHSDTYYNQKF
KGRATLTADTSTSTAYMELSSLRSEDTAVYYCTRGAIYNGYDVLDNWGQGTLVTVSSASTKGPSVFP
LAPSSKSTSGGTAALGCLVKDYFPEPVTVSWNSGALTSGVHTFPAVLQSSGLYSLSSVVTVPSSSLG
TQTYICNVNHKPSNTKVDKKVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNGQPENNYKTTP
PVLDSDGSFFLYSKLTVDKSRWQQGNVFSCSVMHEALHNHYTQKSLSLSPGK

SEQ ID 52 - CA8 J4 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGGCAGCGGCTACACCTTCACCAACTACTGGATGCACTGGGTGAGGCAGGCCCCCGG
ACAGGGCCTGGAGTGGATCGGCGCCACCTACAGGGGCCACAGCGACACCTACTACAACCAGAA
GTTCAAGGGCCGGGCGACCCTCACCGCCGACACGAGCACCAGCACCGCCTACATGGAACTGAG
CAGCCTCAGGAGCGAGGACACCGCTGTGTATTACTGCACCAGGGGCGCCATCTACAACGGCTA
CGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG
GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC
AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA

TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 53 - CA8 J5 Humanised heavy chain

QVQLVQSGAEVKKPGSSVKVSCKGSGYTFTNYWMHWVRQAPGQGLEWMGATYRGHSDTYYNQK
FKGRVTITADTSTSTAYMELSSLRSEDTAVYYCTRGAIYDGYDVLDNWGQGTLVTVSSASTKGPSVF
PLAPSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID 54 - CA8 J5 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGGCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACACGAGCACCAGCACCGCCTACATGGAACTGAG
CAGCCTCAGGAGCGAGGACACCGCTGTGTATTACTGCACCAGGGGCGCCATCTACGACGGCTA
CGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG
GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC
AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 55 - CA8 J6 Humanised heavy chain

QVQLVQSGAEVKKPGSSVKVSCKASGGTFSNYWMHWVRQAPGQGLEWMGATYRGHSDTYYNQK
FKGRVTITADKSTSTAYMELSSLRSEDTAVYYCARGAIYDGYDVLDNWGQGTLVTVSSASTKGPSVF
PLAPSSKSTSGGTAALGCLVKDYFPEPVTVSWNSGALTSGVHTFPAVLQSSGLYSLSSVVTVPSSSL

GTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID 56 - CA8 J6 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGCCAGCGGCGGCACCTTCAGCAACTACTGGATGCACTGGGTGAGGCAGGCCCCCG
GACAGGGCCTGGAGTGGATGGGCGCCACCTACAGGGGCCACAGCGACACCTACTACAACCAGA
AGTTCAAGGGCCGGGTGACCATCACCGCCGACAAGAGCACCAGCACCGCCTACATGGAACTGA
GCAGCCTCAGGAGCGAGGACACCGCTGTGTATTACTGCGCCAGGGGCGCCATCTACGACGGCT
ACGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG
GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC
AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 57 - CA8 J7 Humanised heavy chain

QVQLVQSGAEVKKPGSSVKVSCKASGYTFTNYWMHWVRQAPGQGLEWMGATYRGHSDTYYNQK
FKGRVTITADKSTSTAYMELSSLRSEDTAVYYCARGAIYDGYDVLDNWGQGTLVTVSSASTKGPSVF
PLAPSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID 58 - CA8 J7 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGCCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACAAGAGCACCAGCACCGCCTACATGGAACTGAG
CAGCCTCAGGAGCGAGGACACCGCTGTGTATTACTGCGCCAGGGGCGCCATCTACGACGGCTA
CGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG
GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC
AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 59 - CA8 J8 Humanised heavy chain

QVQLVQSGAEVKKPGSSVKVSCKASGYTFTNYWMHWVRQAPGQGLEWMGATYRGHSDTYYNQK
FKGRVTITADKSTSTAYMELSSLRSEDTAVYYCTRGAIYDGYDVLDNWGQGTLVTVSSASTKGPSVF
PLAPSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMHEALHNHYTQKSLSLSPGK

SEQ ID 60 - CA8 J8 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGCCAGCGGCTACACCTTCACCAACTACTGGATGCACTGGGTGAGGCAGGCCCCCGG
ACAGGGCCTGGAGTGGATGGGCGCCACCTACAGGGGCCACAGCGACACCTACTACAACCAGAA
GTTCAAGGGCCGGGTGACCATCACCGCCGACAAGAGCACCAGCACCGCCTACATGGAACTGAG
CAGCCTCAGGAGCGAGGACACCGCTGTGTATTACTGCACCAGGGGCGCCATCTACGACGGCTA
CGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG

GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC
AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 61 - CA8 J9 Humanised heavy chain

QVQLVQSGAEVKKPGSSVKVSCKGSGYTFTNYWMHWVRQAPGQGLEWIGATYRGHSDTYYNQKF
KGRATLTADTSTSTAYMELSSLRSEDTAVYYCTRGAIYDGYDVLDNWGQGTLVTVSSASTKGPSVFP
LAPSSKSTSGGTAALGCLVKDYFPEPVTVSWNSGALTSGVHTFPAVLQSSGLYSLSSVVTVPSSSLG
TQTYICNVNHKPSNTKVDKKVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNGQPENNYKTTP
PVLDSDGSFFLYSKLTVDKSRWQQGNVFSCSVMHEALHNHYTQKSLSLSPGK

SEQ ID 62 - CA8 J9 Humanised heavy chain (Polynucleotide)

CAGGTGCAGCTGGTCCAGAGCGGCGCCGAAGTGAAGAAGCCCGGCAGCTCCGTGAAAGTGAG
CTGCAAGGGCAGCGGCTACACCTTCACCAACTACTGGATGCACTGGGTGAGGCAGGCCCCCGG
ACAGGGCCTGGAGTGGATCGGCGCCACCTACAGGGGCCACAGCGACACCTACTACAACCAGAA
GTTCAAGGGCCGGGCGACCCTCACCGCCGACACGAGCACCAGCACCGCCTACATGGAACTGAG
CAGCCTCAGGAGCGAGGACACCGCTGTGTATTACTGCACCAGGGGCGCCATCTACGACGGCTA
CGACGTGCTGGACAACTGGGGCCAGGGCACACTAGTGACCGTGTCCAGCGCCAGCACCAAGG
GCCCCAGCGTGTTCCCCCTGGCCCCCAGCAGCAAGAGCACCAGCGGCGGCACAGCCGCCCTG
GGCTGCCTGGTGAAGGACTACTTCCCCGAACCGGTGACCGTGTCCTGGAACAGCGGAGCCCTG
ACCAGCGGCGTGCACACCTTCCCCGCCGTGCTGCAGAGCAGCGGCCTGTACAGCCTGAGCAGC
GTGGTGACCGTGCCCAGCAGCAGCCTGGGCACCCAGACCTACATCTGTAACGTGAACCACAAG
CCCAGCAACACCAAGGTGGACAAGAAGGTGGAGCCCAAGAGCTGTGACAAGACCCACACCTGC
CCCCCCTGCCCTGCCCCCGAGCTGCTGGGAGGCCCCAGCGTGTTCCTGTTCCCCCCCAAGCCT
AAGGACACCCTGATGATCAGCAGAACCCCCGAGGTGACCTGTGTGGTGGTGGATGTGAGCCAC
GAGGACCCTGAGGTGAAGTTCAACTGGTACGTGGACGGCGTGGAGGTGCACAATGCCAAGACC

AAGCCCAGGGAGGAGCAGTACAACAGCACCTACCGGGTGGTGTCCGTGCTGACCGTGCTGCAC
CAGGATTGGCTGAACGGCAAGGAGTACAAGTGTAAGGTGTCCAACAAGGCCCTGCCTGCCCCTA
TCGAGAAAACCATCAGCAAGGCCAAGGGCCAGCCCAGAGAGCCCCAGGTGTACACCCTGCCCC
CTAGCAGAGATGAGCTGACCAAGAACCAGGTGTCCCTGACCTGCCTGGTGAAGGGCTTCTACCC
CAGCGACATCGCCGTGGAGTGGGAGAGCAACGGCCAGCCCGAGAACAACTACAAGACCACCCC
CCCTGTGCTGGACAGCGATGGCAGCTTCTTCCTGTACAGCAAGCTGACCGTGGACAAGAGCAGA
TGGCAGCAGGGCAACGTGTTCAGCTGCTCCGTGATGCACGAGGCCCTGCACAATCACTACACC
CAGAAGAGCCTGAGCCTGTCCCCTGGCAAG

SEQ ID 63 - CA8 M0 Humanised light chain

DIQMTQSPSSLSASVGDRVTITCSASQDISNYLNWYQQKPGKAPKLLIYYTSNLHSGVPSRFSGSGS
GTDFTLTISSLQPEDFATYYCQQYRKLPWTFGQGTKLEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLS
SPVTKSFNRGEC

SEQ ID 64 - CA8 M0 Humanised light chain (Polynucleotide)

GACATCCAGATGACCCAGAGCCCTAGCTCACTGAGCGCCAGCGTGGGCGACAGGGTGACCATT
ACCTGCTCCGCCAGCCAGGACATCAGCAACTACCTGAACTGGTACCAGCAGAAGCCCGGCAAG
GCCCCCAAGCTGCTGATCTACTACACCTCCAACCTGCACTCCGGCGTGCCCAGCAGGTTCAGCG
GAAGCGGCAGCGGCACCGATTTCACCCTGACCATCTCCAGCCTGCAGCCCGAGGACTTCGCCA
CCTACTACTGCCAGCAGTACAGGAAGCTCCCCTGGACTTTCGGCCAGGGCACCAAACTGGAGAT
CAAGCGTACGGTGGCCGCCCCCAGCGTGTTCATCTTCCCCCCCAGCGATGAGCAGCTGAAGAG
CGGCACCGCCAGCGTGGTGTGTCTGCTGAACAACTTCTACCCCCGGGAGGCCAAGGTGCAGTG
GAAGGTGGACAATGCCCTGCAGAGCGGCAACAGCCAGGAGAGCGTGACCGAGCAGGACAGCA
AGGACTCCACCTACAGCCTGAGCAGCACCCTGACCCTGAGCAAGGCCGACTACGAGAAGCACA
AGGTGTACGCCTGTGAGGTGACCCACCAGGGCCTGTCCAGCCCCGTGACCAAGAGCTTCAACC
GGGGCGAGTGC

SEQ ID 65 - CA8 M1 Humanised light chain

DIQMTQSPSSLSASVGDRVTITCSASQDISNYLNWYQQKPGKAPKLLIYYTSNLHSGVPSRFSGSGS
GTDYTLTISSLQPEDFATYYCQQYRKLPWTFGQGTKLEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLS
SPVTKSFNRGEC

SEQ ID 66 - CA8 M1 Humanised light chain (Polynucleotide)

GACATCCAGATGACCCAGAGCCCTAGCTCACTGAGCGCCAGCGTGGGCGACAGGGTGACCATT
ACCTGCTCCGCCAGCCAGGACATCAGCAACTACCTGAACTGGTACCAGCAGAAGCCCGGCAAG
GCCCCCAAGCTGCTGATCTACTACACCTCCAACCTGCACTCCGGCGTGCCCAGCAGGTTCAGCG
GAAGCGGCAGCGGCACCGATTACACCCTGACCATCTCCAGCCTGCAGCCCGAGGACTTCGCCA
CCTACTACTGCCAGCAGTACAGGAAGCTCCCCTGGACTTTCGGCCAGGGCACCAAACTGGAGAT

CAAGCGTACGGTGGCCGCCCCCAGCGTGTTCATCTTCCCCCCCAGCGATGAGCAGCTGAAGAG
CGGCACCGCCAGCGTGGTGTGTCTGCTGAACAACTTCTACCCCCGGGAGGCCAAGGTGCAGTG
GAAGGTGGACAATGCCCTGCAGAGCGGCAACAGCCAGGAGAGCGTGACCGAGCAGGACAGCA
AGGACTCCACCTACAGCCTGAGCAGCACCCTGACCCTGAGCAAGGCCGACTACGAGAAGCACA
AGGTGTACGCCTGTGAGGTGACCCACCAGGGCCTGTCCAGCCCCGTGACCAAGAGCTTCAACC
GGGGCGAGTGC

SEQ ID 67 - CA8 M2 Humanised light chain

DIQLTQSPSSLSASVGDRVTITCSASQDISNYLNWYQQKPGKAPELVIYYTSNLHSGVPSRFSGSGSG
TDYTLTISSLQPEDFATYYCQQYRKLPWTFGQGTKLEIKRTVAAPSVFIFPPSDEQLKSGTASVVCLLN
NFYPREAKVQWKVDNALQSGNSQESVTEQDSKDSTYSLSSTLTLSKADYEKHKVYACEVTHQGLSS
PVTKSFNRGEC

SEQ ID 68 - CA8 M2 Humanised light chain (Polynucleotide)

GACATCCAGCTGACCCAGAGCCCTAGCTCACTGAGCGCCAGCGTGGGCGACAGGGTGACCATTACCTGCTCCGCCAGCCAGGACATCAGCAACTACCTGAACTGGTACCAGCAGAAGCCCGGCAAGGCCCCCGAGCTGGTGATCTACTACACCTCCAACCTGCACTCCGGCGTGCCCAGCAGGTTCAGCGGAAGCGGCAGCGGCACCGATTACACCCTGACCATCTCCAGCCTGCAGCCCGAGGACTTCGCCACCTACTACTGCCAGCAGTACAGGAAGCTCCCCTGGACTTTCGGCCAGGGCACCAAACTGGAGATCAAGCGTACGGTGGCCGCCCCCAGCGTGTTCATCTTCCCCCCCAGCGATGAGCAGCTGAAGAGCGGCACCGCCAGCGTGGTGTGTCTGCTGAACAACTTCTACCCCCGGGAGGCCAAGGTGCAGTGGAAGGTGGACAATGCCCTGCAGAGCGGCAACAGCCAGGAGAGCGTGACCGAGCAGGACAGCAAGGACTCCACCTACAGCCTGAGCAGCACCCTGACCCTGAGCAAGGCCGACTACGAGAAGCACAAGGTGTACGCCTGTGAGGTGACCCACCAGGGCCTGTCCAGCCCCGTGACCAAGAGCTTCAACCGGGGCGAGTGC

SEQ ID 69 - S307118G03 mouse variable heavy

EVQLQQSGPELVKPGASVKISCKASGYTFTDYYMKWVKQSHGKSLEWIGEIYPNNGGITYNQKFKGKATLTVDKSSSTAYMELRSLTSEDSAVYYCANGYEFVYWGQGTLVTVSA

SEQ ID 70 - S307118G03 mouse variable heavy (DNA sequence)

GAGGTCCAGTTGCAACAATCTGGACCTGAGCTGGTGAAGCCTGGGGCTTCAGTGAAGATATCCTGTAAGGCTTCTGGATACACATTCACTGACTACTACATGAAGTGGGTGAAGCAGAGCCATGGAAAGAGCCTTGAGTGGATTGGAGAGATTTATCCTAATAATGGTGGTATTACCTACAACCAGAAGTTCAAGGGCAAGGCCACATTGACTGTAGACAAGTCCTCCAGCACAGCCTACATGGAGCTCCGCAGCCTGACATCTGAGGACTCTGCAGTCTATTACTGTGCAAATGGTTACGAGTTTGTTTACTGGGGCCAAGGGACTCTGGTCACTGTCTCTGCA

SEQ ID 71 - S307118G03 mouse variable light

DIQMTQTASSLSASLGDRVTISCSASQGISNYLNWYQQKPDGTVKLLIYYTSSLHSGVPSRFSGSGSGTDYSLTISNLEPEDIATYYCQQYSKLPWTFGGGTKLEIKR

SEQ ID 72 - S307118G03 mouse variable light (DNA sequence)

GATATCCAGATGACACAGACTGCATCCTCCCTGTCTGCCTCTCTGGGAGACAGAGTCACCATCAGTTGCAGTGCAAGTCAGGGCATTAGCAATTATTTAAACTGGTATCAGCAGAAACCAGATGGAACTGTTAAACTCCTGATCTATTACACATCAAGTTTACACTCAGGAGTCCCATCAAGGTTCAGTGGCAGTGGGTCTGGGACAGATTATTCTCTCACCATCAGCAACCTGGAACCTGAAGATATTGCCACTTACTATTGTCAGCAGTATAGTAAGCTTCCGTGGACGTTCGGTGGAGGCACCAAGCTGGAAATCAAACGG

SEQ ID 73 - S307118G03 chimeric heavy chain

EVQLQQSGPELVKPGASVKISCKASGYTFTDYYMKWVKQSHGKSLEWIGEIYPNNGGITYNQKFKGK ATLTVDKSSSTAYMELRSLTSEDSAVYYCANGYEFVYWGQGTLVTVSAAKTTAPSVFPLAPSSKSTS GGTAALGCLVKDYFPEPVTVSWNSGALTSGVHTFPAVLQSSGLYSLSSVVTVPSSSLGTQTYICNVN HKPSNTKVDKKVEPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVSNKALPAPIEKTIS KAKGQPREPQVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK

SEQ ID 74 - S307118G03 chimeric heavy chain (DNA sequence)

GAGGTCCAGTTGCAACAATCTGGACCTGAGCTGGTGAAGCCTGGGGCTTCAGTGAAGATATCCT GTAAGGCTTCTGGATACACATTCACTGACTACTACATGAAGTGGGTGAAGCAGAGCCATGGAAA GAGCCTTGAGTGGATTGGAGAGATTTATCCTAATAATGGTGGTATTACCTACAACCAGAAGTTCA AGGGCAAGGCCACATTGACTGTAGACAAGTCCTCCAGCACAGCCTACATGGAGCTCCGCAGCCT GACATCTGAGGACTCTGCAGTCTATTACTGTGCAAATGGTTACGAGTTTGTTTACTGGGGCCAAG GGACTCTGGTCACTGTCTCTGCAGCCAAAACAACAGCCCCCAGCGTGTTCCCCCTGGCCCCCAG CAGCAAGAGCACCAGCGGCGGCACAGCCGCCCTGGGCTGCCTGGTGAAGGACTACTTCCCCGA ACCGGTGACCGTGTCCTGGAACAGCGGAGCCCTGACCAGCGGCGTGCACACCTTCCCCGCCGT GCTGCAGAGCAGCGGCCTGTACAGCCTGAGCAGCGTGGTGACCGTGCCCAGCAGCAGCCTGG GCACCCAGACCTACATCTGTAACGTGAACCACAAGCCCAGCAACACCAAGGTGGACAAGAAGGT GGAGCCCAAGAGCTGTGACAAGACCCACACCTGCCCCCCCTGCCCTGCCCCCGAGCTGCTGGG AGGCCCCAGCGTGTTCCTGTTCCCCCCCAAGCCTAAGGACACCCTGATGATCAGCAGAACCCCC GAGGTGACCTGTGTGGTGGTGGATGTGAGCCACGAGGACCCTGAGGTGAAGTTCAACTGGTAC GTGGACGGCGTGGAGGTGCACAATGCCAAGACCAAGCCCAGGGAGGAGCAGTACAACAGCACC TACCGGGTGGTGTCCGTGCTGACCGTGCTGCACCAGGATTGGCTGAACGGCAAGGAGTACAAG TGTAAGGTGTCCAACAAGGCCCTGCCTGCCCCTATCGAGAAAACCATCAGCAAGGCCAAGGGCC AGCCCAGAGAGCCCCAGGTGTACACCCTGCCCCCTAGCAGAGATGAGCTGACCAAGAACCAGG TGTCCCTGACCTGCCTGGTGAAGGGCTTCTACCCCAGCGACATCGCCGTGGAGTGGGAGAGCA ACGGCCAGCCCGAGAACAACTACAAGACCACCCCCCCTGTGCTGGACAGCGATGGCAGCTTCT

TCCTGTACAGCAAGCTGACCGTGGACAAGAGCAGATGGCAGCAGGGCAACGTGTTCAGCTGCT CCGTGATGCACGAGGCCCTGCACAATCACTACACCCAGAAGAGCCTGAGCCTGTCCCCTGGCA AG

SEQ ID 75 - S307118G03 chimeric light chain

DIQMTQTASSLSASLGDRVTISCSASQGISNYLNWYQQKPDGTVKLLIYYTSSLHSGVPSRFSGSGSG TDYSLTISNLEPEDIATYYCQQYSKLPWTFGGGTKLELKRTVAAPSVFIFPPSDEQLKSGTASVVCLLN NFYPREAKVQWKVDNALQSGNSQESVTEQDSKDSTYSLSSTLTLSKADYEKHKVYACEVTHQGLSS PVTKSFNRGEC

SEQ ID 76 - S307118G03 chimeric light chain (DNA sequence)

GATATCCAGATGACACAGACTGCATCCTCCCTGTCTGCCTCTCTGGGAGACAGAGTCACCATCA GTTGCAGTGCAAGTCAGGGCATTAGCAATTATTTAAACTGGTATCAGCAGAAACCAGATGGAACT GTTAAACTCCTGATCTATTACACATCAAGTTTACACTCAGGAGTCCCATCAAGGTTCAGTGGCAG TGGGTCTGGGACAGATTATTCTCTCACCATCAGCAACCTGGAACCTGAAGATATTGCCACTTACT ATTGTCAGCAGTATAGTAAGCTTCCGTGGACGTTCGGTGGAGGCACCAAGCTGGAGCTGAAACG TACGGTGGCCGCCCCCAGCGTGTTCATCTTCCCCCCCAGCGATGAGCAGCTGAAGAGCGGCAC CGCCAGCGTGGTGTGTCTGCTGAACAACTTCTACCCCCGGGAGGCCAAGGTGCAGTGGAAGGT GGACAATGCCCTGCAGAGCGGCAACAGCCAGGAGAGCGTGACCGAGCAGGACAGCAAGGACT CCACCTACAGCCTGAGCAGCACCCTGACCCTGAGCAAGGCCGACTACGAGAAGCACAAGGTGT ACGCCTGTGAGGTGACCCACCAGGGCCTGTCCAGCCCCGTGACCAAGAGCTTCAACCGGGGCG AGTGC

SEQ ID 77 - S307118G03 humanised H0 variable heavy

QVQLVQSGAEVKKPGSSVKVSCKASGGTFSDYYMKWVRQAPGQGLEWMGEIYPNNGGITYNQKFK GRVTITADKSTSTAYMELSSLRSEDTAVYYCARGYEFVYWGQGTLVTVSS

SEQ ID 78 - S307118G03 humanised H0 variable heavy (DNA sequence)

CAGGTGCAGCTGGTGCAGAGCGGCGCCGAAGTGAAGAAGCCCGGCTCCAGCGTGAAGGTGAG CTGCAAGGCTAGCGGCGGCACCTTCAGCGACTACTACATGAAGTGGGTGAGGCAGGCCCCCGG CCAGGGACTGGAGTGGATGGGCGAGATCTACCCCAACAACGGGGGCATCACCTACAACCAGAA GTTCAAGGGCAGGGTGACCATCACCGCCGACAAAAGCACCAGCACCGCCTACATGGAACTGAG CAGCCTGAGGAGCGAGGACACCGCCGTGTACTACTGCGCCAGGGGCTACGAGTTCGTGTATTG GGGCCAGGGCACACTAGTGACCGTGTCCAGC

SEQ ID 79 - S307118G03 humanised H1 variable heavy

QVQLVQSGAEVKKPGSSVKVSCKASGYTFTDYYMKWVRQAPGQGLEWMGEIYPNNGGITYNQKFK GRVTITADKSTSTAYMELSSLRSEDTAVYYCARGYEFVYWGQGTLVTVSS

SEQ ID 80 - S307118G03 humanised H1 variable heavy (DNA sequence)

CAGGTGCAGCTGGTGCAGAGCGGCGCCGAAGTGAAGAAGCCCGGCTCCAGCGTGAAGGTGAG CTGCAAGGCTAGCGGCTACACCTTCACCGACTACTACATGAAGTGGGTGAGGCAGGCCCCCGG CCAGGGACTGGAGTGGATGGGCGAGATCTACCCCAACAACGGGGGCATCACCTACAACCAGAA GTTCAAGGGCAGGGTGACCATCACCGCCGACAAAAGCACCAGCACCGCCTACATGGAACTGAG CAGCCTGAGGAGCGAGGACACCGCCGTGTACTACTGCGCCAGGGGCTACGAGTTCGTGTATTG GGGCCAGGGCACACTAGTGACCGTGTCCAGC

SEQ ID 81 - S307118G03 humanised H2 variable heavy

QVQLVQSGAEVKKPGSSVKVSCKASGYTFTDYYMKWVRQAPGQGLEWMGEIYPNNGGITYNQKFK GRVTITADKSTSTAYMELSSLRSEDTAVYYCANGYEFVYWGQGTLVTVSS

SEQ ID 82 - S307118G03 humanised H2 variable heavy (DNA sequence)

CAGGTGCAGCTGGTGCAGAGCGGCGCCGAAGTGAAGAAGCCCGGCTCCAGCGTGAAGGTGAG
CTGCAAGGCTAGCGGCTACACCTTCACCGACTACTACATGAAGTGGGTGAGGCAGGCCCCCGG
CCAGGGACTGGAGTGGATGGGCGAGATCTACCCCAACAACGGGGGCATCACCTACAACCAGAA
GTTCAAGGGCAGGGTGACCATCACCGCCGACAAAAGCACCAGCACCGCCTACATGGAACTGAG
CAGCCTGAGGAGCGAGGACACCGCCGTGTACTACTGCGCCAACGGCTACGAGTTCGTGTATTG
GGGCCAGGGCACACTAGTGACCGTGTCCAGC

SEQ ID 83 - S307118G03 humanised H3 variable heavy

QVQLVQSGAEVKKPGSSVKVSCKASGYTFTDYYMKWVRQAPGQGLEWIGEIYPNNGGITYNQKFKG
RATLTVDKSTSTAYMELSSLRSEDTAVYYCANGYEFVYWGQGTLVTVSS

SEQ ID 84 - S307118G03 humanised H3 variable heavy (DNA sequence)

CAGGTGCAGCTGGTGCAGAGCGGCGCCGAAGTGAAGAAGCCCGGCTCCAGCGTGAAGGTGAG
CTGCAAGGCTAGCGGCTACACCTTCACCGACTACTACATGAAGTGGGTGAGGCAGGCCCCCGG
CCAGGGACTGGAGTGGATAGGCGAGATCTACCCCAACAACGGGGGCATCACCTACAACCAGAA
GTTCAAGGGCAGGGCGACCCTCACCGTCGACAAAAGCACCAGCACCGCCTACATGGAACTGAG
CAGCCTGAGGAGCGAGGACACCGCCGTGTACTACTGCGCCAACGGCTACGAGTTCGTGTATTG
GGGCCAGGGCACACTAGTGACCGTGTCCAGC

SEQ ID 85 - S307118G03 humanised H4 variable heavy

QVQLVQSGAEVKKPGSSVKVSCKASGYTFTDYYMKWVRQAPGQGLEWMGEIYPNNGGITYNQKFK
GRVTITADKSTSTAYMELSSLRSEDTAVYYCADGYEFVYWGQGTLVTVSS

SEQ ID 86 - S307118G03 humanised H4 variable heavy (DNA sequence)

CAGGTGCAGCTGGTGCAGAGCGGCGCCGAAGTGAAGAAGCCCGGCTCCAGCGTGAAGGTGAG
CTGCAAGGCTAGCGGCTACACCTTCACCGACTACTACATGAAGTGGGTGAGGCAGGCCCCCGG
CCAGGGACTGGAGTGGATGGGCGAGATCTACCCCAACAACGGGGGCATCACCTACAACCAGAA
GTTCAAGGGCAGGGTGACCATCACCGCCGACAAAAGCACCAGCACCGCCTACATGGAACTGAG

CAGCCTGAGGAGCGAGGACACCGCCGTGTACTACTGCGCCGACGGCTACGAGTTCGTGTATTG
GGGCCAGGGCACACTAGTGACCGTGTCCAGC

SEQ ID 87 - S307118G03 humanised H5 variable heavy

QVQLVQSGAEVKKPGSSVKVSCKASGYTFTDYYMKWVRQAPGQGLEWIGEIYPNNGGITYNQKFKG
RATLTVDKSTSTAYMELSSLRSEDTAVYYCANGYEFDYWGQGTLVTVSS

SEQ ID 88 - S307118G03 humanised H5 variable heavy (DNA sequence)

CAGGTGCAGCTGGTGCAGAGCGGCGCCGAAGTGAAGAAGCCCGGCTCCAGCGTGAAGGTGAGCTGCAAGGCTAGCGGCTACACCTTCACCGACTACTACATGAAGTGGGTGAGGCAGGCCCCCGGCCAGGGACTGGAGTGGATAGGCGAGATCTACCCCAACAACGGGGGCATCACCTACAACCAGAAGTTCAAGGGCAGGGCGACCCTCACCGTCGACAAAAGCACCAGCACCGCCTACATGGAACTGAGCAGCCTGAGGAGCGAGGACACCGCCGTGTACTACTGCGCCAACGGCTACGAGTTCGACTATTGGGGCCAGGGCACACTAGTGACCGTGTCCAGC

SEQ ID 89 - S307118G03 humanised L0 variable light

DIQMTQSPSSLSASVGDRVTITCSASQGISNYLNWYQQKPGKAPKLLIYYTSSLHSGVPSRFSGSGSGTDFTLTISSLQPEDFATYYCQQYSKLPWTFGQGTKLEIKR

SEQ ID 90 - S307118G03 humanised L0 variable light (DNA sequence)

GACATCCAGATGACCCAGAGCCCCTCAAGCCTGAGCGCCAGCGTGGGCGACAGGGTGACTATCACCTGCAGCGCCTCCCAGGGCATCAGCAACTACCTGAACTGGTACCAGCAGAAGCCCGGCAAGGCCCCTAAGCTGCTGATCTACTACACCAGCAGCCTGCACAGCGGCGTGCCCAGCAGGTTCTCCGGCAGCGGCAGCGGAACCGACTTCACCCTGACCATTAGCAGCCTCCAGCCCGAGGACTTCGCCACCTACTACTGCCAGCAGTACAGCAAGCTGCCCTGGACCTTCGGCCAGGGCACCAAACTGGAGATCAAGCGT

SEQ ID 91 - S307118G03 humanised L1 variable light

DIQMTQSPSSLSASVGDRVTITCSASQGISNYLNWYQQKPGKAPKLLIYYTSSLHSGVPSRFSGSGSGTDYTLTISSLQPEDFATYYCQQYSKLPWTFGQGTKLEIKR

SEQ ID 92 - S307118G03 humanised L1 variable light (DNA sequence)

GACATCCAGATGACCCAGAGCCCCTCAAGCCTGAGCGCCAGCGTGGGCGACAGGGTGACTATCACCTGCAGCGCCTCCCAGGGCATCAGCAACTACCTGAACTGGTACCAGCAGAAGCCCGGCAAGGCCCCTAAGCTGCTGATCTACTACACCAGCAGCCTGCACAGCGGCGTGCCCAGCAGGTTCTCCGGCAGCGGCAGCGGAACCGACTACACCCTGACCATTAGCAGCCTCCAGCCCGAGGACTTCGCCACCTACTACTGCCAGCAGTACAGCAAGCTGCCCTGGACCTTCGGCCAGGGCACCAAACTGGAGATCAAGCGT

SEQ ID 93 - S307118G03 CDRH1
DYYMK
SEQ ID 94 - S307118G03 CDRH2
EIYPNNGGITYNQKFKG
SEQ ID 95 - S307118G03 CDRH3
GYEFVY
SEQ ID 96 - S307118G03 CDRL1
SASQGISNYLN
SEQ ID 97 - S307118G03 CDRL2
YTSSLHS
SEQ ID 98 - S307118G03 CDRL3
QQYSKLPWT

SEQ ID 99 - S307118G03 humanised H5 CDRH3
GYEFDY
SEQ ID 100 - S307118G03 humanised H0 heavy chain

QVQLVQSGAEVKKPGSSVKVSCKASGGTFSDYYMKWVRQAPGQGLEWMGEIYPNNGGITYNQKFK
GRVTITADKSTSTAYMELSSLRSEDTAVYYCARGYEFVYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK

SEQ ID 101 - S307118G03 humanised H0 heavy chain (polynucleotide)

CAGGTGCAGCTGGTGCAGAGCGGCGCCGAAGTGAAGAAGCCCGGCTCCAGCGTGAAGGTGAG
CTGCAAGGCTAGCGGCGGCACCTTCAGCGACTACTACATGAAGTGGGTGAGGCAGGCCCCCGG
CCAGGGACTGGAGTGGATGGGCGAGATCTACCCCAACAACGGGGGCATCACCTACAACCAGAA
GTTCAAGGGCAGGGTGACCATCACCGCCGACAAAAGCACCAGCACCGCCTACATGGAACTGAG
CAGCCTGAGGAGCGAGGACACCGCCGTGTACTACTGCGCCAGGGGCTACGAGTTCGTGTATTG
GGGCCAGGGCACACTAGTGACCGTGTCCAGCGCCAGCACCAAGGGCCCCAGCGTGTTCCCCCT
GGCCCCCAGCAGCAAGAGCACCAGCGGCGGCACAGCCGCCCTGGGCTGCCTGGTGAAGGACT
ACTTCCCCGAACCGGTGACCGTGTCCTGGAACAGCGGAGCCCTGACCAGCGGCGTGCACACCT
TCCCCGCCGTGCTGCAGAGCAGCGGCCTGTACAGCCTGAGCAGCGTGGTGACCGTGCCCAGCA
GCAGCCTGGGCACCCAGACCTACATCTGTAACGTGAACCACAAGCCCAGCAACACCAAGGTGG

ACAAGAAGGTGGAGCCCAAGAGCTGTGACAAGACCCACACCTGCCCCCCCTGCCCTGCCCCCG
AGCTGCTGGGAGGCCCCAGCGTGTTCCTGTTCCCCCCCAAGCCTAAGGACACCCTGATGATCA
GCAGAACCCCCGAGGTGACCTGTGTGGTGGTGGATGTGAGCCACGAGGACCCTGAGGTGAAGT
TCAACTGGTACGTGGACGGCGTGGAGGTGCACAATGCCAAGACCAAGCCCAGGGAGGAGCAGT
ACAACAGCACCTACCGGGTGGTGTCCGTGCTGACCGTGCTGCACCAGGATTGGCTGAACGGCA
AGGAGTACAAGTGTAAGGTGTCCAACAAGGCCCTGCCTGCCCCTATCGAGAAAACCATCAGCAA
GGCCAAGGGCCAGCCCAGAGAGCCCCAGGTGTACACCCTGCCCCCTAGCAGAGATGAGCTGAC
CAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGA
TGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTG
TCCCCTGGCAAG

SEQ ID 102 - S307118G03 humanised H1 heavy chain

QVQLVQSGAEVKKPGSSVKVSCKASGYTFTDYYMKWVRQAPGQGLEWMGEIYPNNGGITYNQKFK
GRVTITADKSTSTAYMELSSLRSEDTAVYYCARGYEFVYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK

SEQ ID 103 - S307118G03 humanised H1 heavy chain (DNA sequence)

CAGGTGCAGCTGGTGCAGAGCGGCGCCGAAGTGAAGAAGCCCGGCTCCAGCGTGAAGGTGAG
CTGCAAGGCTAGCGGCTACACCTTCACCGACTACTACATGAAGTGGGTGAGGCAGGCCCCCGG
CCAGGGACTGGAGTGGATGGGCGAGATCTACCCCAACAACGGGGGCATCACCTACAACCAGAA
GTTCAAGGGCAGGGTGACCATCACCGCCGACAAAAGCACCAGCACCGCCTACATGGAACTGAG
CAGCCTGAGGAGCGAGGACACCGCCGTGTACTACTGCGCCAGGGGCTACGAGTTCGTGTATTG
GGGCCAGGGCACACTAGTGACCGTGTCCAGCGCCAGCACCAAGGGCCCCAGCGTGTTCCCCCT
GGCCCCCAGCAGCAAGAGCACCAGCGGCGGCACAGCCGCCCTGGGCTGCCTGGTGAAGGACT
ACTTCCCCGAACCGGTGACCGTGTCCTGGAACAGCGGAGCCCTGACCAGCGGCGTGCACACCT
TCCCCGCCGTGCTGCAGAGCAGCGGCCTGTACAGCCTGAGCAGCGTGGTGACCGTGCCCAGCA
GCAGCCTGGGCACCCAGACCTACATCTGTAACGTGAACCACAAGCCCAGCAACACCAAGGTGG
ACAAGAAGGTGGAGCCCAAGAGCTGTGACAAGACCCACACCTGCCCCCCCTGCCCTGCCCCCG
AGCTGCTGGGAGGCCCCAGCGTGTTCCTGTTCCCCCCCAAGCCTAAGGACACCCTGATGATCA
GCAGAACCCCCGAGGTGACCTGTGTGGTGGTGGATGTGAGCCACGAGGACCCTGAGGTGAAGT
TCAACTGGTACGTGGACGGCGTGGAGGTGCACAATGCCAAGACCAAGCCCAGGGAGGAGCAGT
ACAACAGCACCTACCGGGTGGTGTCCGTGCTGACCGTGCTGCACCAGGATTGGCTGAACGGCA
AGGAGTACAAGTGTAAGGTGTCCAACAAGGCCCTGCCTGCCCCTATCGAGAAAACCATCAGCAA
GGCCAAGGGCCAGCCCAGAGAGCCCCAGGTGTACACCCTGCCCCCTAGCAGAGATGAGCTGAC

CAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGA
TGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTG
TCCCCTGGCAAG

SEQ ID 104 - S307118G03 humanised H2 heavy chain

QVQLVQSGAEVKKPGSSVKVSCKASGYTFTDYYMKWVRQAPGQGLEWMGEIYPNNGGITYNQKFK
GRVTITADKSTSTAYMELSSLRSEDTAVYYCANGYEFVYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK

SEQ ID 105 - S307118G03 humanised H2 heavy chain (DNA sequence)

CAGGTGCAGCTGGTGCAGAGCGGCGCCGAAGTGAAGAAGCCCGGCTCCAGCGTGAAGGTGAG
CTGCAAGGCTAGCGGCTACACCTTCACCGACTACTACATGAAGTGGGTGAGGCAGGCCCCCGG
CCAGGGACTGGAGTGGATGGGCGAGATCTACCCCAACAACGGGGGCATCACCTACAACCAGAA
GTTCAAGGGCAGGGTGACCATCACCGCCGACAAAAGCACCAGCACCGCCTACATGGAACTGAG
CAGCCTGAGGAGCGAGGACACCGCCGTGTACTACTGCGCCAACGGCTACGAGTTCGTGTATTG
GGGCCAGGGCACACTAGTGACCGTGTCCAGCGCCAGCACCAAGGGCCCCAGCGTGTTCCCCCT
GGCCCCCAGCAGCAAGAGCACCAGCGGCGGCACAGCCGCCCTGGGCTGCCTGGTGAAGGACT
ACTTCCCCGAACCGGTGACCGTGTCCTGGAACAGCGGAGCCCTGACCAGCGGCGTGCACACCT
TCCCCGCCGTGCTGCAGAGCAGCGGCCTGTACAGCCTGAGCAGCGTGGTGACCGTGCCCAGCA
GCAGCCTGGGCACCCAGACCTACATCTGTAACGTGAACCACAAGCCCAGCAACACCAAGGTGG
ACAAGAAGGTGGAGCCCAAGAGCTGTGACAAGACCCACACCTGCCCCCCCTGCCCTGCCCCCG
AGCTGCTGGGAGGCCCCAGCGTGTTCCTGTTCCCCCCCAAGCCTAAGGACACCCTGATGATCA
GCAGAACCCCCGAGGTGACCTGTGTGGTGGTGGATGTGAGCCACGAGGACCCTGAGGTGAAGT
TCAACTGGTACGTGGACGGCGTGGAGGTGCACAATGCCAAGACCAAGCCCAGGGAGGAGCAGT
ACAACAGCACCTACCGGGTGGTGTCCGTGCTGACCGTGCTGCACCAGGATTGGCTGAACGGCA
AGGAGTACAAGTGTAAGGTGTCCAACAAGGCCCTGCCTGCCCCTATCGAGAAAACCATCAGCAA
GGCCAAGGGCCAGCCCAGAGAGCCCCAGGTGTACACCCTGCCCCCTAGCAGAGATGAGCTGAC
CAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGA
TGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTG
TCCCCTGGCAAG

SEQ ID 106 - S307118G03 humanised H3 heavy chain

QVQLVQSGAEVKKPGSSVKVSCKASGYTFTDYYMKWVRQAPGQGLEWIGEIYPNNGGITYNQKFKG
RATLTVDKSTSTAYMELSSLRSEDTAVYYCANGYEFVYWGQGTLVTVSSASTKGPSVFPLAPSSKST
SGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK

SEQ ID 107 - S307118G03 humanised H3 heavy chain (DNA sequence)

CAGGTGCAGCTGGTGCAGAGCGGCGCCGAAGTGAAGAAGCCCGGCTCCAGCGTGAAGGTGAG
CTGCAAGGCTAGCGGCTACACCTTCACCGACTACTACATGAAGTGGGTGAGGCAGGCCCCCGG
CCAGGGACTGGAGTGGATAGGCGAGATCTACCCCAACAACGGGGGCATCACCTACAACCAGAA
GTTCAAGGGCAGGGCGACCCTCACCGTCGACAAAAGCACCAGCACCGCCTACATGGAACTGAG
CAGCCTGAGGAGCGAGGACACCGCCGTGTACTACTGCGCCAACGGCTACGAGTTCGTGTATTG
GGGCCAGGGCACACTAGTGACCGTGTCCAGCGCCAGCACCAAGGGCCCCAGCGTGTTCCCCCT
GGCCCCCAGCAGCAAGAGCACCAGCGGCGGCACAGCCGCCCTGGGCTGCCTGGTGAAGGACT
ACTTCCCCGAACCGGTGACCGTGTCCTGGAACAGCGGAGCCCTGACCAGCGGCGTGCACACCT
TCCCCGCCGTGCTGCAGAGCAGCGGCCTGTACAGCCTGAGCAGCGTGGTGACCGTGCCCAGCA
GCAGCCTGGGCACCCAGACCTACATCTGTAACGTGAACCACAAGCCCAGCAACACCAAGGTGG
ACAAGAAGGTGGAGCCCAAGAGCTGTGACAAGACCCACACCTGCCCCCCCTGCCCTGCCCCCG
AGCTGCTGGGAGGCCCCAGCGTGTTCCTGTTCCCCCCCAAGCCTAAGGACACCCTGATGATCA
GCAGAACCCCCGAGGTGACCTGTGTGGTGGTGGATGTGAGCCACGAGGACCCTGAGGTGAAGT
TCAACTGGTACGTGGACGGCGTGGAGGTGCACAATGCCAAGACCAAGCCCAGGGAGGAGCAGT
ACAACAGCACCTACCGGGTGGTGTCCGTGCTGACCGTGCTGCACCAGGATTGGCTGAACGGCA
AGGAGTACAAGTGTAAGGTGTCCAACAAGGCCCTGCCTGCCCCTATCGAGAAAACCATCAGCAA
GGCCAAGGGCCAGCCCAGAGAGCCCCAGGTGTACACCCTGCCCCCTAGCAGAGATGAGCTGAC
CAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGA
TGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTG
TCCCCTGGCAAG

SEQ ID 108 - S307118G03 humanised H4 heavy chain

QVQLVQSGAEVKKPGSSVKVSCKASGYTFTDYYMKWVRQAPGQGLEWMGEIYPNNGGITYNQKFK
GRVTITADKSTSTAYMELSSLRSEDTAVYYCADGYEFVYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEYKCKVSNKALPAPIEK
TISKAKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK

SEQ ID 109 - S307118G03 humanised H4 heavy chain (DNA sequence)

CAGGTGCAGCTGGTGCAGAGCGGCGCCGAAGTGAAGAAGCCCGGCTCCAGCGTGAAGGTGAG
CTGCAAGGCTAGCGGCTACACCTTCACCGACTACTACATGAAGTGGGTGAGGCAGGCCCCCGG
CCAGGGACTGGAGTGGATGGGCGAGATCTACCCCAACAACGGGGGCATCACCTACAACCAGAA
GTTCAAGGGCAGGGTGACCATCACCGCCGACAAAAGCACCAGCACCGCCTACATGGAACTGAG
CAGCCTGAGGAGCGAGGACACCGCCGTGTACTACTGCGCCGACGGCTACGAGTTCGTGTATTG
GGGCCAGGGCACACTAGTGACCGTGTCCAGCGCCAGCACCAAGGGCCCCAGCGTGTTCCCCCT
GGCCCCCAGCAGCAAGAGCACCAGCGGCGGCACAGCCGCCCTGGGCTGCCTGGTGAAGGACT
ACTTCCCCGAACCGGTGACCGTGTCCTGGAACAGCGGAGCCCTGACCAGCGGCGTGCACACCT
TCCCCGCCGTGCTGCAGAGCAGCGGCCTGTACAGCCTGAGCAGCGTGGTGACCGTGCCCAGCA
GCAGCCTGGGCACCCAGACCTACATCTGTAACGTGAACCACAAGCCCAGCAACACCAAGGTGG
ACAAGAAGGTGGAGCCCAAGAGCTGTGACAAGACCCACACCTGCCCCCCCTGCCCTGCCCCCG
AGCTGCTGGGAGGCCCCAGCGTGTTCCTGTTCCCCCCCAAGCCTAAGGACACCCTGATGATCA
GCAGAACCCCCGAGGTGACCTGTGTGGTGGTGGATGTGAGCCACGAGGACCCTGAGGTGAAGT
TCAACTGGTACGTGGACGGCGTGGAGGTGCACAATGCCAAGACCAAGCCCAGGGAGGAGCAGT
ACAACAGCACCTACCGGGTGGTGTCCGTGCTGACCGTGCTGCACCAGGATTGGCTGAACGGCA
AGGAGTACAAGTGTAAGGTGTCCAACAAGGCCCTGCCTGCCCCTATCGAGAAAACCATCAGCAA
GGCCAAGGGCCAGCCCAGAGAGCCCCAGGTGTACACCCTGCCCCCTAGCAGAGATGAGCTGAC
CAAGAACCAGGTGTCCCTGACCTGCCTGGTGAAGGGCTTCTACCCCAGCGACATCGCCGTGGA
GTGGGAGAGCAACGGCCAGCCCGAGAACAACTACAAGACCACCCCCCCTGTGCTGGACAGCGA
TGGCAGCTTCTTCCTGTACAGCAAGCTGACCGTGGACAAGAGCAGATGGCAGCAGGGCAACGT
GTTCAGCTGCTCCGTGATGCACGAGGCCCTGCACAATCACTACACCCAGAAGAGCCTGAGCCTG
TCCCCTGGCAAG

SEQ ID 110 - S307118G03 humanised H5 heavy chain

QVQLVQSGAEVKKPGSSVKVSCKASGYTFTDYYMKWVRQAPGQGLEWIGEIYPNNGGITYNQKFKG
RATLTVDKSTSTAYMELSSLRSEDTAVYYCANGYEFDYWGQGTLVTVSSASTKGPSVFPLAPSSKST
SGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPVLDSDG
SFFLYSKLTVDKSRWQQGNVFSCSVMHEALHNHYTQKSLSLSPGK

SEQ ID 111 - S307118G03 humanised H5 heavy chain (DNA sequence)

CAGGTGCAGCTGGTGCAGAGCGGCGCCGAAGTGAAGAAGCCCGGCTCCAGCGTGAAGGTGAG
CTGCAAGGCTAGCGGCTACACCTTCACCGACTACTACATGAAGTGGGTGAGGCAGGCCCCCGG
CCAGGGACTGGAGTGGATAGGCGAGATCTACCCCAACAACGGGGGCATCACCTACAACCAGAA
GTTCAAGGGCAGGGCGACCCTCACCGTCGACAAAAGCACCAGCACCGCCTACATGGAACTGAG
CAGCCTGAGGAGCGAGGACACCGCCGTGTACTACTGCGCCAACGGCTACGAGTTCGACTATTG

GGGCCAGGGCACACTAGTGACCGTGTCCAGCGCCAGCACCAAGGGCCCCAGCGTGTTCCCCCT GGCCCCCAGCAGCAAGAGCACCAGCGGCGGCACAGCCGCCCTGGGCTGCCTGGTGAAGGACT ACTTCCCCGAACCGGTGACCGTGTCCTGGAACAGCGGAGCCCTGACCAGCGGCGTGCACACCT TCCCCGCCGTGCTGCAGAGCAGCGGCCTGTACAGCCTGAGCAGCGTGGTGACCGTGCCCAGCA GCAGCCTGGGCACCCAGACCTACATCTGTAACGTGAACCACAAGCCCAGCAACACCAAGGTGG ACAAGAAGGTGGAGCCCAAGAGCTGTGACAAGACCCACACCTGCCCCCCCTGCCCTGCCCCCG AGCTGCTGGGAGGCCCCAGCGTGTTCCTGTTCCCCCCCAAGCCTAAGGACACCCTGATGATCA GCAGAACCCCCGAGGTGACCTGTGTGGTGGTGGATGTGAGCCACGAGGACCCTGAGGTGAAGT TCAACTGGTACGTGGACGGCGTGGAGGTGCACAATGCCAAGACCAAGCCCAGGGAGGAGCAGT ACAACAGCACCTACCGGGTGGTGTCCGTGCTGACCGTGCTGCACCAGGATTGGCTGAACGGCA AGGAGTACAAGTGTAAGGTGTCCAACAAGGCCCTGCCTGCCCCTATCGAGAAAACCATCAGCAA GGCCAAGGGCCAGCCCAGAGAGCCCCAGGTGTACACCCTGCCCCCTAGCAGAGATGAGCTGAC CAAGAACCAGGTGTCCCTGACCTGCCTGGTGAAGGGCTTCTACCCCAGCGACATCGCCGTGGA GTGGGAGAGCAACGGCCAGCCCGAGAACAACTACAAGACCACCCCCCCTGTGCTGGACAGCGA TGGCAGCTTCTTCCTGTACAGCAAGCTGACCGTGGACAAGAGCAGATGGCAGCAGGGCAACGT GTTCAGCTGCTCCGTGATGCACGAGGCCCTGCACAATCACTACACCCAGAAGAGCCTGAGCCTG TCCCCTGGCAAG

SEQ ID 112 - S307118G03 humanised L0 light chain

DIQMTQSPSSLSASVGDRVTITCSASQGISNYLNWYQQKPGKAPKLLIYYTSSLHSGVPSRFSGSGS GTDFTLTISSLQPEDFATYYCQQYSKLPWTFGQGTKLEIKRTVAAPSVFIFPPSDEQLKSGTASVVCLL NNFYPREAKVQWKVDNALQSGNSQESVTEQDSKDSTYSLSSTLTLSKADYEKHKVYACEVTHQGLS SPVTKSFNRGEC

SEQ ID 113 - S307118G03 humanised L0 light chain (DNA sequence)

GACATCCAGATGACCCAGAGCCCCTCAAGCCTGAGCGCCAGCGTGGGCGACAGGGTGACTATC ACCTGCAGCGCCTCCCAGGGCATCAGCAACTACCTGAACTGGTACCAGCAGAAGCCCGGCAAG GCCCCTAAGCTGCTGATCTACTACACCAGCAGCCTGCACAGCGGCGTGCCCAGCAGGTTCTCC GGCAGCGGCAGCGGAACCGACTTCACCCTGACCATTAGCAGCCTCCAGCCCGAGGACTTCGCC ACCTACTACTGCCAGCAGTACAGCAAGCTGCCCTGGACCTTCGGCCAGGGCACCAAACTGGAG ATCAAGCGTACGGTGGCCGCCCCCAGCGTGTTCATCTTCCCCCCCAGCGATGAGCAGCTGAAG AGCGGCACCGCCAGCGTGGTGTGTCTGCTGAACAACTTCTACCCCCGGGAGGCCAAGGTGCAG TGGAAGGTGGACAATGCCCTGCAGAGCGGCAACAGCCAGGAGAGCGTGACCGAGCAGGACAG CAAGGACTCCACCTACAGCCTGAGCAGCACCCTGACCCTGAGCAAGGCCGACTACGAGAAGCA CAAGGTGTACGCCTGTGAGGTGACCCACCAGGGCCTGTCCAGCCCCGTGACCAAGAGCTTCAA CCGGGGCGAGTGC

SEQ ID 114 - S307118G03 humanised L1 light chain

DIQMTQSPSSLSASVGDRVTITCSASQGISNYLNWYQQKPGKAPKLLIYYTSSLHSGVPSRFSGSGS
GTDYTLTISSLQPEDFATYYCQQYSKLPWTFGQGTKLEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLS
SPVTKSFNRGEC

SEQ ID 115 - S307118G03 humanised L1 light chain (DNA sequence)

GACATCCAGATGACCCAGAGCCCCTCAAGCCTGAGCGCCAGCGTGGGCGACAGGGTGACTATC
ACCTGCAGCGCCTCCCAGGGCATCAGCAACTACCTGAACTGGTACCAGCAGAAGCCCGGCAAG
GCCCCTAAGCTGCTGATCTACTACACCAGCAGCCTGCACAGCGGCGTGCCCAGCAGGTTCTCC
GGCAGCGGCAGCGGAACCGACTACACCCTGACCATTAGCAGCCTCCAGCCCGAGGACTTCGCC
ACCTACTACTGCCAGCAGTACAGCAAGCTGCCCTGGACCTTCGGCCAGGGCACCAAACTGGAG
ATCAAGCGTACGGTGGCCGCCCCCAGCGTGTTCATCTTCCCCCCCAGCGATGAGCAGCTGAAG
AGCGGCACCGCCAGCGTGGTGTGTCTGCTGAACAACTTCTACCCCCGGGAGGCCAAGGTGCAG
TGGAAGGTGGACAATGCCCTGCAGAGCGGCAACAGCCAGGAGAGCGTGACCGAGCAGGACAG
CAAGGACTCCACCTACAGCCTGAGCAGCACCCTGACCCTGAGCAAGGCCGACTACGAGAAGCA
CAAGGTGTACGCCTGTGAGGTGACCCACCAGGGCCTGTCCAGCCCCGTGACCAAGAGCTTCAA
CCGGGGCGAGTGC

SEQ ID 116 - S332121F02 murine variable heavy chain

EVQLQQSGPVLVKPGASVKMSCEASGYTFTDYYMNWVKQSHGKTLEWIGVINPYNGGTDYNQKFK
GKATLTVDKSSSTAYMELNSLTSEDSAVYYCARSVYDYPFDYWGQGTLVTVSS

SEQ ID 117 S332121F02 murine variable heavy chain (DNA sequence)

GAGGTGCAGCTGCAGCAGAGCGGCCCCGTGCTGGTGAAGCCTGGAGCCAGCGTGAAAATGAG
CTGCGAAGCCAGCGGCTACACCTTCACCGACTACTACATGAACTGGGTGAAGCAGAGCCACGG
CAAGACCCTGGAGTGGATCGGCGTGATCAACCCCTACAACGGGGGCACCGACTACAACCAGAA
GTTCAAGGGCAAGGCCACTCTGACCGTGGACAAGAGCTCCAGCACCGCCTACATGGAACTGAA
CAGCCTCACCTCTGAGGACAGCGCCGTCTATTACTGCGCCAGGAGCGTGTACGACTACCCCTTC
GACTACTGGGGCCAGGGCACACTAGTGACCGTGTCCAGC

SEQ ID 118 - S332121F02 chimeric heavy chain

EVQLQQSGPVLVKPGASVKMSCEASGYTFTDYYMNWVKQSHGKTLEWIGVINPYNGGTDYNQKFK
GKATLTVDKSSSTAYMELNSLTSEDSAVYYCARSVYDYPFDYWGQGTLVTVSSASTKGPSVFPLAPS
SKSTSGGTAALGCLVKDYFPEPVTVSWNSGALTSGVHTFPAVLQSSGLYSLSSVVTVPSSSLGTQTYI
CNVNHKPSNTKVDKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK

SEQ ID 119 - S332121F02 chimeric heavy chain (DNA sequence)

GAGGTGCAGCTGCAGCAGAGCGGCCCCGTGCTGGTGAAGCCTGGAGCCAGCGTGAAAATGAG
CTGCGAAGCCAGCGGCTACACCTTCACCGACTACTACATGAACTGGGTGAAGCAGAGCCACGG
CAAGACCCTGGAGTGGATCGGCGTGATCAACCCCTACAACGGGGGCACCGACTACAACCAGAA
GTTCAAGGGCAAGGCCACTCTGACCGTGGACAAGAGCTCCAGCACCGCCTACATGGAACTGAA
CAGCCTCACCTCTGAGGACAGCGCCGTCTATTACTGCGCCAGGAGCGTGTACGACTACCCCTTC
GACTACTGGGGCCAGGGCACACTAGTGACCGTGTCCAGCGCCAGCACCAAGGGCCCCAGCGT
GTTCCCCCTGGCCCCCAGCAGCAAGAGCACCAGCGGCGGCACAGCCGCCCTGGGCTGCCTGG
TGAAGGACTACTTCCCCGAACCGGTGACCGTGTCCTGGAACAGCGGAGCCCTGACCAGCGGCG
TGCACACCTTCCCCGCCGTGCTGCAGAGCAGCGGCCTGTACAGCCTGAGCAGCGTGGTGACCG
TGCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCC
TGCCCCCGAGCTGCTGGGAGGCCCCAGCGTGTTCCTGTTCCCCCCCAAGCCTAAGGACACCCT
GATGATCAGCAGAACCCCCGAGGTGACCTGTGTGGTGGTGGATGTGAGCCACGAGGACCCTGA
GGTGAAGTTCAACTGGTACGTGGACGGCGTGGAGGTGCACAATGCCAAGACCAAGCCCAGGGA
GGAGCAGTACAACAGCACCTACCGGGTGGTGTCCGTGCTGACCGTGCTGCACCAGGATTGGCT
GAACGGCAAGGAGTACAAGTGTAAGGTGTCCAACAAGGCCCTGCCTGCCCCTATCGAGAAAACC
ATCAGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGACATC
GCCGTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAGCAG
GGCAACGTGTTCAGCTGCTCCGTGATGCACGAGGCCCTGCACAATCACTACACCCAGAAGAGCC
TGAGCCTGTCCCCTGGCAAG

SEQ ID 120 - S332121F02 murine variable light chain

DIVLTQSPASLAVSLGQRATISCRASESVSIHGTHLMHWYQQKPGQPPKLLIYAASNLESGVPARFSG
SGSETDFTLNIHPVEEEDAATYFCQQSIEDPRTFGGGTKLEIK

SEQ ID 121 - S332121F02 murine variable light chain (DNA sequence)

GACATCGTCCTGACCCAGAGCCCCGCCAGCCTGGCCGTGAGCCTGGGCCAGAGGGCCACAATC
AGCTGCAGGGCCTCTGAGTCCGTGAGCATCCACGGCACCCACCTGATGCACTGGTATCAGCAG
AAGCCCGGCCAGCCTCCCAAGCTGCTGATCTACGCCGCCAGCAACCTGGAGAGCGGCGTGCCC
GCTAGGTTCAGCGGAAGCGGCAGCGAGACCGACTTCACCCTGAACATCCACCCCGTGGAGGAG
GAAGACGCCGCCACCTACTTCTGCCAGCAGAGCATCGAGGACCCCAGGACCTTCGGCGGGGGC
ACCAAGCTCGAGATTAAGCGT

SEQ ID 122 - S332121F02 chimeric light chain

MGWSCIILFLVATATGVHSDIVLTQSPASLAVSLGQRATISCRASESVSIHGTHLMHWYQQKPGQPPK
LLIYAASNLESGVPARFSGSGSETDFTLNIHPVEEEDAATYFCQQSIEDPRTFGGGTKLEIKRTVAAPS
VFIFPPSDEQLKSGTASVVCLLNNFYPREAKVQWKVDNALQSGNSQESVTEQDSKDSTYSLSSTLTL
SKADYEKHKVYACEVTHQGLSSPVTKSFNRGEC

SEQ ID 123 - S332121F02 chimeric light chain (DNA sequence)

ATGGGCTGGTCCTGCATCATCCTGTTTCTGGTGGCCACCGCCACCGGCGTGCACAGCGACATC
GTCCTGACCCAGAGCCCCGCCAGCCTGGCCGTGAGCCTGGGCCAGAGGGCCACAATCAGCTG
CAGGGCCTCTGAGTCCGTGAGCATCCACGGCACCCACCTGATGCACTGGTATCAGCAGAAGCC
CGGCCAGCCTCCCAAGCTGCTGATCTACGCCGCCAGCAACCTGGAGAGCGGCGTGCCCGCTAG
GTTCAGCGGAAGCGGCAGCGAGACCGACTTCACCCTGAACATCCACCCCGTGGAGGAGGAAGA
CGCCGCCACCTACTTCTGCCAGCAGAGCATCGAGGACCCCAGGACCTTCGGCGGGGGCACCAA
GCTCGAGATTAAGCGTACGGTGGCCGCCCCCAGCGTGTTCATCTTCCCCCCCAGCGATGAGCA
GCTGAAGAGCGGCACCGCCAGCGTGGTGTGTCTGCTGAACAACTTCTACCCCCGGGAGGCCAA
GGTGCAGTGGAAGGTGGACAATGCCCTGCAGAGCGGCAACAGCCAGGAGAGCGTGACCGAGC

AGGACAGCAAGGACTCCACCTACAGCCTGAGCAGCACCCTGACCCTGAGCAAGGCCGACTACG
AGAAGCACAAGGTGTACGCCTGTGAGGTGACCCACCAGGGCCTGTCCAGCCCCGTGACCAAGA
GCTTCAACCGGGGCGAGTGC

SEQ ID 124 - S322110D07 murine variable heavy chain

EVQLQQSGPELVKPGTSVKIPCKTSGYIFTDYSIDWVKQSHGKSLEWIGDIDPNYGDPIYNHKFKGKA
TLTVDRSSSTAYMELRSLTSEDTAVYFCARRATGTDWFAFWGQGTLVTVSS

SEQ ID 125 - S322110D07 murine variable heavy chain (DNA sequence)

GAGGTGCAGCTGCAGCAGAGCGGCCCCGAGCTGGTGAAACCCGGCACCAGCGTGAAGATCCC
CTGCAAGACCTCTGGCTACATCTTCACCGACTACAGCATCGACTGGGTGAAGCAGAGCCACGGC
AAGTCTCTGGAGTGGATTGGGGACATCGACCCCAACTACGGCGACCCCATCTACAACCACAAGT
TCAAGGGCAAGGCCACCCTGACCGTGGACAGGAGCAGCAGCACCGCCTACATGGAACTCAGGA
GCCTGACCAGCGAGGACACCGCCGTGTATTTTTGCGCCAGGAGGGCCACCGGCACTGATTGGT
TCGCCTTCTGGGGCCAGGGCACACTAGTGACCGTGTCCAGC

SEQ ID 126 - S322110D07 chimeric heavy chain

EVQLQQSGPELVKPGTSVKIPCKTSGYIFTDYSIDWVKQSHGKSLEWIGDIDPNYGDPIYNHKFKGKA
TLTVDRSSSTAYMELRSLTSEDTAVYFCARRATGTDWFAFWGQGTLVTVSSASTKGPSVFPLAPSSK
STSGGTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK

SEQ ID 127 - S322110D07 chimeric heavy chain (DNA sequence)

GAGGTGCAGCTGCAGCAGAGCGGCCCCGAGCTGGTGAAACCCGGCACCAGCGTGAAGATCCC
CTGCAAGACCTCTGGCTACATCTTCACCGACTACAGCATCGACTGGGTGAAGCAGAGCCACGGC
AAGTCTCTGGAGTGGATTGGGGACATCGACCCCAACTACGGCGACCCCATCTACAACCACAAGT
TCAAGGGCAAGGCCACCCTGACCGTGGACAGGAGCAGCAGCACCGCCTACATGGAACTCAGGA
GCCTGACCAGCGAGGACACCGCCGTGTATTTTTGCGCCAGGAGGGCCACCGGCACTGATTGGT
TCGCCTTCTGGGGCCAGGGCACACTAGTGACCGTGTCCAGCGCCAGCACCAAGGGCCCCAGCG
TGTTCCCCCTGGCCCCCAGCAGCAAGAGCACCAGCGGCGGCACAGCCGCCCTGGGCTGCCTG
GTGAAGGACTACTTCCCCGAACCGGTGACCGTGTCCTGGAACAGCGGAGCCCTGACCAGCGGC
GTGCACACCTTCCCCGCCGTGCTGCAGAGCAGCGGCCTGTACAGCCTGAGCAGCGTGGTGACC
GTGCCCAGCAGCAGCCTGGGCACCCAGACCTACATCTGTAACGTGAACCACAAGCCCAGCAAC
ACCAAGGTGGACAAGAAGGTGGAGCCCAAGAGCTGTGACAAGACCCACACCTGCCCCCCCTGC
CCTGCCCCCGAGCTGCTGGGAGGCCCCAGCGTGTTCCTGTTCCCCCCCAAGCCTAAGGACACC
CTGATGATCAGCAGAACCCCCGAGGTGACCTGTGTGGTGGTGGATGTGAGCCACGAGGACCCT
GAGGTGAAGTTCAACTGGTACGTGGACGGCGTGGAGGTGCACAATGCCAAGACCAAGCCCAGG
GAGGAGCAGTACAACAGCACCTACCGGGTGGTGTCCGTGCTGACCGTGCTGCACCAGGATTGG
CTGAACGGCAAGGAGTACAAGTGTAAGGTGTCCAACAAGGCCCTGCCTGCCCCTATCGAGAAAA
CCATCAGCAAGGCCAAGGGCCAGCCCAGAGAGCCCCAGGTGTACACCCTGCCCCCTAGCAGAG
ATGAGCTGACCAAGAACCAGGTGTCCCTGACCTGCCTGGTGAAGGGCTTCTACCCCAGCGACAT
CGCCGTGGAGTGGGAGAGCAACGGCCAGCCCGAGAACAACTACAAGACCACCCCCCCTGTGCT
GGACAGCGATGGCAGCTTCTTCCTGTACAGCAAGCTGACCGTGGACAAGAGCAGATGGCAGCA
GGGCAACGTGTTCAGCTGCTCCGTGATGCACGAGGCCCTGCACAATCACTACACCCAGAAGAG
CCTGAGCCTGTCCCCTGGCAAG

SEQ ID 128 - S322110D07 murine variable light chain

DIQMTQSPASLSVSVGETVTITCRASENIYNNLAWYQQKQGKSPQLLVYAATILADGVPSRFSGSGSG
TQYSLKINSLQSGDFGTYYCQHFWGTPLTFGAGTKLELKR

SEQ ID 129 - S322110D07 murine variable light chain (DNA sequence)

GACATCCAGATGACCCAGAGCCCCGCTAGCCTCAGCGTGTCCGTCGGCGAGACCGTGACCATC
ACCTGCAGGGCCAGCGAGAACATCTACAACAACCTGGCCTGGTATCAGCAGAAGCAGGGCAAA
AGCCCCCAGCTGCTGGTGTACGCCGCCACCATTCTGGCCGACGGCGTGCCCAGCAGGTTCTCT
GGAAGCGGCAGCGGCACCCAGTACAGCCTGAAGATCAACAGCCTGCAGAGCGGGGACTTCGG
CACCTACTACTGCCAGCACTTCTGGGGCACTCCCCTGACCTTCGGAGCCGGCACCAAGCTGGA
GCTGAAGCGT

SEQ ID 130 - S322110D07 chimeric light chain

DIQMTQSPASLSVSVGETVTITCRASENIYNNLAWYQQKQGKSPQLLVYAATILADGVPSRFSGSGSG
TQYSLKINSLQSGDFGTYYCQHFWGTPLTFGAGTKLELKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLS
SPVTKSFNRGEC

SEQ ID 131 - S322110D07 chimeric light chain (DNA sequence)

GACATCCAGATGACCCAGAGCCCCGCTAGCCTCAGCGTGTCCGTCGGCGAGACCGTGACCATC
ACCTGCAGGGCCAGCGAGAACATCTACAACAACCTGGCCTGGTATCAGCAGAAGCAGGGCAAA
AGCCCCCAGCTGCTGGTGTACGCCGCCACCATTCTGGCCGACGGCGTGCCCAGCAGGTTCTCT
GGAAGCGGCAGCGGCACCCAGTACAGCCTGAAGATCAACAGCCTGCAGAGCGGGGACTTCGG
CACCTACTACTGCCAGCACTTCTGGGGCACTCCCCTGACCTTCGGAGCCGGCACCAAGCTGGA
GCTGAAGCGTACGGTGGCCGCCCCCAGCGTGTTCATCTTCCCCCCCAGCGATGAGCAGCTGAA
GAGCGGCACCGCCAGCGTGGTGTGTCTGCTGAACAACTTCTACCCCCGGGAGGCCAAGGTGCA
GTGGAAGGTGGACAATGCCCTGCAGAGCGGCAACAGCCAGGAGAGCGTGACCGAGCAGGACA
GCAAGGACTCCACCTACAGCCTGAGCAGCACCCTGACCCTGAGCAAGGCCGACTACGAGAAGC
ACAAGGTGTACGCCTGTGAGGTGACCCACCAGGGCCTGTCCAGCCCCGTGACCAAGAGCTTCA
ACCGGGGCGAGTGC

SEQ ID 132 - S332126E04 murine variable heavy chain

QVQLQQPGAELVKPGASVKLSCKASGYTFTNYWMHWVKQRPGQGLEWIGIIHPNSGSTNYNEKFKS
KATLTVDKSSSTAYMQLSSLTSEDSAVYYCARGIYDYPFAYWGQGTLVTVSS

SEQ ID 133 - S332126E04 murine variable heavy chain (DNA sequence)

CAGGTGCAGCTCCAGCAGCCCGGAGCCGAACTGGTGAAGCCCGGAGCCAGCGTCAAACTGTCC
TGCAAGGCCAGCGGCTACACCTTCACCAACTACTGGATGCACTGGGTGAAGCAGAGGCCCGGC
CAGGGCCTGGAGTGGATCGGCATCATCCACCCCAACAGCGGGAGCACCAACTACAACGAGAAG
TTCAAGAGCAAGGCCACCCTGACCGTGGACAAGAGCAGCAGCACTGCCTACATGCAGCTGAGC
AGCCTGACCAGCGAGGACAGCGCTGTGTACTACTGCGCCAGGGGCATCTACGACTACCCCTTC
GCCTATTGGGGCCAGGGCACACTAGTGACCGTGTCCAGC

SEQ ID 134 - S332126E04 Chimeric heavy chain

QVQLQQPGAELVKPGASVKLSCKASGYTFTNYWMHWVKQRPGQGLEWIGIIHPNSGSTNYNEKFKS
KATLTVDKSSSTAYMQLSSLTSEDSAVYYCARGIYDYPFAYWGQGTLVTVSSASTKGPSVFPLAPSS
KSTSGGTAALGCLVKDYFPEPVTVSWNSGALTSGVHTFPAVLQSSGLYSLSSVVTVPSSSLGTQTYI
CNVNHKPSNTKVDKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPAPI

EKTISKAKGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK

SEQ ID 135 - S332126E04 Chimeric heavy chain (DNA sequence)

CAGGTGCAGCTCCAGCAGCCCGGAGCCGAACTGGTGAAGCCCGGAGCCAGCGTCAAACTGTCC
TGCAAGGCCAGCGGCTACACCTTCACCAACTACTGGATGCACTGGGTGAAGCAGAGGCCCGGC
CAGGGCCTGGAGTGGATCGGCATCATCCACCCCAACAGCGGGAGCACCAACTACAACGAGAAG
TTCAAGAGCAAGGCCACCCTGACCGTGGACAAGAGCAGCAGCACTGCCTACATGCAGCTGAGC
AGCCTGACCAGCGAGGACAGCGCTGTGTACTACTGCGCCAGGGGCATCTACGACTACCCCTTC
GCCTATTGGGGCCAGGGCACACTAGTGACCGTGTCCAGCGCCAGCACCAAGGGCCCCAGCGTG
TTCCCCCTGGCCCCCAGCAGCAAGAGCACCAGCGGCGGCACAGCCGCCCTGGGCTGCCTGGT
GAAGGACTACTTCCCCGAACCGGTGACCGTGTCCTGGAACAGCGGAGCCCTGACCAGCGGCGT
GCACACCTTCCCCGCCGTGCTGCAGAGCAGCGGCCTGTACAGCCTGAGCAGCGTGGTGACCGT
GCCCAGCAGCAGCCTGGGCACCCAGACCTACATCTGTAACGTGAACCACAAGCCCAGCAACAC
CAAGGTGGACAAGAAGGTGGAGCCCAAGAGCTGTGACAAGACCCACACCTGCCCCCCCTGCCC
TGCCCCCGAGCTGCTGGGAGGCCCCAGCGTGTTCCTGTTCCCCCCCAAGCCTAAGGACACCCT
GATGATCAGCAGAACCCCCGAGGTGACCTGTGTGGTGGTGGATGTGAGCCACGAGGACCCTGA
GGTGAAGTTCAACTGGTACGTGGACGGCGTGGAGGTGCACAATGCCAAGACCAAGCCCAGGGA
GGAGCAGTACAACAGCACCTACCGGGTGGTGTCCGTGCTGACCGTGCTGCACCAGGATTGGCT
GAACGGCAAGGAGTACAAGTGTAAGGTGTCCAACAAGGCCCTGCCTGCCCCTATCGAGAAAACC
ATCAGCAAGGCCAAGGGCCAGCCCAGAGAGCCCCAGGTGTACACCCTGCCCCCTAGCAGAGAT
GAGCTGACCAAGAACCAGGTGTCCCTGACCTGCCTGGTGAAGGGCTTCTACCCCAGCGACATC
GCCGTGGAGTGGGAGAGCAACGGCCAGCCCGAGAACAACTACAAGACCACCCCCCCTGTGCTG
GACAGCGATGGCAGCTTCTTCCTGTACAGCAAGCTGACCGTGGACAAGAGCAGATGGCAGCAG
GGCAACGTGTTCAGCTGCTCCGTGATGCACGAGGCCCTGCACAATCACTACACCCAGAAGAGCC
TGAGCCTGTCCCCTGGCAAG

SEQ ID 136 - S332126E04 murine variable light chain

DIVLTQSPASLAVSLGQRATISCRASESVSIHGTHLMHWYQQKPGQPPKLLIYAASNLESGVPARFSG
SGSETDFTLNIHPVEEEDAATYFCQQSIEDPYTFGGGTKLEIKR

SEQ ID 137 - S332126E04 murine variable light chain (DNA sequence)

GACATCGTGCTGACCCAGTCTCCCGCTAGCCTGGCCGTGTCTCTGGGCCAGAGGGCCACAATC
AGCTGCAGGGCCAGCGAGAGCGTCAGCATTCACGGCACCCACCTGATGCACTGGTACCAGCAG
AAGCCCGGCCAGCCTCCCAAGCTCCTGATCTACGCCGCCAGCAACCTGGAAAGCGGAGTGCCC
GCCAGGTTCAGCGGCAGCGGCTCCGAGACCGACTTCACCCTGAACATCCACCCCGTGGAGGAG
GAGGACGCCGCCACCTACTTCTGCCAGCAGAGCATCGAGGACCCCTACACCTTCGGCGGCGGC
ACCAAGCTGGAGATCAAGCGTSEQ ID 138 - S332126E04 Chimeric light chain

DIVLTQSPASLAVSLGQRATISCRASESVSIHGTHLMHWYQQKPGQPPKLLIYAASNLESGVPARFSG
SGSETDFTLNIHPVEEEDAATYFCQQSIEDPYTFGGGTKLEIKRTVAAPSVFIFPPSDEQLKSGTASVV
CLLNNFYPREAKVQWKVDNALQSGNSQESVTEQDSKDSTYSLSSTLTLSKADYEKHKVYACEVTHQ
GLSSPVTKSFNRGEC

SEQ ID 139 - S332126E04 Chimeric light chain (DNA sequence)

GACATCGTGCTGACCCAGTCTCCCGCTAGCCTGGCCGTGTCTCTGGGCCAGAGGGCCACAATC
AGCTGCAGGGCCAGCGAGAGCGTCAGCATTCACGGCACCCACCTGATGCACTGGTACCAGCAG
AAGCCCGGCCAGCCTCCCAAGCTCCTGATCTACGCCGCCAGCAACCTGGAAAGCGGAGTGCCC
GCCAGGTTCAGCGGCAGCGGCTCCGAGACCGACTTCACCCTGAACATCCACCCCGTGGAGGAG

GAGGACGCCGCCACCTACTTCTGCCAGCAGAGCATCGAGGACCCCTACACCTTCGGCGGCGGC
ACCAAGCTGGAGATCAAGCGTACGGTGGCCGCCCCCAGCGTGTTCATCTTCCCCCCCAGCGAT
GAGCAGCTGAAGAGCGGCACCGCCAGCGTGGTGTGTCTGCTGAACAACTTCTACCCCCGGGAG
GCCAAGGTGCAGTGGAAGGTGGACAATGCCCTGCAGAGCGGCAACAGCCAGGAGAGCGTGAC
CGAGCAGGACAGCAAGGACTCCACCTACAGCCTGAGCAGCACCCTGACCCTGAGCAAGGCCGA
CTACGAGAAGCACAAGGTGTACGCCTGTGAGGTGACCCACCAGGGCCTGTCCAGCCCCGTGAC
CAAGAGCTTCAACCGGGGCGAGTGC

SEQ ID 140 - S336105A07 murine variable heavy chain

EVKLLQSGGGLVQPGGSLKLSCAASGIDFSRYWMSWVRRAPGKGLEWIGEINPDRSTINYAPSLKDK
FIISRDNAKNTLYLQMSKVRSEDTALYYCAVFYYDYEGAMDYWGQGTSVTVSS

SEQ ID 141 - S336105A07 murine variable heavy chain (DNA sequence)

GAGGTGAAGCTTCTCCAGTCTGGAGGTGGCCTGGTGCAGCCTGGAGGATCCCTGAAACTCTCCT
GTGCAGCCTCAGGAATCGATTTTAGTAGATACTGGATGAGTTGGGTTCGGCGGGCTCCAGGGAA
AGGACTAGAATGGATTGGAGAAATTAATCCAGATAGGAGTACAATCAACTATGCACCATCTCTAA
AGGATAAATTCATCATCTCCAGAGACAACGCCAAAAATACGCTGTACCTGCAAATGAGCAAAGTG
AGATCTGAGGACACAGCCCTTTATTACTGTGCAGTTTTCTACTATGATTACGAGGGTGCTATGGA
CTACTGGGGTCAAGGAACCTCAGTCACCGTCTCCTCA

SEQ ID 142 - S336105A07 Chimeric heavy chain

EVKLLQSGGGLVQPGGSLKLSCAASGIDFSRYWMSWVRRAPGKGLEWIGEINPDRSTINYAPSLKDK
FIISRDNAKNTLYLQMSKVRSEDTALYYCAVFYYDYEGAMDYWGQGTSVTVSSAKTTAPSVFPLAPS
SKSTSGGTAALGCLVKDYFPEPVTVSWNSGALTSGVHTFPAVLQSSGLYSLSSVVTVPSSSLGTQTYI
CNVNHKPSNTKVDKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK

SEQ ID 143 - S336105A07 Chimeric heavy chain (DNA sequence)

GAGGTGAAGCTTCTCCAGTCTGGAGGTGGCCTGGTGCAGCCTGGAGGATCCCTGAAACTCTCCT
GTGCAGCCTCAGGAATCGATTTTAGTAGATACTGGATGAGTTGGGTTCGGCGGGCTCCAGGGAA
AGGACTAGAATGGATTGGAGAAATTAATCCAGATAGGAGTACAATCAACTATGCACCATCTCTAA
AGGATAAATTCATCATCTCCAGAGACAACGCCAAAAATACGCTGTACCTGCAAATGAGCAAAGTG
AGATCTGAGGACACAGCCCTTTATTACTGTGCAGTTTTCTACTATGATTACGAGGGTGCTATGGA
CTACTGGGGTCAAGGAACCTCAGTCACCGTCTCCTCAGCCAAAACAACAGCCCCCAGCGTGTTC
CCCCTGGCCCCCAGCAGCAAGAGCACCAGCGGCGGCACAGCCGCCCTGGGCTGCCTGGTGAA
GGACTACTTCCCCGAACCGGTGACCGTGTCCTGGAACAGCGGAGCCCTGACCAGCGGCGTGCA
CACCTTCCCCGCCGTGCTGCAGAGCAGCGGCCTGTACAGCCTGAGCAGCGTGGTGACCGTGCC
CAGCAGCAGCCTGGGCACCCAGACCTACATCTGTAACGTGAACCACAAGCCCAGCAACACCAAG
GTGGACAAGAAGGTGGAGCCCAAGAGCTGTGACAAGACCCACACCTGCCCCCCCTGCCCTGCC
CCCGAGCTGCTGGGAGGCCCCAGCGTGTTCCTGTTCCCCCCCAAGCCTAAGGACACCCTGATG
ATCAGCAGAACCCCCGAGGTGACCTGTGTGGTGGTGGATGTGAGCCACGAGGACCCTGAGGTG
AAGTTCAACTGGTACGTGGACGGCGTGGAGGTGCACAATGCCAAGACCAAGCCCAGGGAGGAG
CAGTACAACAGCACCTACCGGGTGGTGTCCGTGCTGACCGTGCTGCACCAGGATTGGCTGAAC
GGCAAGGAGTACAAGTGTAAGGTGTCCAACAAGGCCCTGCCTGCCCCTATCGAGAAAACCATCA
GCAAGGCCAAGGGCCAGCCCAGAGAGCCCCAGGTGTACACCCTGCCCCCTAGCAGAGATGAGC
TGACCAAGAACCAGGTGTCCCTGACCTGCCTGGTGAAGGGCTTCTACCCCAGCGACATCGCCGT

GGAGTGGGAGAGCAACGGCCAGCCCGAGAACAACTACAAGACCACCCCCCCTGTGCTGGACAG
CGATGGCAGCTTCTTCCTGTACAGCAAGCTGACCGTGGACAAGAGCAGATGGCAGCAGGGCAA
CGTGTTCAGCTGCTCCGTGATGCACGAGGCCCTGCACAATCACTACACCCAGAAGAGCCTGAGC
CTGTCCCCTGGCAAG

SEQ ID 144 - S336105A07 murine varaible light chain

DIVMTQSQKFMSTSVGDRVSVTCKASQNVDTNVAWYQQKPGQSPKALIYSASYRFSGVPDRFTGSG
SGTDFTLTISNVQSEDLAEYFCQQYNSFPFTFGSGTKLEIKR

SEQ ID 145 - S336105A07 murine variable light chain (DNA sequence)

GACATTGTGATGACCCAGTCTCAAAAATTCATGTCCACATCAGTAGGAGACAGGGTCAGCGTCAC
CTGCAAGGCCAGTCAGAATGTGGATACTAATGTAGCCTGGTATCAACAAAAACCAGGGCAATCTC
CTAAAGCACTGATTTACTCGGCATCCTACCGGTTCAGTGGAGTCCCTGATCGCTTCACAGGCAGT
GGATCTGGGACAGATTTCACTCTCACCATCAGCAATGTGCAGTCTGAAGACTTGGCAGAGTATTT
CTGTCAGCAATATAACAGCTTTCCATTCACGTTCGGCTCGGGGACAAAGTTGGAAATAAAACGT

SEQ ID 146 - S336105A07 chimeric light chain

DIVMTQSQKFMSTSVGDRVSVTCKASQNVDTNVAWYQQKPGQSPKALIYSASYRFSGVPDRFTGSG
SGTDFTLTISNVQSEDLAEYFCQQYNSFPFTFGSGTKLEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQGL
SSPVTKSFNRGEC

SEQ ID 147 - S336105A07 chimeric light chain (DNA sequence)

GACATTGTGATGACCCAGTCTCAAAAATTCATGTCCACATCAGTAGGAGACAGGGTCAGCGTCAC
CTGCAAGGCCAGTCAGAATGTGGATACTAATGTAGCCTGGTATCAACAAAAACCAGGGCAATCTC
CTAAAGCACTGATTTACTCGGCATCCTACCGGTTCAGTGGAGTCCCTGATCGCTTCACAGGCAGT
GGATCTGGGACAGATTTCACTCTCACCATCAGCAATGTGCAGTCTGAAGACTTGGCAGAGTATTT
CTGTCAGCAATATAACAGCTTTCCATTCACGTTCGGCTCGGGGACAAAGTTGGAAATAAAACGTA
CGGTGGCCGCCCCCAGCGTGTTCATCTTCCCCCCCAGCGATGAGCAGCTGAAGAGCGGCACCG
CCAGCGTGGTGTGTCTGCTGAACAACTTCTACCCCCGGGAGGCCAAGGTGCAGTGGAAGGTGG
ACAATGCCCTGCAGAGCGGCAACAGCCAGGAGAGCGTGACCGAGCAGGACAGCAAGGACTCCA
CCTACAGCCTGAGCAGCACCCTGACCCTGAGCAAGGCCGACTACGAGAAGCACAAGGTGTACG
CCTGTGAGGTGACCCACCAGGGCCTGTCCAGCCCCGTGACCAAGAGCTTCAACCGGGGCGAGT
GC

SEQ ID 148 - S335115G01 murine variable heavy chain

PVQLQQPGTELVRPGTSVKLSCKASGYTFTSYWMHWVKQRPGQGLEWIGVIDPSDSYTNYNQKFK
GKATLTVDTSSSTAYMQLSSLTSEDSAVYYCARQVFDYPMDYWGQGTSVTVSS

SEQID 149 - S335115G01 murine variable heavy chain (DNA sequence)

CCGGTCCAACTGCAGCAGCCTGGGACTGAGCTGGTGAGGCCTGGGACTTCAGTGAAGTTGTCC
TGCAAGGCTTCTGGCTACACCTTCACCAGCTACTGGATGCACTGGGTAAAGCAGAGGCCTGGAC
AAGGCCTTGAGTGGATCGGAGTGATTGATCCTTCTGATAGTTATACTAACTACAATCAAAAGTTCA
AGGGCAAGGCCACATTGACTGTAGACACATCCTCCAGCACAGCCTACATGCAGCTCAGCAGCCT
GACATCTGAGGACTCTGCGGTCTATTACTGTGCAAGACAGGTGTTTGACTATCCTATGGACTACT
GGGGTCAAGGAACCTCAGTCACCGTCTCCTCA

SEQ ID 150 - S335115G01 Chimeric heavy chain

PVQLQQPGTELVRPGTSVKLSCKASGYTFTSHWMHWVKQRPGQGLEWIGVIDPSDSYTNYNQKFK
GKATLTVDTSSSTAYMQLSSLTSEDSAVYYCARQVFDYPMDYWGQGTLVTVSSASTKGPSVFPLAP
SSKSTSGGTAALGCLVKDYFPEPVTVSWNSGALTSGVHTFPAVLQSSGLYSLSSVVTVPSSSLGTQT

YICNVNHKPSNTKVDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNGKEYKCKVSNKALPAP
IEKTISKAKGQPREPQVYTLPPSRDELTKNQVSLTCLVKGFYPSDIAVEWESNGQPENNYKTTPPVLD
SDGSFFLYSKLTVDKSRWQQGNVFSCSVMHEALHNHYTQKSLSLSPGK

SEQ ID 151 - S335115G01 Chimeric heavy chain (DNA sequence)

CCGGTCCAACTGCAGCAGCCTGGGACTGAGCTGGTGAGGCCTGGGACTTCAGTGAAGTTGTCC TGCAAGGCTTCTGGCTACACCTTCACCAGCCACTGGATGCACTGGGTAAAGCAGAGGCCTGGAC AAGGCCTTGAGTGGATCGGAGTGATTGATCCTTCTGATAGTTATACTAACTACAATCAAAAGTTCA AGGGCAAGGCCACATTGACTGTAGACACATCCTCCAGCACAGCCTACATGCAGCTCAGCAGCCT GACATCTGAGGACTCTGCGGTCTATTACTGTGCAAGACAGGTGTTTGACTATCCTATGGACTACT GGGGTCAAGGAACACTAGTGACCGTGTCCAGCGCCAGCACCAAGGGCCCCAGCGTGTTCCCCC TGGCCCCCAGCAGCAAGAGCACCAGCGGCGGCACAGCCGCCCTGGGCTGCCTGGTGAAGGAC TACTTCCCCGAACCGGTGACCGTGTCCTGGAACAGCGGAGCCCTGACCAGCGGCGTGCACACC TTCCCCGCCGTGCTGCAGAGCAGCGGCCTGTACAGCCTGAGCAGCGTGGTGACCGTGCCCAGC AGCAGCCTGGGCACCCAGACCTACATCTGTAACGTGAACCACAAGCCCAGCAACACCAAGGTG GACAAGAAGGTGGAGCCCAAGAGCTGTGACAAGACCCACACCTGCCCCCCCTGCCCTGCCCCC GAGCTGCTGGGAGGCCCCAGCGTGTTCCTGTTCCCCCCCAAGCCTAAGGACACCCTGATGATC AGCAGAACCCCCGAGGTGACCTGTGTGGTGGTGGATGTGAGCCACGAGGACCCTGAGGTGAAG TTCAACTGGTACGTGGACGGCGTGGAGGTGCACAATGCCAAGACCAAGCCCAGGGAGGAGCAG TACAACAGCACCTACCGGGTGGTGTCCGTGCTGACCGTGCTGCACCAGGATTGGCTGAACGGC AAGGAGTACAAGTGTAAGGTGTCCAACAAGGCCCTGCCTGCCCCTATCGAGAAAACCATCAGCA AGGCCAAGGGCCAGCCCAGAGAGCCCCAGGTGTACACCCTGCCCCCTAGCAGAGATGAGCTGA CCAAGAACCAGGTGTCCCTGACCTGCCTGGTGAAGGGCTTCTACCCCAGCGACATCGCCGTGG AGTGGGAGAGCAACGGCCAGCCCGAGAACAACTACAAGACCACCCCCCCTGTGCTGGACAGCG ATGGCAGCTTCTTCCTGTACAGCAAGCTGACCGTGGACAAGAGCAGATGGCAGCAGGGCAACG TGTTCAGCTGCTCCGTGATGCACGAGGCCCTGCACAATCACTACACCCAGAAGAGCCTGAGCCT GTCCCCTGGCAAG

SEQ ID 152 - S335115G01 murine variable light chain

DIVLTQSPASLAVSLGQRATISCRASESVSIHGTHLMHWYQQKPGQPPKLLIYAASNLESGVPARFSG SGSETDFTLNIHPVEEEDAATYFCQQSIEDPWTFGGGTKLEIKR

SEQ ID 153 - S335115G01 murine variable light chain (DNA sequence)

GACATTGTGCTGACCCAATCTCCAGCTTCTTTGGCTGTGTCTCTAGGGCAGAGGGCCACCATCT CCTGCAGAGCCAGTGAAAGTGTCAGTATTCATGGTACTCATTTAATGCACTGGTACCAACAGAAA CCAGGACAGCCACCCAAACTCCTCATCTATGCTGCATCCAACCTAGAATCTGGAGTCCCTGCCA GGTTCAGTGGCAGTGGGTCTGAGACAGACTTCACCCTCAACATCCATCCTGTGGAGGAGGAGGA TGCTGCAACCTATTTCTGTCAGCAAAGTATTGAGGATCCGTGGACGTTCGGTGGAGGCACCAAG CTGGAAATCAAACGT

SEQ ID 154 - S335115G01 Chimeric light chain

DIVLTQSPASLAVSLGQRATISCRASESVSIHGTHLMHWYQQKPGQPPKLLIYAASNLESGVPARFSG SGSETDFTLNIHPVEEEDAATYFCQQSIEDPWTFGGGTKLEINRTVAAPSVFIFPPSDEQLKSGTASVV CLLNNFYPREAKVQWKVDNALQSGNSQESVTEQDSKDSTYSLSSTLTLSKADYEKHKVYACEVTHQ GLSSPVTKSFNRGEC

SEQ ID 155 - S335115G01 Chimeric light chain (DNA sequence)

GACATTGTGCTGACCCAATCTCCAGCTTCTTTGGCTGTGTCTCTAGGGCAGAGGGCCACCATCT
CCTGCAGAGCCAGTGAAAGTGTCAGTATTCATGGTACTCATTTAATGCACTGGTACCAACAGAAA
CCAGGACAGCCACCCAAACTCCTCATCTATGCTGCATCCAACCTAGAATCTGGAGTCCCTGCCA
GGTTCAGTGGCAGTGGGTCTGAGACAGACTTCACCCTCAACATCCATCCTGTGGAGGAGGAGGA
TGCTGCAACCTATTTCTGTCAGCAAAGTATTGAGGATCCGTGGACGTTCGGTGGAGGCACCAAG
CTGGAAATCAATCGTACGGTGGCCGCCCCCAGCGTGTTCATCTTCCCCCCCAGCGATGAGCAGC
TGAAGAGCGGCACCGCCAGCGTGGTGTGTCTGCTGAACAACTTCTACCCCCGGGAGGCCAAGG
TGCAGTGGAAGGTGGACAATGCCCTGCAGAGCGGCAACAGCCAGGAGAGCGTGACCGAGCAG
GACAGCAAGGACTCCACCTACAGCCTGAGCAGCACCCTGACCCTGAGCAAGGCCGACTACGAG
AAGCACAAGGTGTACGCCTGTGAGGTGACCCACCAGGGCCTGTCCAGCCCCGTGACCAAGAGC
TTCAACCGGGGCGAGTGC

SEQ ID 156 - S335122F05 murine variable heavy chain

QVQLQQSGAELVRPGASVTLSCKASGYTFTDYEMHWVKQTPVHGLEWIGAIDPETGGTAYNQKFKG
KAILTADKSSSTAYMELRSLTSEDSAVYYCTRSIYDYYFDYWGQGTTLTVSS

SEQ ID 157 - S335122F05 murine variable heavy chain (DNA sequence)

CAGGTTCAACTGCAGCAGTCTGGGGCTGAGCTGGTGAGGCCTGGGGCTTCAGTGACGCTGTCC
TGCAAGGCTTCGGGCTACACATTTACTGACTATGAAATGCACTGGGTGAAGCAGACACCTGTGC
ATGGCCTGGAATGGATTGGAGCTATTGATCCTGAAACTGGTGGTACTGCCTACAATCAGAAGTTC
AAGGGCAAGGCCATACTGACTGCAGACAAATCCTCCAGCACAGCCTACATGGAGCTCCGCAGCC
TGACATCTGAGGACTCTGCCGTCTATTACTGTACAAGATCGATTTATGATTACTACTTTGACTACT
GGGGCCAAGGCACCACTCTCACAGTCTCCTCA

SEQ ID 158 - S335122F05 Chimeric heavy chain

QVQLQQSGAELVRPGASVTLSCKASGYTFTDYEMHWVKQTPVHGLEWIGAIDPETGGTAYNQKFKG
KAILTADKSSSTAYMELRSLTSEDSAVYYCTRSIYDYYFDYWGQGTTLTVSSAKTTPPSVFPLAPSSK
STSGGTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPENNYKTTPPVLDS
DGSFFLYSKLTVDKSRWQQGNVFSCSVMHEALHNHYTQKSLSLSPGK

SEQ ID 159 - S335122F05 Chimeric heavy chain (DNA sequence)

CAGGTTCAACTGCAGCAGTCTGGGGCTGAGCTGGTGAGGCCTGGGGCTTCAGTGACGCTGTCC TGCAAGGCTTCGGGCTACACATTTACTGACTATGAAATGCACTGGGTGAAGCAGACACCTGTGC ATGGCCTGGAATGGATTGGAGCTATTGATCCTGAAACTGGTGGTACTGCCTACAATCAGAAGTTC AAGGGCAAGGCCATACTGACTGCAGACAAATCCTCCAGCACAGCCTACATGGAGCTCCGCAGCC TGACATCTGAGGACTCTGCCGTCTATTACTGTACAAGATCGATTTATGATTACTACTTTGACTACT GGGGCCAAGGCACCACTCTCACAGTCTCCTCAGCCAAAACGACACCCCCCAGCGTGTTCCCCCT GGCCCCCAGCAGCAAGAGCACCAGCGGCGGCACAGCCGCCCTGGGCTGCCTGGTGAAGGACT ACTTCCCCGAACCGGTGACCGTGTCCTGGAACAGCGGAGCCCTGACCAGCGGCGTGCACACCT TCCCCGCCGTGCTGCAGAGCAGCGGCCTGTACAGCCTGAGCAGCGTGGTGACCGTGCCCAGCA GCAGCCTGGGCACCCAGACCTACATCTGTAACGTGAACCACAAGCCCAGCAACACCAAGGTGG ACAAGAAGGTGGAGCCCAAGAGCTGTGACAAGACCCACACCTGCCCCCCCTGCCCTGCCCCCG AGCTGCTGGGAGGCCCCAGCGTGTTCCTGTTCCCCCCCAAGCCTAAGGACACCCTGATGATCA GCAGAACCCCCGAGGTGACCTGTGTGGTGGTGGATGTGAGCCACGAGGACCCTGAGGTGAAGT TCAACTGGTACGTGGACGGCGTGGAGGTGCACAATGCCAAGACCAAGCCCAGGGAGGAGCAGT

ACAACAGCACCTACCGGGTGGTGTCCGTGCTGACCGTGCTGCACCAGGATTGGCTGAACGGCA AGGAGTACAAGTGTAAGGTGTCCAACAAGGCCCTGCCTGCCCCTATCGAGAAAACCATCAGCAA GGCCAAGGGCCAGCCCAGAGAGCCCCAGGTGTACACCCTGCCCCCTAGCAGAGATGAGCTGAC CAAGAACCAGGTGTCCCTGACCTGCCTGGTGAAGGGCTTCTACCCCAGCGACATCGCCGTGGA GTGGGAGAGCAACGGCCAGCCCGAGAACAACTACAAGACCACCCCCCCTGTGCTGGACAGCGA TGGCAGCTTCTTCCTGTACAGCAAGCTGACCGTGGACAAGAGCAGATGGCAGCAGGGCAACGT GTTCAGCTGCTCCGTGATGCACGAGGCCCTGCACAATCACTACACCCAGAAGAGCCTGAGCCTG TCCCCTGGCAAG

SEQ ID 160 - S335122F05 murine variable light chain

DIVLTQSPASLAVSLGQRATISCRASESVSIHGTHLMHWYQQKPGQPPKLLIYAASNLESGVPARFSG GGSETDFTLNIHPVEEEDGATYFCQQSIEYPRTFGGGTKLEINR

SEQ ID 161 - S335122F05 murine variable light chain (DNA sequence)

GACATTGTGCTGACCCAATCTCCAGCTTCTTTGGCTGTGTCTCTAGGGCAGAGGGCCACCATCT CCTGCAGAGCCAGTGAAAGTGTCAGTATTCATGGTACTCATTTAATGCACTGGTACCAACAGAAA CCAGGACAGCCACCCAAACTCCTCATCTATGCTGCATCCAACCTAGAATCTGGAGTCCCTGCCA GGTTCAGTGGCGGTGGGTCTGAGACAGACTTCACCCTCAACATCCATCCTGTGGAGGAGGAGG ATGGTGCAACCTATTTCTGTCAGCAAAGTATTGAGTATCCTCGGACGTTCGGTGGAGGCACCAA GCTGGAAATCAATCGT

SEQ ID 162 - S335122F05 Chimeric light chain

DIVLTQSPASLAVSLGQRATISCRASESVSIHGTHLMHWYQQKPGQPPKLLIYAASNLESGVPARFSG GGSETDFTLNIHPVEEEDGATYFCQQSIEYPRTFGGGTKLEINRTVAAPSVFIFPPSDEQLKSGTASVV CLLNNFYPREAKVQWKVDNALQSGNSQESVTEQDSKDSTYSLSSTLTLSKADYEKHKVYACEVTHQ GLSSPVTKSFNRGEC

SEQ ID 163 - S335122F05 Chimeric light chain (DNA sequence)

GACATTGTGCTGACCCAATCTCCAGCTTCTTTGGCTGTGTCTCTAGGGCAGAGGGCCACCATCT
CCTGCAGAGCCAGTGAAAGTGTCAGTATTCATGGTACTCATTTAATGCACTGGTACCAACAGAAA
CCAGGACAGCCACCCAAACTCCTCATCTATGCTGCATCCAACCTAGAATCTGGAGTCCCTGCCA
GGTTCAGTGGCGGTGGGTCTGAGACAGACTTCACCCTCAACATCCATCCTGTGGAGGAGGAGG
ATGGTGCAACCTATTTCTGTCAGCAAAGTATTGAGTATCCTCGGACGTTCGGTGGAGGCACCAA
GCTGGAAATCAATCGTACGGTGGCCGCCCCCAGCGTGTTCATCTTCCCCCCCAGCGATGAGCA
GCTGAAGAGCGGCACCGCCAGCGTGGTGTGTCTGCTGAACAACTTCTACCCCCGGGAGGCCAA
GGTGCAGTGGAAGGTGGACAATGCCCTGCAGAGCGGCAACAGCCAGGAGAGCGTGACCGAGC
AGGACAGCAAGGACTCCACCTACAGCCTGAGCAGCACCCTGACCCTGAGCAAGGCCGACTACG
AGAAGCACAAGGTGTACGCCTGTGAGGTGACCCACCAGGGCCTGTCCAGCCCCGTGACCAAGA
GCTTCAACCGGGGCGAGTGC

SEQ.I.D.NO: 164 - S332121F02 CDRH1
DYYNM
SEQ.I.D.NO: 165 - S332121F02 CDRH2
VINPYNGGTDYNQKFG
SEQ.I.D.NO: 166 - S332121F02 CDRH3
SVYDYPFDY
SEQ.I.D.NO: 167 - S332121F02 CDRL1
RASESVSIHGTHLMH
SEQ.I.D.NO: 168 - S332121F02 CDRL2
AASNLES
SEQ.I.D.NO: 169 - S332121F02 CDRL3
QQSIEDPRT
SEQ.I.D.NO: 170 - S322110D07 CDRH1
DYSID
SEQ.I.D.NO: 171 - S322110D07 CDRH2
DIDPNYGDPIYNHKFKG
SEQ.I.D.NO: 172 - S322110D07 CDRH3
RATGTDWFAF
SEQ.I.D.NO: 173 - S322110D07CDRL1
RASENIYNNLA
SEQ.I.D.NO: 174 - S322110D07 CDRL2
AATILAD
SEQ.I.D.NO: 175 - S322110D07 CDRL3
QHFWGTPLT
SEQ.I.D.NO: 176 - S332126E04CDRH1
NYWMH
SEQ.I.D.NO: 177 - S332126E04 CDRH2
IIHPNSGSTNYNEKFKS
SEQ.I.D.NO: 178 - S332126E04 CDRH3
GIYDYPFAY
SEQ.I.D.NO: 179 - S332126E04 CDRL1
RASESVSIHGTHLMH
SEQ.I.D.NO: 180 - S332126E04 CDRL2
AASNLES
SEQ.I.D.NO: 181 - S332126E04 CDRL3
QQSIEDPYT
SEQ.I.D.NO: 182 - S336105A07 CDRH1
RYWMS
SEQ.I.D.NO: 183 - S336105A07 CDRH2
EINPDRSTINYAPSLKD

SEQ.I.D.NO: 184 - S336105A07 CDRH3
FYYDYEGAMDY
SEQ.I.D.NO: 185 - S336105A07 CDRL1
KASQNVDTNVA
SEQ.I.D.NO: 186 - S336105A07 CDRL2
SASYRFS
SEQ.I.D.NO: 187 - S336105A07 CDRL3
QQYNSFPFT
SEQ.I.D.NO: 188 - S335115G01 CDRH1
SYWMH
SEQ.I.D.NO: 189 - S335115G01 CDRH2
VIDPSDSYTNYNQKFKG
SEQ.I.D.NO: 190 - S335115G01 CDRH3
QVFDYPMDY
SEQ.I.D.NO: 191 - S335115G01 CDRL1
RASESVSIHGTHLMH
SEQ.I.D.NO: 192 - S335115G01 CDRL2
AASNLES
SEQ.I.D.NO: 193 - S335115G01 CDRL3
QQSIEDPWT
SEQ.I.D.NO: 194 - S335122F05 CDRH1
DYEMH
SEQ.I.D.NO: 195 - S335122F05 CDRH2
AIDPETGGTAYNQKFKG
SEQ.I.D.NO: 196 - S335122F05 CDRH3
SIYDYYFDY
SEQ.I.D.NO: 197 - S335122F05 CDRL1
RASESVSIHGTHLMH
SEQ.I.D.NO: 198 - S335122F05 CDRL2
AASNLES
SEQ.I.D.NO: 199 - S335122F05 CDRL3
QQSIEYPRT

## Claims

**1.** An anti B-Cell Maturation Antigen (CD269) antibody comprising a heavy chain variable region of SEQ. ID. NO:23 and a light chain variable region of SEQ. ID. NO:31.

**2.** An anti B-Cell Maturation Antigen (CD269) antibody according to claim 1 which comprises a heavy chain of SEQ. ID. NO:55 and a light chain of SEQ. ID. NO:63.

**3.** An anti B-Cell Maturation Antigen (CD269) antibody according to any preceding claim wherein the antibody is a monoclonal antibody.

**4.** An anti- B-cell maturation antigen (CD269) antibody according to any preceding claim wherein the antibody has enhanced binding to FcγRIIIA or has enhanced FcyRIIIA mediated effector function.

**5.** An anti- B-cell maturation antigen (CD269) antibody according to claim 4 wherein the antibody is defucosylated.

**6.** Nucleic acid sequences encoding both of the heavy chain variable and light chain variable sequences of the antibody of any preceding claim.

**7.** Nucleic acid sequences comprising SEQ ID NO 56 encoding the heavy chain; and SEQ ID NO: 64 encoding the light chain of the antibody of any preceding claim.

**8.** An expression vector comprising the nucleic acid sequences according to claim 6 or claim 7.

9. A recombinant transformed or transfected host cell comprising the nucleic acid sequences according to claim 6 or claim 7, or the expression vector according to claim 8 wherein the host cell comprises both heavy and light chain encoding nucleic acid sequences.

10. A method for the production of an antibody, which method comprises culturing the host cell according to claim 9 under conditions suitable for expression of said nucleic acid sequences or vector(s), whereby the antibody is produced.

11. An antibody as produced by the method of claim 10.

12. An immunoconjugate comprising the antibody of any one of claims 1 to 5 or claim 11 and a cytotoxic agent.

13. An immunoconjugate according to claim 12 wherein the cytotoxic agent is monomethyl auristatin E (MMAE) or monomethyl auristatin F (MMAF).

14. An immunoconjugate of claim 13 wherein the cytotoxic agent is monomethyl auristatin F (MMAF).

15. An immunoconjugate of any one of claims 12-14 wherein the antibody is linked to the cytotoxic agent via a linker.

16. An immunoconjugate of claim 15 wherein the linker is selected from 6-maleimidocaproyl (MC), maleimidopropanoyl (MP), valine-citrulline (val-cit), alanine-phenylalanine (ala-phe), p-aminobenzyloxycarbonyl (PAB), N-Succinimidyl 4-(2-pyridylthio)pentanoate (SPP), N-succinimidyl 4-(N-maleimidomethyl)cyclohexane-1 carboxylate (SMCC), and N-Succinimidyl (4-iodo-acetyl) aminobenzoate (SIAB).

17. An immunoconjugate of claim 16 wherein the linker is 6-maleimidocaproyl (MC).

18. An immunoconjugate comprising an anti-B Cell Maturation Antigen (CD269) antibody and a cytotoxic agent wherein the anti-B Cell Maturation Antigen (CD269) antibody has enhanced binding to FcγRIIIA or has enhanced FcyRIIIA mediated effector function and comprises the heavy chain amino acid sequence according to SEQ ID NO: 55 and the light chain amino acid sequence according to SEQ ID NO:63, and wherein the antibody is defucosylated and wherein the cytotoxic agent is monomethyl auristatin F (MMAF) and wherein said MMAF is linked to said antibody via a 6-maleimidocaproyl( MC) linker.

19. A pharmaceutical composition comprising the antibody according to any one of claims 1-5 or claim 11 or the immunoconjugate according to any one of claims 12-18 and a pharmaceutically acceptable carrier.

20. An antibody according to any one of claims 1-5 and 11, an immunoconjugate according to any one of claims 12-18, or a pharmaceutical composition according to claim 19 for use in treating a human patient afflicted with a B-cell disease or disorder selected from Multiple Myeloma (MM), chronic lymphocytic leukemia (CLL), Non-secretory multiple myeloma, Smoldering multiple myeloma, Monoclonal gammopathy of undetermined significance (MGUS), Solitary plasmacytoma (Bone, Extramedullary), Lymphoplasmacytic lymphoma (LPL), Waldenström's Macroglobulinemia, Plasma cell leukemia, Primary Amyloidosis (AL), Heavy chain disease, Systemic lupus erythematosus (SLE), POEMS syndrome / osteosclerotic myeloma, Type I and II cryoglobulinemia, Light chain deposition disease, Goodpasture's syndrome, Idiopathic thrombocytopenic purpura (ITP), Acute glomerulonephritis, Pemphigus and Pemphigoid disorders, and Epidermolysis bullosa acquisita; or any Non-Hodgkin's Lymphoma B-cell leukemia or Hodgkin's lymphoma (HL).

21. The antibody, immunoconjugate or pharmaceutical composition for use according to claim 20 wherein the B-cell disease is Multiple Myeloma (MM).

**Patentansprüche**

1. Antikörper des Anti-B-Zellreifungsantigens (CD269), umfassend eine variable Region der schweren Kette von SEQ. ID. NO:23 und eine variable Region der leichten Kette von SEQ. ID. NO:31.

2. Antikörper des Anti-B-Zellreifungsantigens (CD269) nach Anspruch 1, der eine schwere Kette von SEQ. ID. NO:55 und eine leichte Kette von SEQ. ID:63 umfasst.

**3.** Antikörper des Anti-B-Zellreifungsantigens (CD269) nach einem der vorstehenden Ansprüche, wobei der Antikörper ein monoklonaler Antikörper ist.

**4.** Antikörper des Anti-B-Zellreifungsantigens (CD269) nach einem der vorstehenden Ansprüche, wobei der Antikörper eine verbesserte Bindung an FcγRIIIA aufweist oder eine verbesserte FcγRIIIA-vermittelte Effektorfunktion aufweist.

**5.** Antikörper des Anti-B-Zellreifungsantigens (CD269) nach Anspruch 4, wobei der Antikörper defucosyliert ist.

**6.** Nukleinsäuresequenzen, die sowohl für die variable Sequenz der schweren Kette als auch für die variable Sequenz der leichten Kette des Antikörpers nach einem der vorstehenden Ansprüche codieren.

**7.** Nukleinsäuresequenzen, umfassend SEQ ID NO 56, die für die schwere Kette codiert; und SEQ ID NO: 64, die für die leichte Kette des Antikörpers nach einem der vorstehenden Ansprüche codiert.

**8.** Expressionsvektor, umfassend die Nukleinsäuresequenzen nach Anspruch 6 oder 7.

**9.** Rekombinante transformierte oder transfizierte Wirtszelle, umfassend die Nukleinsäuresequenzen nach Anspruch 6 oder 7 oder den Expressionsvektor nach Anspruch 8, wobei die Wirtszelle Nukleinsäuresequenzen, die sowohl für die schwere als auch leichte Kette codieren, umfasst.

**10.** Verfahren für die Erzeugung eines Antikörpers, wobei das Verfahren ein Kultivieren der Wirtszelle nach Anspruch 9 unter Bedingungen, die für eine Expression der Nukleinsäuresequenzen oder -vektor(en) geeignet sind, umfasst, wodurch der Antikörper erzeugt wird.

**11.** Antikörper, wie durch das Verfahren nach Anspruch 10 erzeugt.

**12.** Immunkonjugat, umfassend den Antikörper nach einem der Ansprüche 1 bis 5 oder Anspruch 11 und ein cytotoxisches Mittel.

**13.** Immunkonjugat nach Anspruch 12, wobei das cytotoxische Mittel Monomethylauristatin E (MMAE) oder Monomethylauristatin F (MMAF) ist.

**14.** Immunkonjugat nach Anspruch 13, wobei das cytotoxische Mittel Monomethylauristatin F (MMAF) ist.

**15.** Immunkonjugat nach einem der Ansprüche 12 bis 14, wobei der Antikörper mit dem cytotoxischen Mittel über einen Linker verknüpft ist.

**16.** Immunkonjugat nach Anspruch 15, wobei der Linker aus 6-Maleimidocaproyl (MC), Maleimidopropanoyl (MP), Valin-Citrullin (val-cit), Alanin-Phenylalanin (ala-phe), p-Aminobenzyloxycarbonyl (PAB), N-succinimidyl 4-(2-pyridylthio)pentanoat (SPP), N-succinimidyl 4-(N-maleimidomethyl)cyclohexan-1-carboxylat (SMCC) und N-succinimidyl(4-iod-acetyl)aminobenzoat (SIAB) ausgewählt ist.

**17.** Immunkonjugat nach Anspruch 16, wobei der Linker 6-Maleimidocaproyl (MC) ist.

**18.** Immunkonjugat, umfassend einen Antikörper des Anti-B-Zellreifungsantigens (CD269) und ein cytotoxisches Mittel, wobei der Antikörper des Anti-B-Zellreifungsantigens (CD269) eine verbesserte Bindung an FcγRIIIA aufweist oder eine verbesserte FcγRIIIA-vermittelte Effektorfunktion aufweist und die Aminosäuresequenz der schweren Kette gemäß SEQ ID NO: 55 und die Aminosäuresequenz der leichten Kette gemäß SEQ ID NO:63 umfasst und wobei der Antikörper defucosyliert ist und wobei das cytotoxische Mittel Monomethylauristatin F (MMAF) ist und wobei das MMAF mit dem Antikörper über einen Linker von 6-Maleimidocaproyl (MC) verknüpft ist.

**19.** Pharmazeutische Zusammensetzung, umfassend den Antikörper nach einem der Ansprüche 1 bis 5 oder Anspruch 11 oder das Immunkonjugat nach einem der Ansprüche 12 bis 18 und einen pharmazeutisch verträglichen Träger.

**20.** Antikörper nach einem der Ansprüche 1 bis 5 und 11, ein Immunkonjugat nach einem der Ansprüche 12 bis 18 oder eine pharmazeutische Zusammensetzung nach Anspruch 19 zur Verwendung bei einem Behandeln eines menschlichen Patienten, der von einer B-Zellkrankheit oder -störung betroffen ist, die aus multiplem Myelom (MM), chronischer lymphatischer Leukämie (CLL), nicht sekretorischem multiplem Myelom, schwelendem multiplem Myelom,

monoklonaler Gammopathie unklarer Signifikanz (MGUS), solitärem Plasmozytom (Knochen, extramedullär), lymphoplasmazytischem Lymphom (LPL), Waldenströms Makroglobulinämie, Plasmazellleukämie, primärer Amyloidose (AL), Schwerkettenkrankheit, systemischem Lupus erythematodes (SLE), POEMS-Syndrom / osteosklerotischem Myelom, Typ-I- und -II-Kryoglobulinämie, Leichtkettenablagerungskrankheit, Goodpasture-Syndrom, idiopathischer thrombozytopenischer Purpura (ITP), akuter Glomerulonephritis, Pemphigus- und Pemphigoid-Störungen, und Epidermolysis bullosa acquisita; oder einer beliebigen Nicht-Hodgkin-Lymphom-B-Zellleukämie oder Hodgkin-Lymphom (HL) ausgewählt ist.

**21.** Antikörper, Immunkonjugat oder pharmazeutische Zusammensetzung zur Verwendung nach Anspruch 20, wobei die B-Zellkrankheit multiples Myelom (MM) ist.

**Revendications**

**1.** Anticorps anti-antigène de maturation de lymphocytes B (CD269) comprenant une région variable de chaîne lourde de SEQ. ID. NO:23 et une région variable de chaîne légère de SEQ. ID. NO:31.

**2.** Anticorps anti-antigène de maturation de lymphocytes B (CD269) selon la revendication 1 qui comprend une chaîne lourde de SEQ. ID. NO:55 et une chaîne légère de SEQ. ID. NO:63.

**3.** Anticorps anti-antigène de maturation de lymphocytes B (CD269) selon l'une quelconque revendication précédente, dans lequel l'anticorps est un anticorps monoclonal.

**4.** Anticorps anti-antigène de maturation de lymphocytes B (CD269) selon l'une quelconque revendication précédente, dans lequel l'anticorps a une liaison renforcée à FcγRIIIA ou a une fonction effectrice médiée par FcγRIIIA renforcée.

**5.** Anticorps anti-antigène de maturation de lymphocytes B (CD269) selon la revendication 4, dans lequel l'anticorps est défucosylé.

**6.** Séquences d'acides nucléiques codant à la fois pour les séquences variable de chaîne lourde et variable de chaîne légère de l'anticorps selon l'une quelconque revendication précédente.

**7.** Séquences d'acide nucléique comprenant SEQ ID NO 56 codant pour la chaîne lourde ; et SEQ ID NO: 64 codant pour la chaîne légère de l'anticorps selon l'une quelconque revendication précédente.

**8.** Vecteur d'expression comprenant les séquences d'acide nucléique selon la revendication 6 ou la revendication 7.

**9.** Cellule hôte recombinée transformée ou transfectée comprenant les séquences d'acide nucléique selon la revendication 6 ou la revendication 7, ou le vecteur d'expression selon la revendication 8, dans laquelle la cellule hôte comprend à la fois les séquences d'acide nucléique codant pour les chaînes lourde et légère.

**10.** Procédé de production d'un anticorps, lequel procédé comprend la culture de la cellule hôte selon la revendication 9 dans des conditions appropriées pour une expression desdites séquences d'acide nucléique ou de vecteur(s), selon lequel l'anticorps est produit.

**11.** Anticorps tel que produit par le procédé selon la revendication 10.

**12.** Immunoconjugué comprenant l'anticorps selon l'une quelconque des revendications 1 à 5 ou la revendication 11 et un agent cytotoxique.

**13.** Immunoconjugué selon la revendication 12 dans lequel l'agent cytotoxique est la monométhyl-auristatine E (MMAE) ou la monométhyl-auristatine F (MMAF).

**14.** Immunoconjugué selon la revendication 13 dans lequel l'agent cytotoxique est la monométhyl-auristatine F (MMAF).

**15.** Immunoconjugué selon l'une quelconque des revendications 12 à 14, dans lequel l'anticorps est lié à l'agent cytotoxique par l'intermédiaire d'un lieur.

16. Immunoconjugué selon la revendication 15 dans lequel le lieur est choisi parmi 6-maléimidocaproyle (MC), maléimidopropanoyle (MP), valine-citrulline (val-cit), alanine-phénylalanine (ala-phé), p-aminobenzyloxycarbonyle (PAB), 4-(2-pyridylthio)pentanoate de N-succinimidyle (SPP), 4-(N-maléimidométhyl)cyclohexane-1 carboxylate de N-succinimidyle (SMCC), et (4-iodo-acétyl) aminobenzoate de N-succinimidyle (SIAB).

17. Immunoconjugué selon la revendication 16 dans lequel le lieur est 6-maléimidocaproyle (MC).

18. Immunoconjugué comprenant un anticorps anti-antigène de maturation de lymphocytes B (CD269) et un agent cytotoxique, dans lequel l'anticorps anti-antigène de maturation de lymphocytes B (CD269) a une liaison renforcée à FcγRIIIA ou a une fonction effectrice médiée par FcγRIIIA renforcée et comprend la séquence d'acides aminés de chaîne lourde selon SEQ ID NO: 55 et la séquence d'acides aminés de chaîne légère selon SEQ ID NO:63, et dans lequel l'anticorps est défucosylé et dans lequel l'agent cytotoxique est la monométhyl-auristatine F (MMAF) et dans lequel ladite MMAF est liée audit anticorps par l'intermédiaire d'un lieur 6-maléimidocaproyle (MC).

19. Composition pharmaceutique comprenant l'anticorps selon l'une quelconque des revendications 1 à 5 ou la revendication 11 ou l'immunoconjugué selon l'une quelconque des revendications 12 à 18 et un véhicule pharmaceutiquement acceptable.

20. Anticorps selon l'une quelconque des revendications 1 à 5 et 11, immunoconjugué selon l'une quelconque des revendications 12 à 18 ou composition pharmaceutique selon la revendication 19 pour utilisation dans le traitement d'un patient humain souffrant d'une maladie ou d'un trouble à lymphocytes B choisi parmi le myélome multiple (MM), la leucémie lymphoïde chronique (LLC), le myélome multiple non sécrétant, le myélome multiple latent, la gammapathie monoclonale de signification indéterminée (GMSI), le plasmocytome solitaire (osseux, extramédullaire), le lymphome lymphoplasmocytaire (LPL), la macroglobulinémie de Waldenström, la leucémie à plasmocytes, l'amylose primaire (AL), la maladie des chaînes lourdes, le lupus érythémateux disséminé (LED), le syndrome POEMS ou le myélome ostéoscléreux, la cryoglobulinémie de type I et II, la maladie des dépôts de chaînes légères, le syndrome de Goodpasture, la purpura thrombopénique idiopathique (PTI), la glomérulonéphrite aiguë, les troubles du pemphigus et pemphigoïdes et l'épidermolyse bulleuse acquise ; ou n'importe quel lymphome non hodgkinien, leucémie à lymphocytes B ou lymphome de Hodgkin (LH).

21. Anticorps, immunoconjugué ou composition pharmaceutique pour utilisation selon la revendication 20, dans lequel la maladie à lymphocytes B est un myélome multiple (MM).

Figure 1:

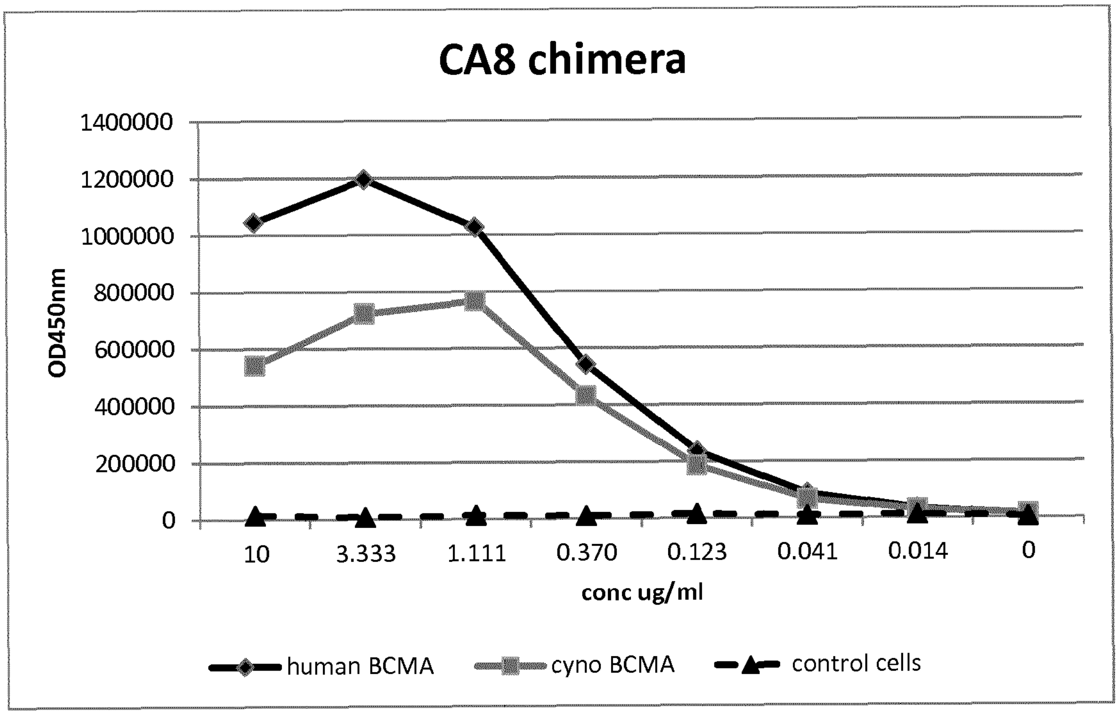
CA8 chimera
1400000
1200000
1000000
800000
600000
400000
200000
0
OD450nm
10
3.333
1.111
0.370
0.123
0.041
0.014
0
conc ug/ml
human BCMA
cyno BCMA
control cells

Figure 2:

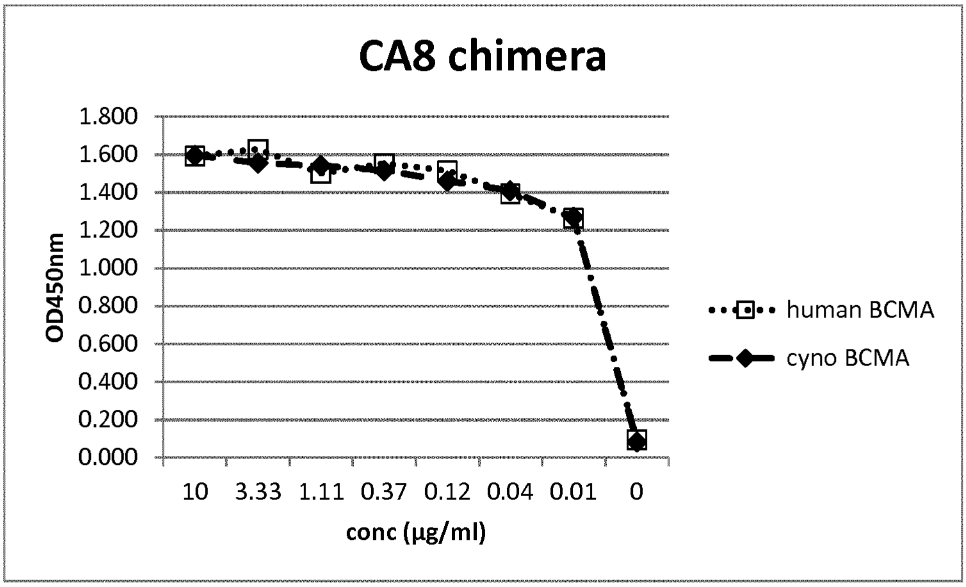
CA8 chimera
1.800
1.600
1.400
1.200
1.000
0.800
0.600
0.400
0.200
0.000
OD450nm
10
3.33
1.11
0.37
0.12
0.04
0.01
0
conc (µg/ml)
human BCMA
cyno BCMA

Figure 3:

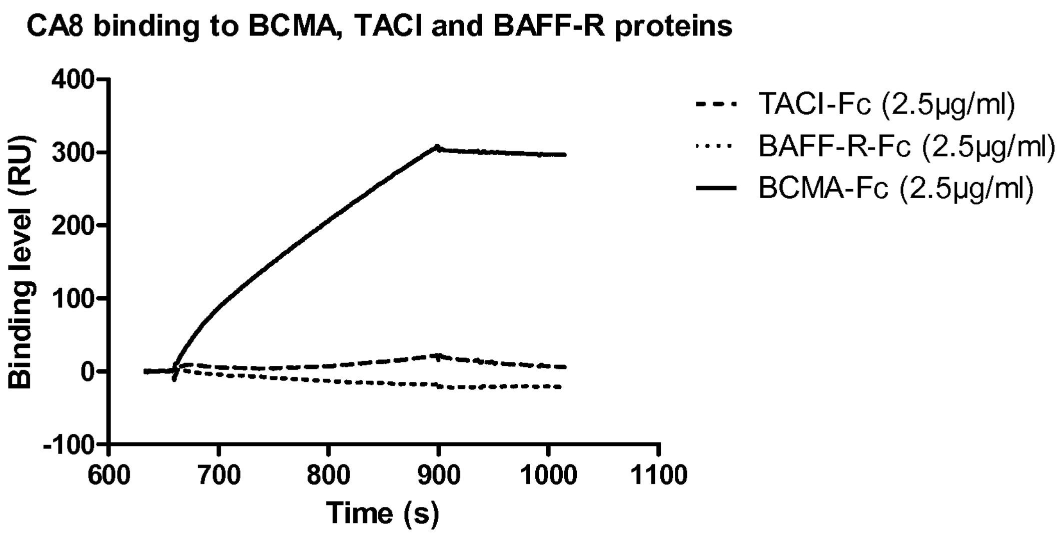
CA8 binding to BCMA, TACI and BAFF-R proteins
400
300
200
100
0
-100
Binding level (RU)
600
700
800
900
1000
1100
Time (s)
TACI-Fc (2.5µg/ml)
BAFF-R-Fc (2.5µg/ml)
BCMA-Fc (2.5µg/ml)

Figure 4:

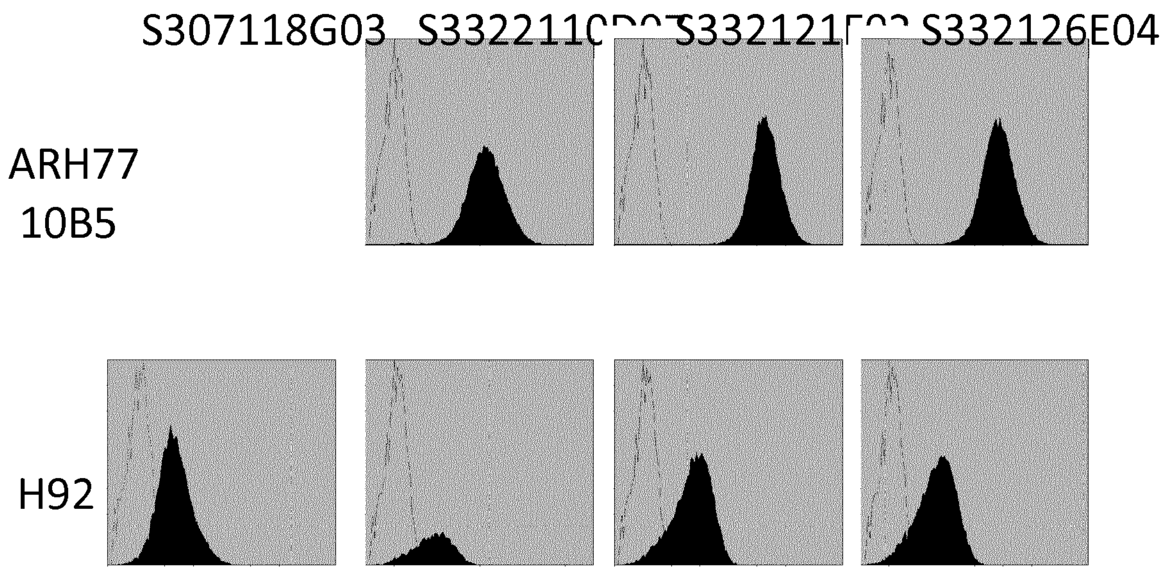

Figure 5:

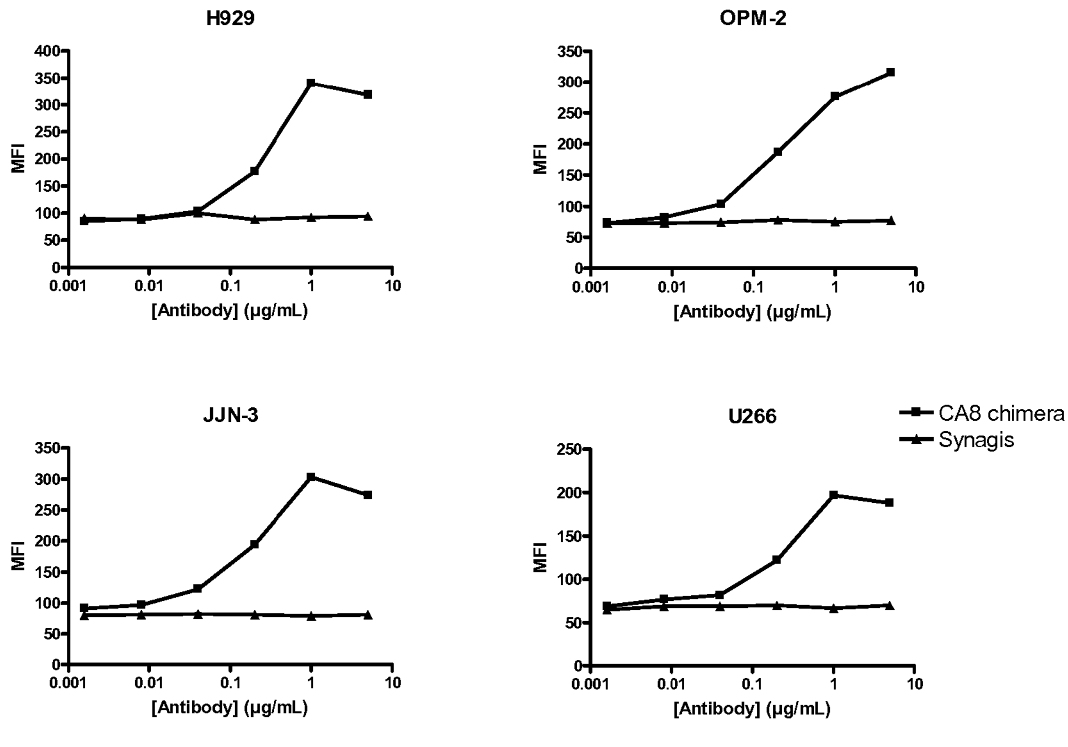
H929
OPM-2
JJN-3
U266
MFI
[Antibody] (µg/mL)
0.001
0.01
0.1
1
10
CA8 chimera
Synagis

Figure 6:

A- Binding to ARH-77 10B5 cells

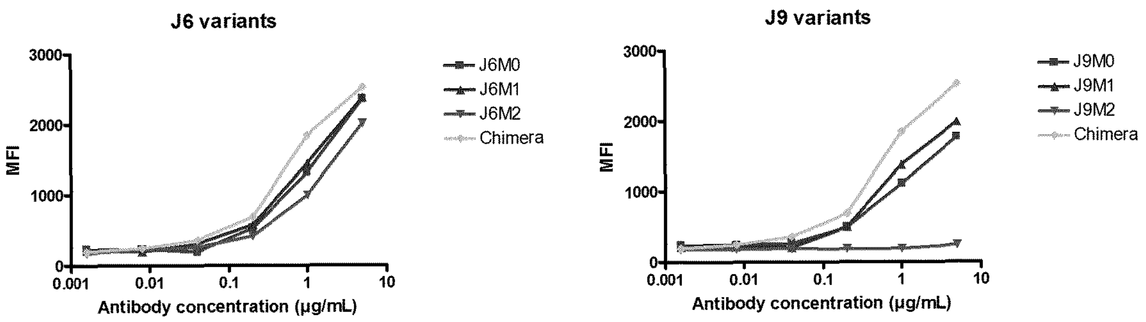


B - Binding to H929 cells

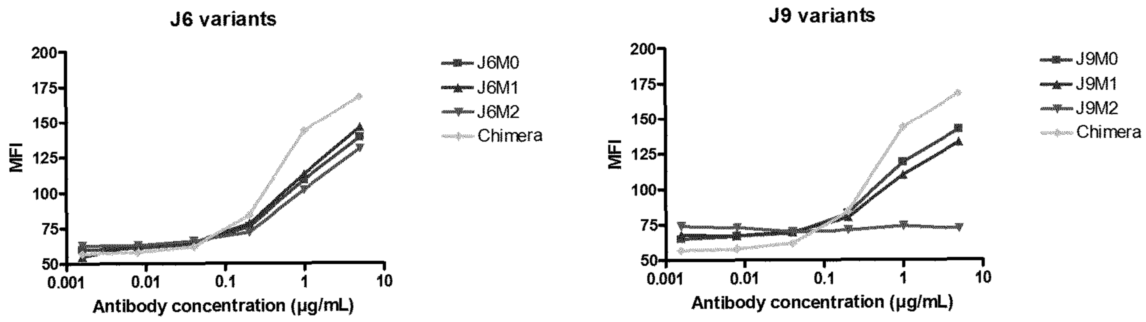

Figure 7:

(A)

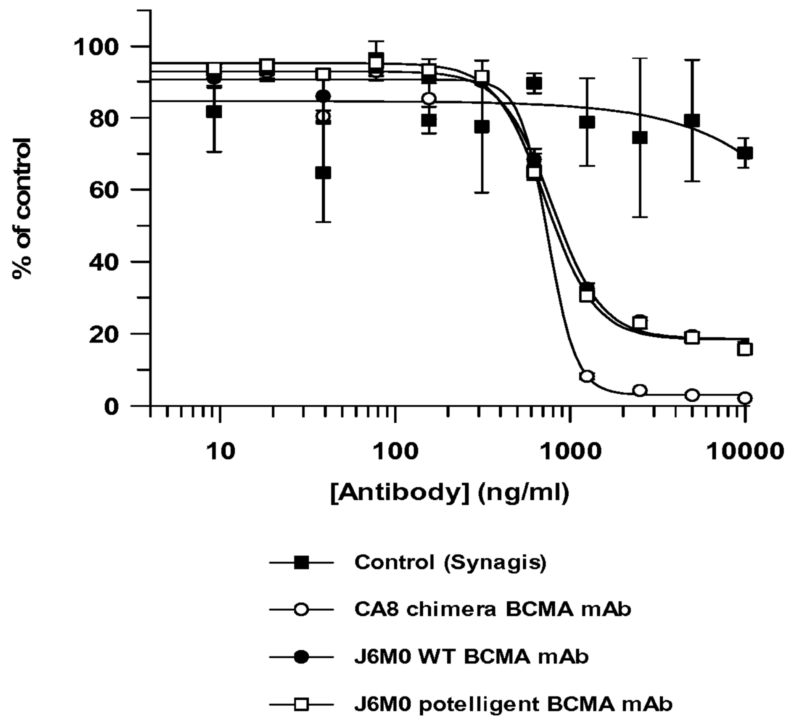
% of control
100
80
60
40
20
0
10
100
1000
10000
[Antibody] (ng/ml)
Control (Synagis)
CA8 chimera BCMA mAb
J6M0 WT BCMA mAb
J6M0 potelligent BCMA mAb

(B)

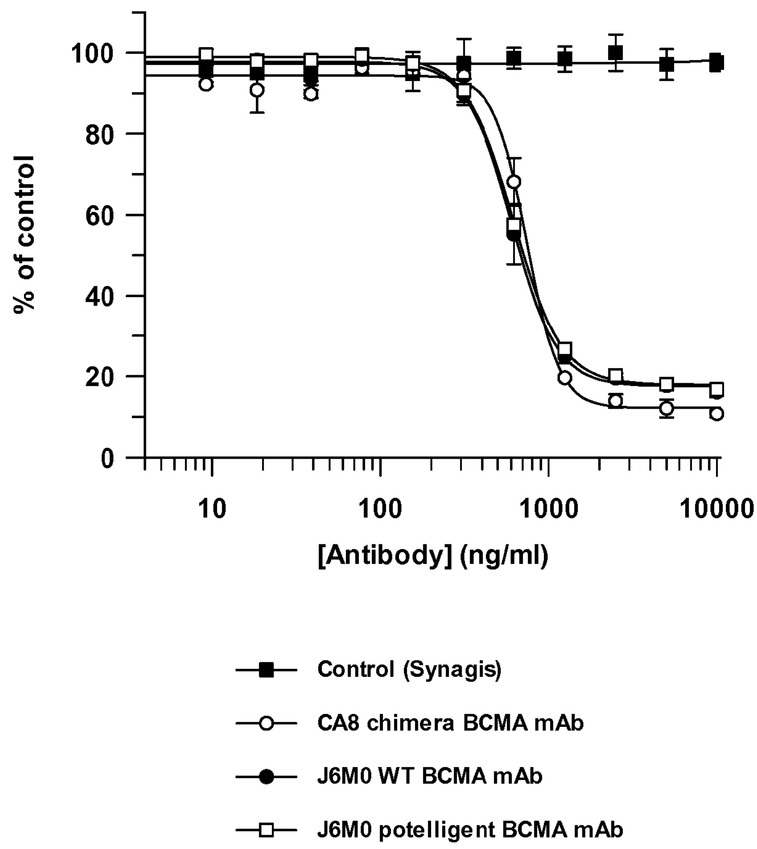
% of control
100
80
60
40
20
0
10
100
1000
10000
[Antibody] (ng/ml)
Control (Synagis)
CA8 chimera BCMA mAb
J6M0 WT BCMA mAb
J6M0 potelligent BCMA mAb

(C)

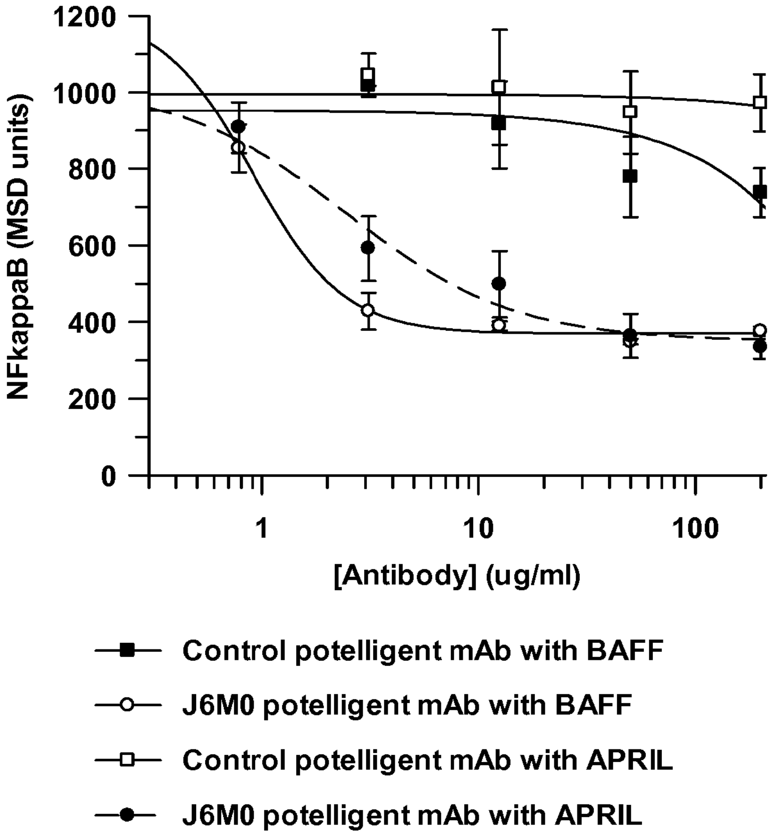

1200
1000
800
600
400
200
0
NFkappaB (MSD units)
1
10
100
[Antibody] (ug/ml)
Control potelligent mAb with BAFF
J6M0 potelligent mAb with BAFF
Control potelligent mAb with APRIL
J6M0 potelligent mAb with APRIL

Figure 8:

(A)

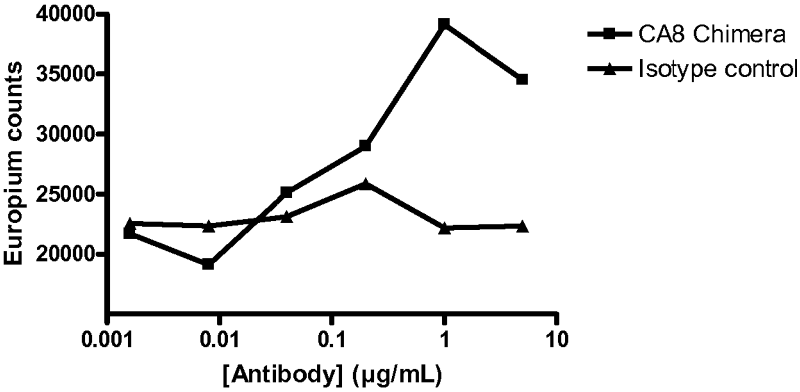
40000
35000
30000
25000
20000
Europium counts
0.001
0.01
0.1
1
10
[Antibody] (μg/mL)
CA8 Chimera
Isotype control

(B)

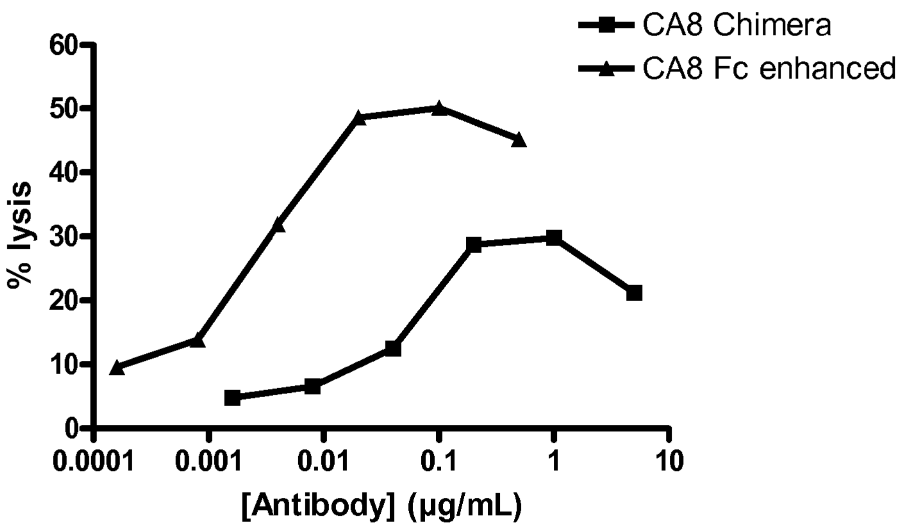
60
50
40
30
20
10
0
% lysis
0.0001
0.001
0.01
0.1
1
10
[Antibody] (μg/mL)
CA8 Chimera
CA8 Fc enhanced

Figure 9:

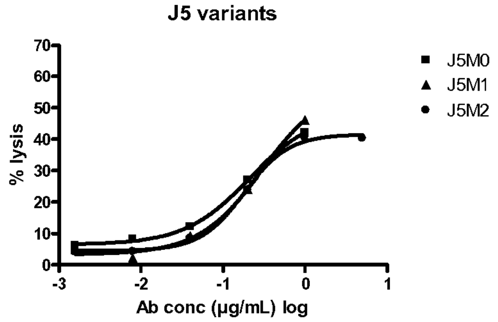


| | J5M0 | J5M1 | J5M2 |
|---|---|---|---|
| EC50 | 0.2087 | 0.3045 | 0.1822 |

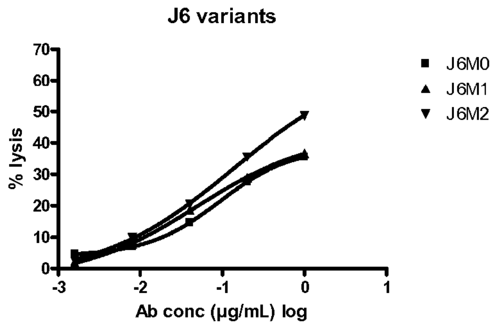


| | J6M0 | J6M1 | J6M2 |
|---|---|---|---|
| EC50 | 0.09187 | 0.04860 | 0.1364 |

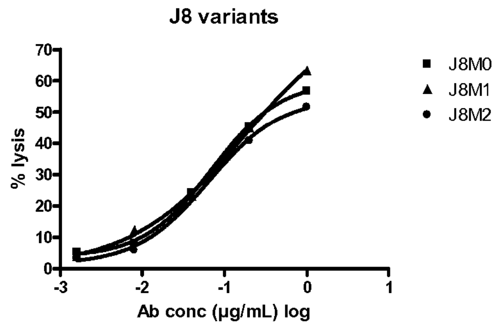


| | J8M0 | J8M1 | J8M2 |
|---|---|---|---|
| EC50 | 0.07106 | 0.1734 | 0.06510 |

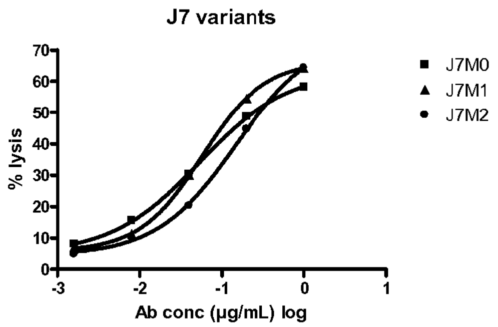


| | J7M0 | J7M1 | J7M2 |
|---|---|---|---|
| EC50 | 0.05386 | 0.05761 | 0.1506 |

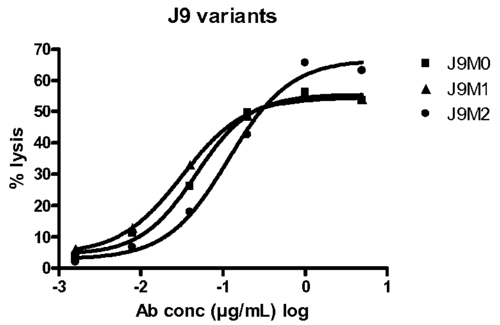


| | J9M0 | J9M1 | J9M2 |
|---|---|---|---|
| EC50 | 0.04738 | 0.03225 | 0.1170 |

Figure 10:

A

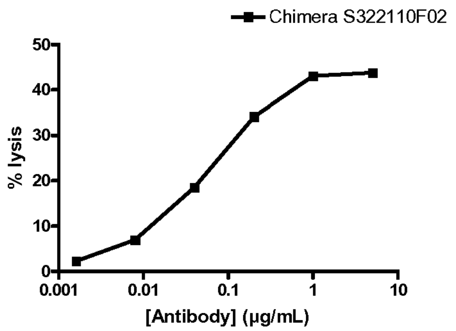


B

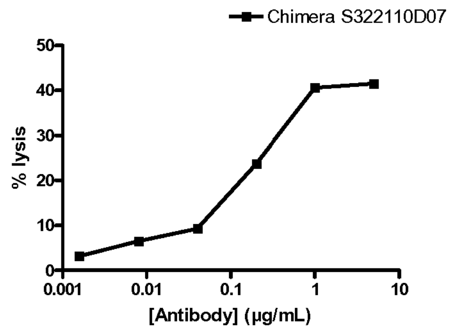


C

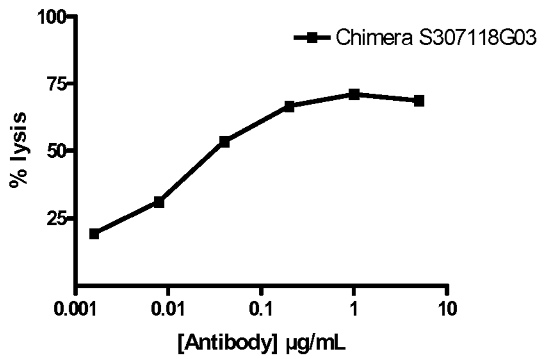


D

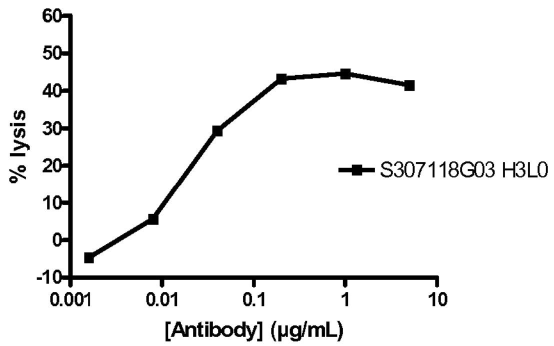

Figure 11:

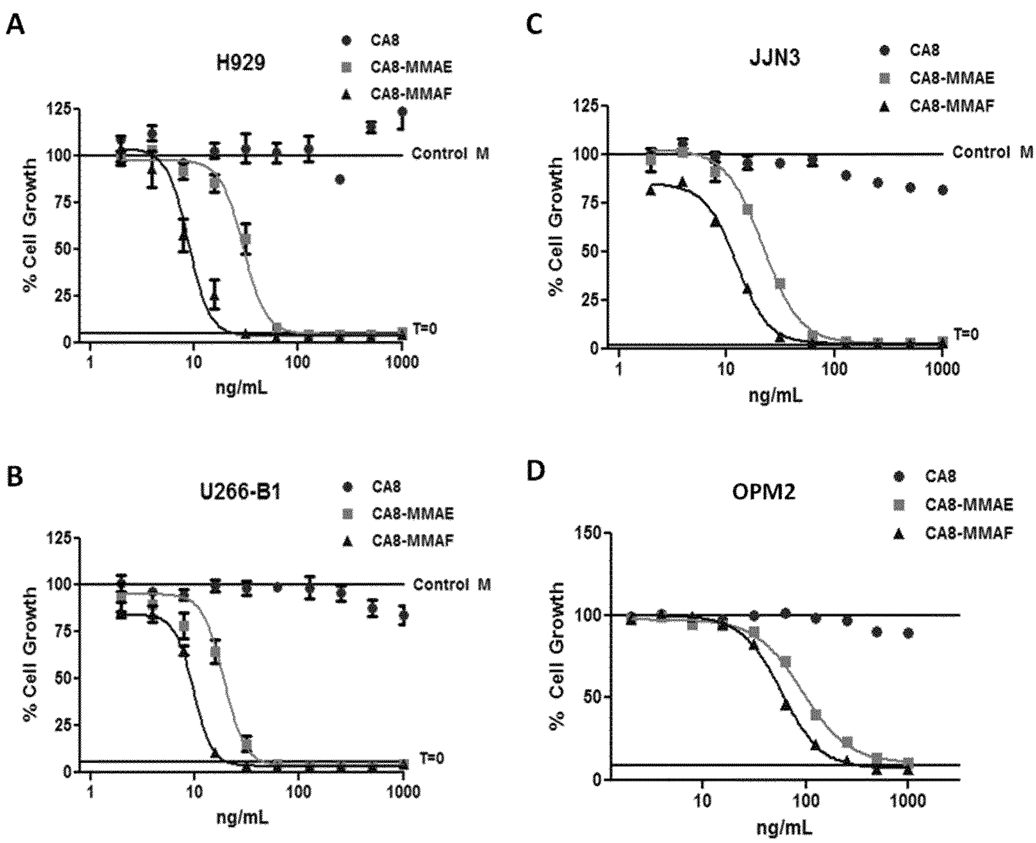
A
H929
CA8
CA8-MMAE
CA8-MMAF
% Cell Growth
ng/mL
Control M
T=0
C
JJN3
CA8
CA8-MMAE
CA8-MMAF
% Cell Growth
ng/mL
Control M
T=0
B
U266-B1
CA8
CA8-MMAE
CA8-MMAF
% Cell Growth
ng/mL
Control M
T=0
D
OPM2
CA8
CA8-MMAE
CA8-MMAF
% Cell Growth
ng/mL

Figure 12:

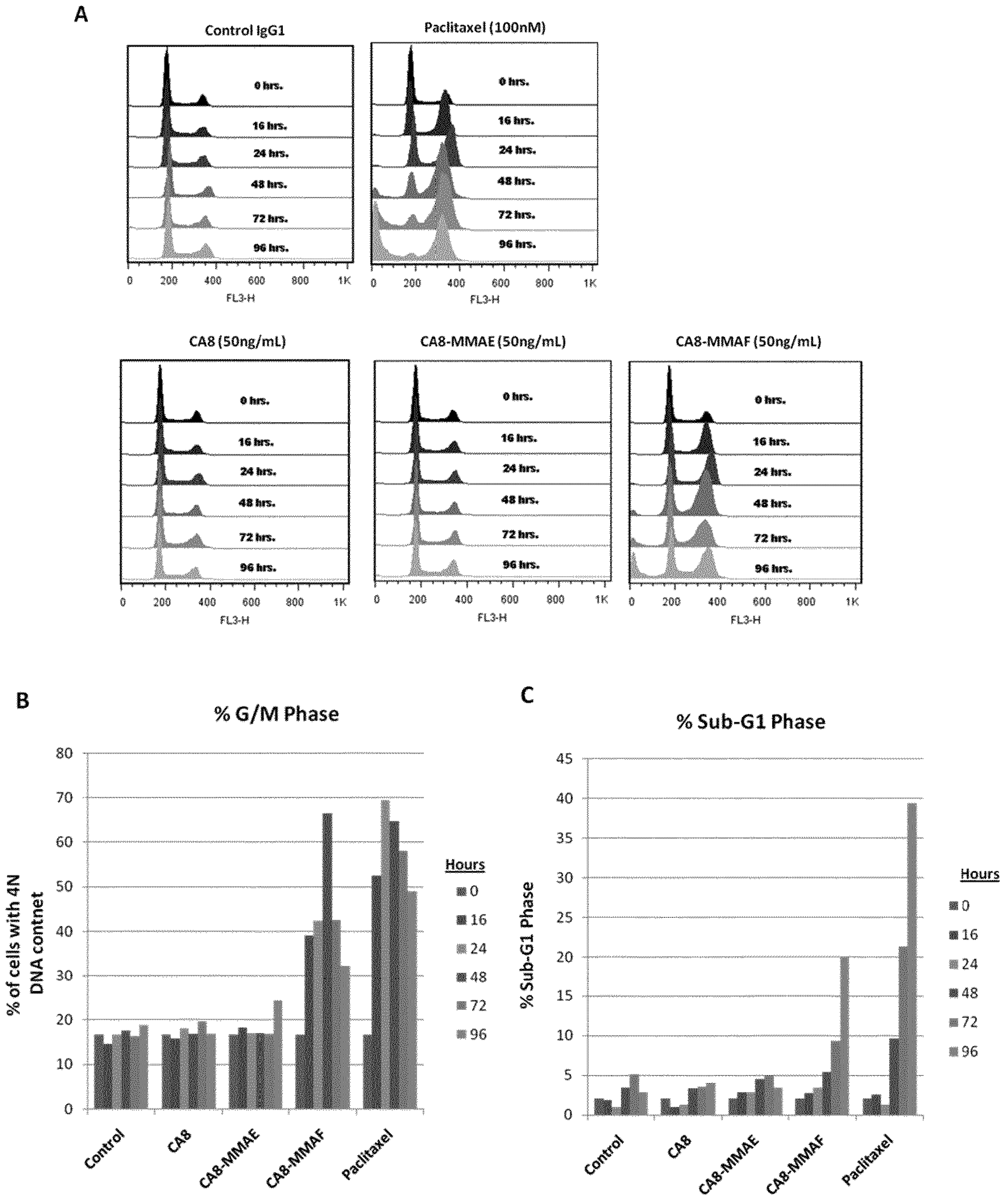
A
Control IgG1
Paclitaxel (100nM)
CA8 (50ng/mL)
CA8-MMAE (50ng/mL)
CA8-MMAF (50ng/mL)
0 hrs.
16 hrs.
24 hrs.
48 hrs.
72 hrs.
96 hrs.
0
200
400
600
800
1K
FL3-H
B
% G/M Phase
% of cells with 4N DNA contnet
Hours
0
16
24
48
72
96
Control
CA8
CA8-MMAE
CA8-MMAF
Paclitaxel
C
% Sub-G1 Phase
% Sub-G1 Phase

Figure 13:

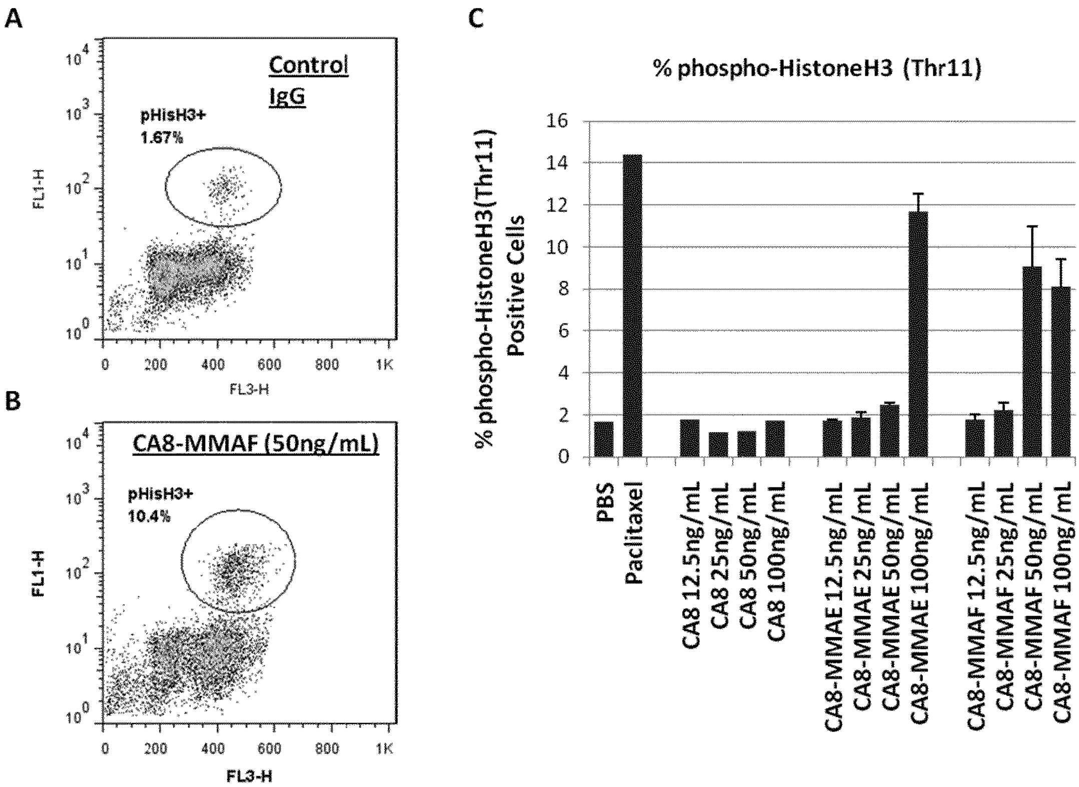
A
Control IgG
pHisH3+
1.67%
FL1-H
FL3-H
B
CA8-MMAF (50ng/mL)
pHisH3+
10.4%
FL1-H
FL3-H
C
% phospho-HistoneH3 (Thr11)
% phospho-HistoneH3(Thr11) Positive Cells
PBS
Paclitaxel
CA8 12.5ng/mL
CA8 25ng/mL
CA8 50ng/mL
CA8 100ng/mL
CA8-MMAE 12.5ng/mL
CA8-MMAE 25ng/mL
CA8-MMAE 50ng/mL
CA8-MMAE 100ng/mL
CA8-MMAF 12.5ng/mL
CA8-MMAF 25ng/mL
CA8-MMAF 50ng/mL
CA8-MMAF 100ng/mL

Figure 14:

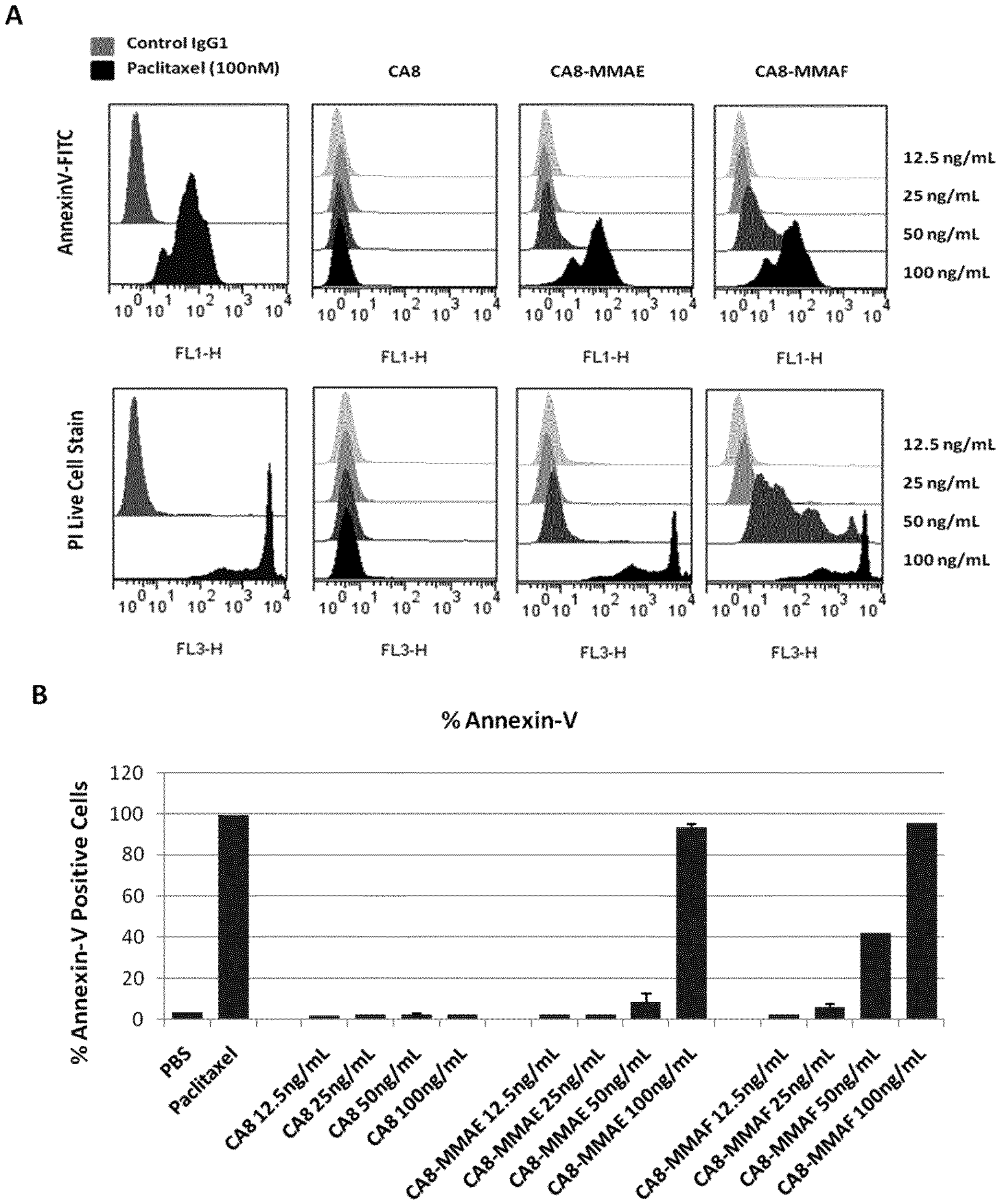
A
Control IgG1
Paclitaxel (100nM)
CA8
CA8-MMAE
CA8-MMAF
AnnexinV-FITC
12.5 ng/mL
25 ng/mL
50 ng/mL
100 ng/mL
FL1-H
PI Live Cell Stain
FL3-H
B
% Annexin-V
% Annexin-V Positive Cells
PBS
Paclitaxel
CA8 12.5ng/mL
CA8 25ng/mL
CA8 50ng/mL
CA8 100ng/mL
CA8-MMAE 12.5ng/mL
CA8-MMAE 25ng/mL
CA8-MMAE 50ng/mL
CA8-MMAE 100ng/mL
CA8-MMAF 12.5ng/mL
CA8-MMAF 25ng/mL
CA8-MMAF 50ng/mL
CA8-MMAF 100ng/mL

Figure 15:

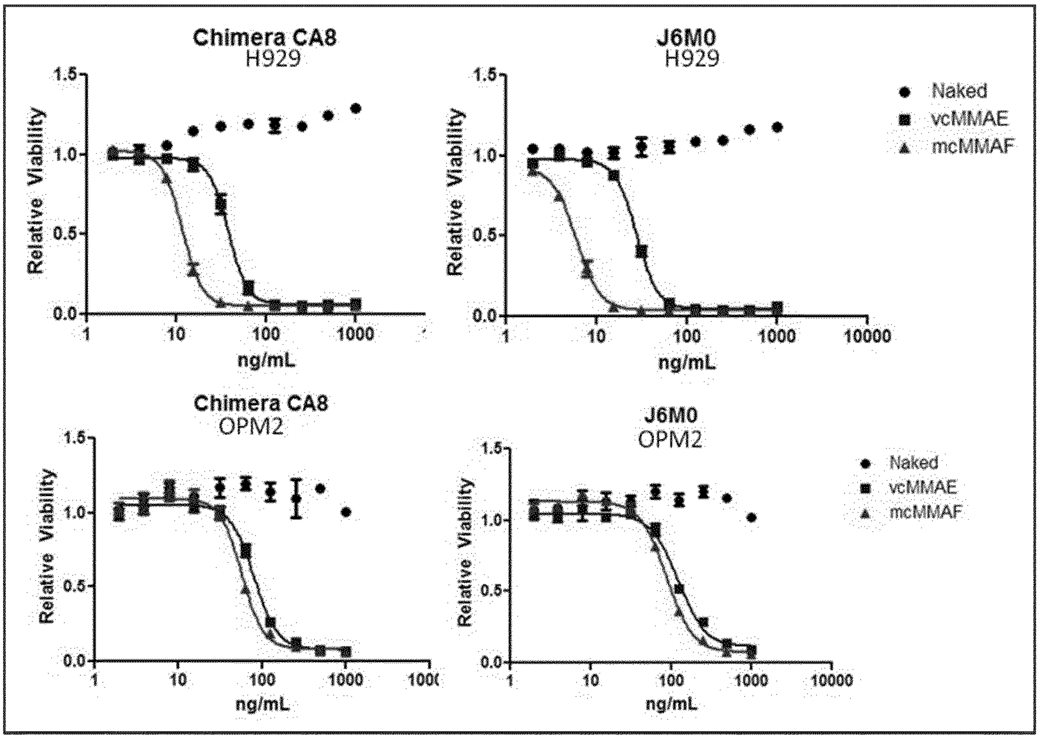
Chimera CA8
H929
J6M0
H929
Chimera CA8
OPM2
J6M0
OPM2
Relative Viability
ng/mL
Naked
vcMMAE
mcMMAF
1.5
1.0
0.5
0.0
1
10
100
1000
10000

Figure 16:

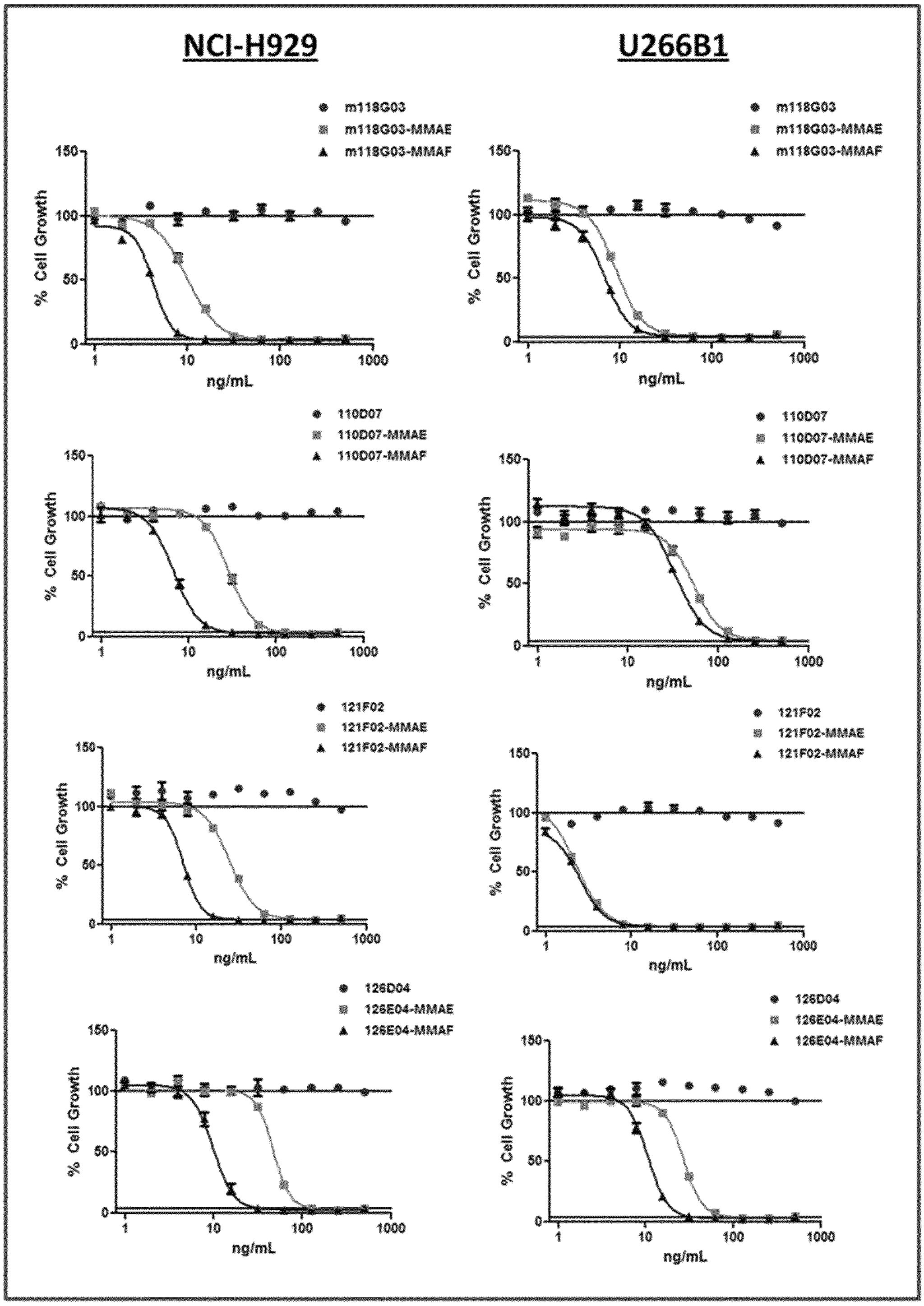
NCI-H929
U266B1
m118G03
m118G03-MMAE
m118G03-MMAF
110D07
110D07-MMAE
110D07-MMAF
121F02
121F02-MMAE
121F02-MMAF
126D04
126E04-MMAE
126E04-MMAF
% Cell Growth
ng/mL

Figure 17:

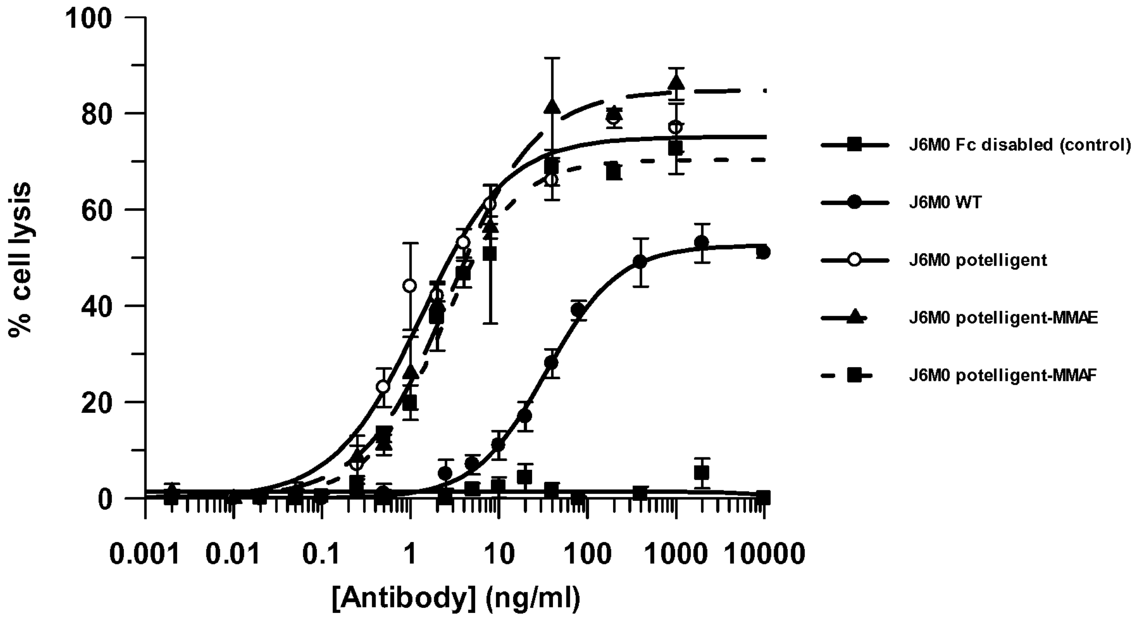


Figure 18:

A

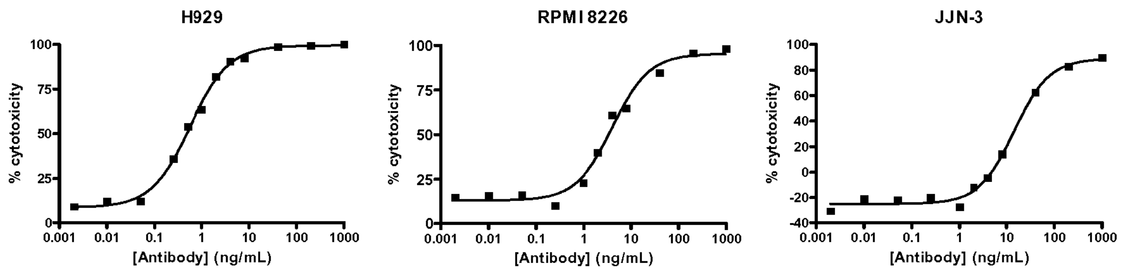


| | CA8 J6M0 potelligent |
|---|---|
| EC50 | 0.5694 |

| | CA8 J6M0 potelligent |
|---|---|
| EC50 | 4.092 |

| | CA8 J6M0 potelligent |
|---|---|
| EC50 | 14.42 |

B

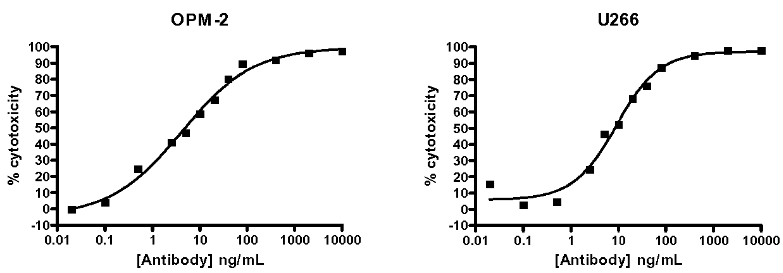


| | CA8 J6M0 potelligent |
|---|---|
| EC50 | 4.153 |

| | CA8 J6M0 potelligent |
|---|---|
| EC50 | 9.042 |

Figure 19:

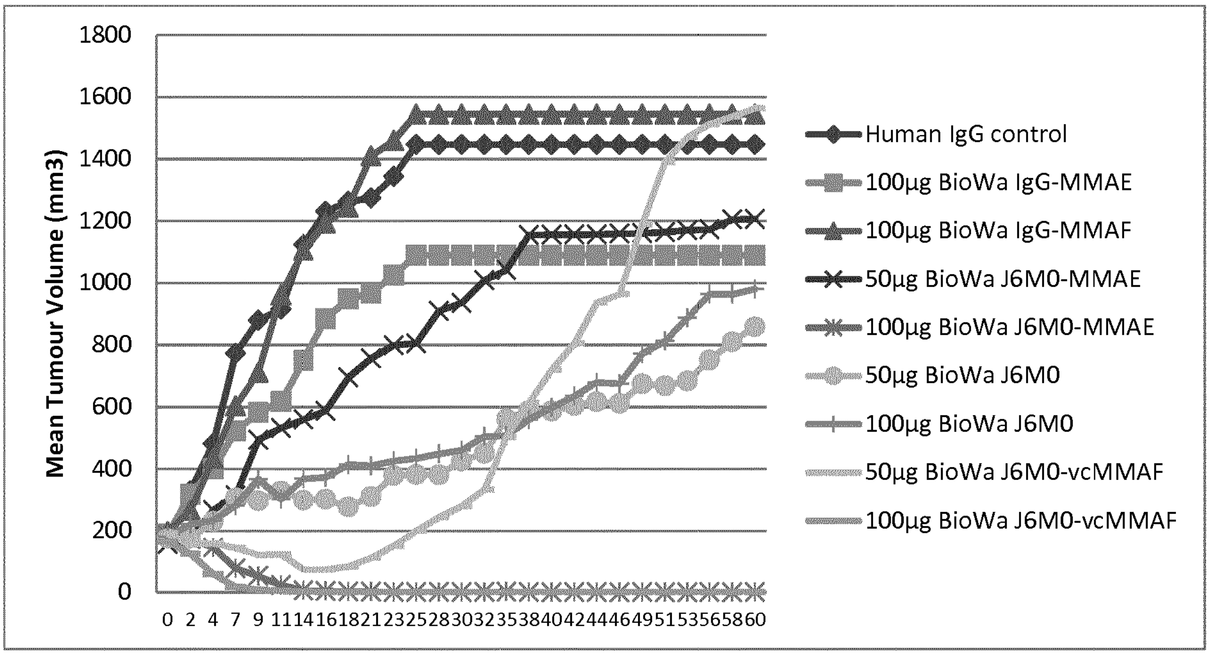
1800
1600
1400
1200
1000
800
600
400
200
0
Mean Tumour Volume (mm3)
0 2 4 7 9 11 14 16 18 21 23 25 28 30 32 35 38 40 42 44 46 49 51 53 56 58 60
Human IgG control
100µg BioWa IgG-MMAE
100µg BioWa IgG-MMAF
50µg BioWa J6M0-MMAE
100µg BioWa J6M0-MMAE
50µg BioWa J6M0
100µg BioWa J6M0
50µg BioWa J6M0-vcMMAF
100µg BioWa J6M0-vcMMAF

Figure 20:

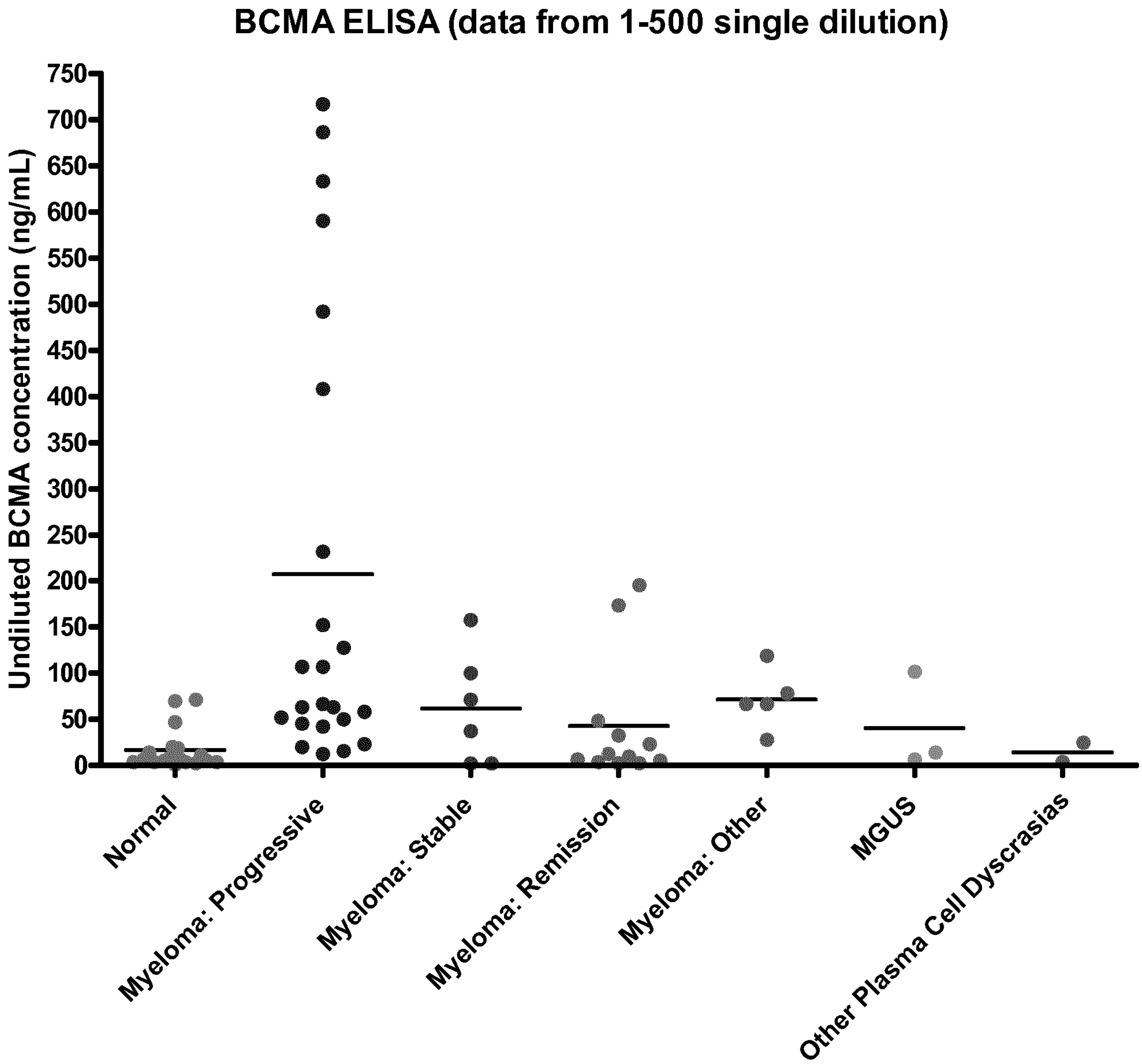


Normal: n=18
Myeloma: Progressive: n=23
Myeloma: Stable:n=6
Myeloma: Remission: n=12
Myeloma: Other: n=5
MGUS: n=3
Other PCD: n=2
*note: data above or below standards not graphed

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010104949 A2
- WO 8601533 A
- WO 9958679 A
- WO 9616990 A
- US 4873316 A
- US 4816567 A
- EP 0239400 A
- EP 054951 A
- WO 0029004 A
- US 20050043519 A
- US 7250297 B1
- US 20070224633 A
- EP 1641818 A1
- US 20040132028 A1
- US 20080139791 A
- WO 2005056764 A
- US 6818418 B1
- WO 2008098796 A
- EP 0307434 A
- WO 2003011878 A
- WO 2006014679 A
- EP 1229125 A
- US 7214775 B
- US 6946292 B
- WO 0061739 A
- WO 0231240 A
- WO 2007011041 A
- US 20070148165 A
- US 4975278 A
- EP 1391213 A
- WO 9321232 A
- US 5635483 A
- US 5780588 A
- US 5663149 A
- WO 02088172 A
- US 7498298 B
- US 6884869 B
- US 7098308 B
- US 7256257 B
- US 7423116 B
- US 7964566 B
- US 3896111 A
- US 4151042 A
- US 6573074 B
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5208020 A
- US 6570024 B
- US 6884874 B
- US 5712374 A
- US 5714586 A
- US 5739116 A
- US 5767285 A
- US 5770701 A
- US 57707105773001 A
- US 5877296 A
- US 5053394 A
- US 5770710 A
- US 6214345 B
- US 20030096743 A
- US 20030130189 A
- US 5362852 A
- US 5122368 A
- US 5824805 A
- US 5622929 A
- US 4880935 A
- WO 2004010957 A
- US 40040302

**Non-patent literature cited in the description**


- Antibody targeting of B-cell maturation antigen on malignant plasma cells. **RYAN MAUREEN et al.** Molecular Cancer Therapeutics. American Association of Cancer Research, 01 November 2007, vol. 6, 3009-3018
- **PLÜCKTHUN, A.** *Immunol. Rev.,* 1992, vol. 130, 151-188
- **MILLER et al.** Genetic Engineering. Plenum Press, 1986, vol. 8, 277-298
- **REMINGTON'S PHARMACEUTICAL SCIENCE.** Mack Publishing Company
- **LASMAR U ; PARKINS D.** The formulation of Biopharmaceutical products. *Pharma. Sci.Tech.today,* 03 April 2000, vol. 3, 129-137
- **WANG, W.** Instability, stabilisation and formulation of liquid protein pharmaceuticals. *Int. J. Pharm,* 1999, vol. 185, 129-188
- Stability of Protein Pharmaceuticals Part A and B. Plenum Press, 1992
- **AKERS,M.J.** Excipient-Drug interactions in Parenteral Formulations. *J.Pharm Sci,* 2002, vol. 91, 2283-2300
- **IMAMURA, K et al.** Effects of types of sugar on stabilization of Protein in the dried state. *J Pharm Sci,* 2003, vol. 92, 266-274
- **IZUTSU, KKOJIMA, S.** Excipient crystalinity and its protein-structure-stabilizing effect during freeze-drying. *J Pharm. Pharmacol,* 2002, vol. 54, 1033-1039
- **JOHNSON, R.** Mannitol-sucrose mixtures-versatile formulations for protein peroxidise19g19n. *J. Pharm. Sci,* 2002, vol. 91, 914-922
- **HA,E ; WANG W ; WANG Y.J.** Peroxide formation in polysorbate 80 and protein stability. *J. Pharm Sci,* 2002, vol. 91, 2252-2264
- **HARLOW et al.** Antibodies A Laboratory Manual. Cold Spring Harbor Laboratory, 1988
- **MORRISON et al.** *Proc. Natl. Acad. Sci. USA,* 1984, vol. 81, 6851-6855
- **QUEEN et al.** *Proc. Natl Acad Sci USA,* 1989, vol. 86, 10029-10032
- **HODGSON et al.** *Bio/Technology,* 1991, vol. 9, 421
- **KABAT et al.** *Sequences of proteins of Immunological Interest NIH,* 1987
- **AL-LAZIKANI et al.** *JMB,* 1997, vol. 273, 927-948
- **MACCALLUM R.M. ; MARTIN A.C.R. ; THORNTON J.M.** *Journal of Molecular Biology,* 1996, vol. 262 (5), 732-745
- **MARTIN ; THORNTON.** *J Mol Biol,* 1996, vol. 263, 800-815
- *Mol. Immunol.,* 2006, vol. 44, 656-665
- *Journal of Immunological Methods,* 2001, vol. 248 (1-2), 31-45
- *Biochim Biophys Acta,* 2000, vol. 1482, 337-350
- *Protein Eng. Des. Sel.,* 2004, vol. 17, 455-462
- *Nature Biotechnology,* 2005, vol. 23 (12), 1556-1561
- *Expert Opinion on Investigational Drugs,* June 2007, vol. 16 (6), 909-917
- *J. Biol. Chem,* 1999, vol. 274, 24066-24073
- *J. Mol. Biol.,* 2003, vol. 332, 489-503
- *PNAS,* 2003, vol. 100 (4), 1700-1705
- *J. Mol. Biol.,* 2007, vol. 369, 1015-1028
- *Protein Eng. Des. Sel.,* 2005, vol. 18, 435-444
- *Expert Opin. Biol. Ther.,* 2005, vol. 5, 783-797
- Non-Antibody Scaffolds from Handbook of Therapeutic Antibodies. 2007
- *Protein Science,* 2006, vol. 15, 14-27
- **SHIELDS et al.** *The Journal of Biological Chemistry,* 2001, vol. 276, 6591-6604
- **CHAPPEL et al.** *The Journal of Biological Chemistry,* 1993, vol. 268, 25124-25131
- **LAZAR et al.** *PNAS,* 2006, vol. 103, 4005-4010
- *J Imm Meth,* 1995, vol. 184, 29-38
- **DUNCAN et al.** *Nature,* 1988, vol. 332, 563-564
- **LUND et al.** *J. Immunol.,* 1991, vol. 147, 2657-2662
- **CHAPPEL et al.** *PNAS,* 1991, vol. 88, 9036-9040
- **BURTON ; WOOF.** *Adv. Immunol.,* 1992, vol. 51, 1-84
- **MORGAN et al.** *Immunology,* 1995, vol. 86, 319-324
- **HEZAREH et al.** *J. Virol.,* 2001, vol. 75 (24), 12161-12168
- **SHIELDS et al.** *J Biol Chem,* 2001, vol. 276, 6591-6604
- **NECHANSKY et al.** *Mol Immunol,* 2007, vol. 44, 1815-1817
- **LAMBERT, J.** *Curr. Opinion in Pharmacology,* 2005, vol. 5, 543-549
- **WU et al.** *Nature Biotechnology,* 2005, vol. 23 (9), 1137-1146
- **SYRIGOS ; EPENETOS.** *Anticancer Research,* 1999, vol. 19, 605-614
- **NICULESCU-DUVAZ ; SPRINGER.** *Adv. Drug Deliv. Rev.,* 1997, vol. 26, 151-172
- **BALDWIN et al.** *Lancet,* 15 March 1986, 603-05
- Antibody Carriers Of Cytotoxic Agents In Cancer Therapy: A Review. **THORPE et al.** Monoclonal Antibodies '84: Biological And Clinical Applications. 1985, 475-506
- **ROWLAND et al.** *Cancer Immunol. Immunother.,* 1986, vol. 21, 183-87

- **MANDLER et al.** *J. Nat. Cancer Inst.,* 2000, vol. 92 (19), 1573-1581
- **MANDLERET.** *Bioorganic & Med. Chem. Letters,* 2000, vol. 10, 1025-1028
- **MANDLER et al.** *Bioconjugate Chem.,* 2002, vol. 13, 786-791
- **LIU et al.** *Proc. Natl. Acad. Sci. USA,* 1996, vol. 93, 8618-8623
- **LODE et al.** *Cancer Res.,* 1998, vol. 58, 2928
- **HINMAN et al.** *Cancer Res.,* 1993, vol. 53, 3336-3342
- **WOYKE et al.** *Antimicrob. Agents and Chemother,* 2001, vol. 45 (12), 3580-3584
- **PETTIT et al.** *Antimicrob. Agents Chemother.,* 1998, vol. 42, 2961-2965
- **E. SCHRODER ; K. LUBKE.** The Peptides. Academic Press, 1965, vol. 1, 76-136
- **PETTIT et al.** *J. Am. Chem. Soc.,* 1989, vol. 111, 5463-5465
- **PETTIT et al.** *Anti-Cancer Drug Design,* 1998, vol. 13, 243-277
- **PETTIT, G. R. et al.** *Synthesis,* 1996, 719-725
- **PETTIT et al.** *J. Chem. Soc. Perkin Trans.,* 1996, vol. 15, 859-863
- **DORONINA.** *Nat Biotechnol,* 2003, vol. 21 (7), 778-784
- *Monomethylvaline Compounds Capable of Conjugation to Ligands*
- **HINMAN et al.** *Cancer Research,* 1993, vol. 53, 3336-3342
- **LODE et al.** *Cancer Research,* 1998, vol. 58, 2925-2928
- **FRAKER et al.** *Biochem. Biophys. Res. Commun.,* 1978, vol. 80, 49-57
- **CHATAL.** Monoclonal Antibodies in Immunoscintigraphy. CRC Press, 1989
- **GEOGHEGAN & STROH.** *Bioconjugate Chem.,* 1992, vol. 3, 138-146
- **DUBOWCHIK ; WALKER.** *Pharm. Therapeutics,* 1999, vol. 83, 67-123
- **NEVILLE et al.** *Biol. Chem.,* 1989, vol. 264, 14653-14661
- **THORPE et al.** *Cancer Res.,* 1987, vol. 47, 5924-5931
- **WAWRZYNCZAK et al.** Immunoconjugates: Antibody Conjugates in Radioimagery and Therapy of Cancer. Oxford U. Press, 1987
- **JOHNSON et al.** *Anticancer Res.,* 1995, vol. 15, 1387-93
- **LAU et al.** *Bioorg-Med-Chem.,* 1995, vol. 3 (10), 1299-1304
- **LAU et al.** *Bioorg-Med-Chem.,* 1995, vol. 3 (10), 1305-12